# EUROPEAN PATENT APPLICATION

(11) **EP 4 534 525 A1**
(43) Date of publication of application: **09.04.2025**
(21) Application number: 23815324.1
(22) Date of filing: 02.06.2023
(51) Int. Cl.: C07C 229/26, C07C 227/18, A61K 9/127, A61K 47/18

(54) **AMINO ACID-BASED CATIONIC LIPID CONTAINING UNSATURATED BONDS**

(30) Priority: 02.06.2022 CN 202210622191
(71) Applicant: Xiamen Sinopeg Biotech Co., Ltd., Xiamen, Fujian 361100 (CN)
(72) Inventor: LIN, Minggui, Xiamen, Fujian 361100 (CN); WENG, Wengui, Xiamen, Fujian 361100 (CN); LIU, Chao, Xiamen, Fujian 361100 (CN); WANG, Linlin, Xiamen, Fujian 361100 (CN); LIN, Sheng, Xiamen, Fujian 361100 (CN); WANG, Ailan, Xiamen, Fujian 361100 (CN); WEI, Guohua, Xiamen, Fujian 361100 (CN); ZHU, Qi, Xiamen, Fujian 361100 (CN)
(74) Representative: Algemeen Octrooi- en Merkenbureau B.V.
(86) International application number: PCT/CN2023/098158
(87) International publication number: WO 2023/232148

(57) **Abstract**

The present invention provides a novel amino acid-based cationic lipid containing unsaturated bonds, represented by the general formula (1), with symbols defined as described herein. The amino acids or derivatives used as starting materials in the preparation process are simple and easily obtainable, offering simplicity, safety, and cost-effectiveness. The amino acid-based cationic lipid of this invention features unsaturated hydrocarbon tails and a tertiary amine moiety derived from an amino acid residue. The lipid nanoparticles (LNPs) prepared from this lipid exhibit enhanced membrane fluidity, which improves membrane fusion and facilitates cellular uptake. As a result, the delivery efficiency of the amino acid-based cationic lipid containing unsaturated bonds as a delivery material is significantly improved.

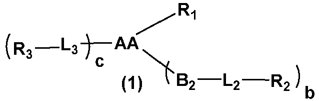

## Description

### CROSS-REFERENCE TO RELATED APPLICATIONS

This application is a U.S. National Phase Application of International Application No. PCT/CN2023/098158, filed Jun. 2, 2023 which claims priority to Chinese Application No. CN202210622191.X, filed Jun. 2, 2022. All of the aforementioned patent applications are hereby incorporated by reference in their entireties.

### TECHNICAL FIELD

The present application belongs to the field of drug delivery and specifically relates to a cationic lipid as a pharmaceutical carrier, in particular to an amino acid-based cationic lipid containing unsaturated bonds, as well as a lipid composition containing the amino acid-based cationic lipid, a lipid pharmaceutical composition, and formulations and applications thereof.

### BACKGROUND

Messenger RNA (mRNA)-based therapeutics have been demonstrated and advanced in a variety of applications, including mRNA vaccines, protein replacement therapy, cancer immunotherapy, gene editing, etc. Despite tremendous progress, mRNA medicines still face significant challenges in effective delivery. Due to physicochemical properties of mRNA, it is easily degraded by ribozymes, cannot pass through cell membranes on its own, and is usually unstable in blood circulation, so it is urgent to develop safe and efficient delivery vectors to improve the efficiency of its gene transfection. Common delivery vectors include viral and non-viral vectors. Although the transfection efficiency of viral vectors is high, their potential immunogenicity, carcinogenicity and other safety risks, as well as their small load and other shortcomings limit their clinical application. Non-viral vectors have the advantages of good safety profile, large load capacity, low cost, large-scale preparation, etc.; for example, lipid nanoparticle (LNP) has received widespread attention for being used in new COVID-19 mRNA vaccines. LNP offers many benefits for mRNA delivery, including simplicity of formulation, modularity, biocompatibility, and large mRNA payload capacity. LNP is usually composed of cationic lipids, phospholipids, steroid lipids, and PEGylated lipids; wherein, cationic lipids and drug molecules (e.g., negatively charged nucleic acids) are electrostatically bound together, while phospholipids play a role in preventing lipid oxidation or connecting ligands to the surface of LNP; steroid lipids have strong membrane fusion properties, facilitating intracellular uptake and cytoplasmic entry of drug molecules; PEGylated lipids are located on the surface of LNP, improving hydrophilicity, avoiding rapid clearance by the immune system, preventing particle aggregation and increasing stability.

Most of the LNP prepared by cationic lipids of the prior art are liver-targeted, and the effective delivery problem of organs other than the liver (such as spleen, lungs, and kidneys) needs to be solved urgently. Most cationic lipids contain hydrocarbyl chains that are saturated aliphatic hydrocarbon tail chains, and the cellular uptake is relatively poor. In this application, the head and tail chains of amino acid lipids are modified to obtain amino acid-based cationic lipids containing unsaturated bonds with higher cellular uptake rate, higher delivery efficiency and certain targeting of other organs.

### SUMMARY

The present application provides a novel amino acid-based cationic lipid containing unsaturated bonds and preparation methods thereof, comprising a lipid composition containing an amino acid-based cationic lipid, a lipid pharmaceutical composition containing the lipid composition and the preparation thereof, a liposome or LNP containing the lipid composition. Especially, the LNP-nucleic acid pharmaceutical composition containing the lipid composition and the preparations thereof have the advantages of high delivery efficiency, safety, low toxicity, and high biocompatibility, which can improve the therapeutic and/or preventive effects of the drugs.

The above-described purposes of this application can be realized via embodiments below.

In one embodiment, provided herein is an amino acid-based cationic lipid: 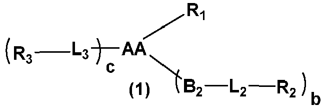
wherein, AA is a residue of an amino acid or amino acid derivative;
B₂ is independently a linking bond or a C₁₋₂₀ alkylene group at each occurrence;
L₂ and L₃ are each independently a linking bond or a divalent linking group at each occurrence;
R₁ is a C₁₀₋₄₀ aliphatic hydrocarbon group containing one, two or more unsaturated bonds, and R₂ is independently a C₁₋₄₀ aliphatic hydrocarbon group or 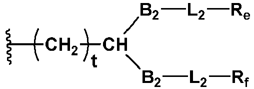 ; wherein, t is an integer from 0 to 12; Rₑ and R_{f} are each independently a C₁₋₂₀ alkyl group, a C₂₋₂₀ alkenyl group, or a C₂₋₂₀ alkynyl group;
R₃ is, at each occurrence, independently a hydrogen atom, an alkyl group, an alkoxy group, a C₃₋₆ carbocyclic group, a nitrogen-containing heterocyclic group, -NR_{d}R_{d}, -SR_{d}, -C(=O)R_{d}, -C(=O)OR_{d}, -OC(=O)R_{d} or a functional group R₀₁ that can interact with bio-related substances; wherein, R_{d} is independently a C₁₋₁₂ alkyl group at each occurrence;
b and c are each independently 1 or 2; when fragments -B₂-L₂-R₂ and/or -L₃-R₃ are protruded from the amino terminus of amino acids and their derivatives, b and c are each independently 1 or 2; when fragments -B₂-L₂-R₂ and/or -L₃-R₃ are protruded from the carboxyl terminus of amino acids and their derivatives, b and c are each independently 1; when b is 2, two -B₂-L₂-R₂ fragments are the same or different; when c is 2, two -L₃-R₃ fragments are the same or different;
or a salt, tautomer, stereoisomer, or solvate thereof.

The present application also provides a lipid composition, embodied as follows:
A lipid composition containing an amino acid-based cationic lipid with the structure represented by general formula (1).

The present application also provides a lipid pharmaceutical composition, embodied as follows:
Provided herein is a lipid pharmaceutical composition containing a lipid composition and a drug; wherein, the lipid composition contains an amino acid-based cationic lipid with the structure represented by the formula (1), and the drug is selected from the group consisting of a nucleic acid drug, a gene vaccine, an anti-tumor drug, a small molecule drug, a peptide drug, and a protein drug.

The present application also provides a formulation of lipid pharmaceutical composition, embodied as follows:
Provided herein is a formulation of lipid pharmaceutical composition containing the aforementioned lipid pharmaceutical composition and pharmaceutically acceptable diluents or excipients.

The present application also provides a liposome or LNP, embodied as follows:
Provided herein is a liposome or LNP containing a lipid composition, the lipid composition contains an amino acid-based cationic lipid with the structure represented by the formula (1).

### Compared with the prior art, the present application brings the following beneficial effects:

To address the problems of poor uptake and low transfection efficiency of LNP-mRNA pharmaceutical compositions, the present application has designed and synthesized a series of amino acid-based cationic lipids containing unsaturated bonds. The unsaturated hydrocarbon-based tail chain is more loosely arranged, which plays a key role in maintaining the stability and fluidity of the membrane, enhancing the membrane fusion ability, increasing the uptake of LNP-mRNA pharmaceutical composition by cells, and thus improving the delivery efficiency of nucleic acid drugs.

The LNP-mRNA pharmaceutical composition prepared by the novel amino acid-based cationic lipid containing unsaturated bonds of the present application is mainly distributed in the liver and spleen, which provides certain ideas for further research on the targeting issues of other organs.

A novel amino acid-based cationic lipid containing unsaturated bonds of the present application uses an amino acid or amino acid derivative as the hydrophilic head group. Through electrostatic interactions, the positively charged hydrophilic head group easily complexes with negatively charged drugs or mRNA, thereby increasing their stability in blood circulation. In addition, the present application uses amino acids or amino acid derivatives with good biocompatibility as the skeletons, further improving the biocompatibility of cationic lipids. Moreover, amino acids or amino acid derivatives are simple and easy to obtain as biological starting materials, which can be obtained through natural or simple synthesis and has the advantages of simplicity, safety, and cost saving.

Degradable groups are chosen as the linking arms of the novel amino acid-based cationic lipids containing unsaturated bonds of the present application. The existence of degradable groups enables the LNP pharmaceutical compositions prepared therefrom to be degraded promptly in vivo with low cytotoxicity, which solves the problem that LNP pharmaceutical compositions prepared from the non-degradable lipids in the prior art will accumulate and acidify the endosomal environment, hinders the endosomal escape of drugs (such as mRNA), and the drugs delivered into cells cannot produce an effect.

### BRIEF DESCRIPTION OF THE DRAWINGS

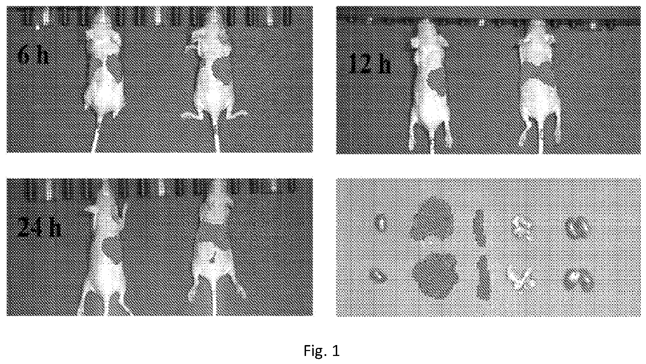

FIG. **1** Animal fluorescence imaging and in vivo organ distribution of LNP-mRNA composition (L-2).

### DETAILED DESCRIPTION OF THE APPLICATION

### Description of terms

In the present application, unless otherwise described, all technical and scientific terms used herein have the same meaning as those generally understood by those of ordinary skill in the art. The disclosures of all patents and other publications referenced herein are incorporated in their entirety by reference. In the event of a conflict between any description of the terms herein and any document incorporated herein by reference, the description and interpretation of the terms described below shall prevail.

In the present application and unless otherwise specified, a structure with isomers may refer to any form of the isomers. For example, when *cis-* and *trans-* isomers are present, it can refer to either a cis-structure or a trans-structure; when E/Z isomers are present, it can refer to either an (E)-structure or a (Z)-structure; and when optical activity is present, it can refer to either a laevoisomer or a dextroisomer.

In the present application, the numerical intervals include both the numerical interval marked by the dash line (e.g., 0-12) and the numerical interval marked by the wavy line such as (0~12), and the numerical interval indicated by "to" (e.g., 0 to 12, 1 to 12). In the present application and unless otherwise specified, an integer interval represents the group consisting of all integers between endpoints included. For example, the integer interval 0 to 12 represents the group composed of 0, 1, 2, 3, 4, 5, 6, 7, 8, 9, 10, 11 and 12. The numerical interval in the present application includes but is not limited to the integer interval, the non-integer interval, the percentage interval, and the fraction interval; unless otherwise specified, all of the foregoing numerical intervals include two endpoints.

In the present application, "about" or "approximately" followed by a numerical value generally suggests a ±10% numerical range; in some cases, the numerical range can be enlarged to a greater one (e.g., ±15%) but is no more than ±20%. For example, when the molar percentage of steroid lipids among the total lipids in a solution containing solvent is about 40%, it can be considered that, generally, the molar percentage of steroid lipids is 30%-50%.

In the present application, unless otherwise specified, "any" includes any one, any two, and any two or more.

The terms include", "contain", and similar expressions in the present application shall be interpreted, unless otherwise specified, as "including but not limited to", "include but are not limited to", or "includes but is not limited to", etc., in the description and claims with openness and inclusiveness.

In the present application, when two or more objects are "each independently preferably" selected from multiple levels of preferable options, the objects are not necessarily selected from preferable options of the same level. It is allowed that one is selected from a wider range of options while another one is selected from a narrower range of options. It is also allowed that one is selected from the maximum range of options while another is selected from any allowable options. It is also allowed that the objects are selected from preferable options of the same level.

In the present application, "each independently at each occurrence", and similar expressions not only mean that different groups can be each independently selected from the definitions, but also means that the same group at different positions can be independently selected from the definitions; the groups including but not limited to the groups mentioned above. For example, "are each independently selected from the group consisting of a linking bond, -OC(=O)-, -C(=O)O-, -OC(=O)O-, -C(=O)-, -O-, -NH-, -O(CR_{c}R_{c})ₛO-, -S-, -C(=O)S-, -SC(=O)-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, and -NR_{c}C(=O)S-; wherein, R_{c} is independently a hydrogen atom or a C₁₋₁₂ alkyl group at each occurrence", two R_{c} in "-NR_{c}C(=O)NR_{c}-" can be the same or different, which are each independently a hydrogen atom or a C₁₋₁₂ alkyl group.

In the present application and unless otherwise specified, for divalent linking groups, e.g., hydrocarbylene group, alkylene group, arylene group, amide bond, and the like, either one of the two linking ends could be chosen to be linked to another group. For example, when an amide bond serves as a divalent linking group between GroupA and GroupB, both GroupA-C(=O)NH-GroupB and GroupB-NHC(=O)-GroupA are allowable.

In the present application, when distinguishing the terminal groups from the substituents of a linking group becomes questionable, " 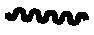 " is used to indicate the connection location between the linking group and the other group. For example, in structural formulas 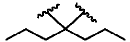 and 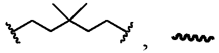 is used to indicate the connection locations between the divalent linking groups and the other groups; the two structural formulas mentioned above represent -CH(CH₂CH₂CH₃)₂- and -CH₂CH₂CH(CH₃)₂-CH₂CH₂-, respectively.

In the present application, a numerical interval written in the subscript of "C" can be used to indicate the number of carbon atoms of a group. For example, a C₁₋₁₂ group is a group having 1 to 12 carbon atoms; C₁₋₃₀ indicates "having 1 to 30 carbon atoms"; a "substituted C₁₋₁₂ alkyl group" means a C₁₋₁₂ alkyl group with one or more hydrogen atoms being substituted. "C₁₋₁₂ substituted alkyl group" refers to a compound having 1 to 12 carbon atoms in which the hydrogen atoms of the alkyl group are substituted. As another example, when a group can be selected from C₁₋₁₂ alkylene groups, it can be any alkylene group with the number of carbon atoms in the range indicated by the subscript, that is, the group can be selected from the group consisting of C₁, C₂, C₃, C₄, C₅, C₆, C₇, C₈, C₉, C₁₀, C₁₁, and C₁₂ alkylene groups. In the present application and unless otherwise specified, a subscript being a numerical interval indicates that the subscript can be any integer within the interval which includes two endpoints.

In the present application, heteroatoms are not particularly limited, including but not limited to O, S, N, P, Si, F, Cl, Br, I, B, etc.

In the present application, the heteroatom used for substitution is referred to as "substituent atom", and the group used for substitution is referred to as "substituent group".

In the present application, a "substituted" group indicates that at least one hydrogen atom of the group (any aforementioned group, unless otherwise specified, e.g., aliphatic hydrocarbon groups, hydrocarbon groups, alkyl groups, or alkylene groups) is replaced by a bond linking to a non-hydrogen atom, the non-hydrogen atom including but not limited to a halogen atom (F, Cl, Br, or I), an oxo group (=O), a hydroxyl group (-OH), a hydrocarbyloxy group (-OR_{d}; wherein, R_{d} is a C₁₋₁₂ alkyl group), a carboxyl group (-COOH), an amine group (-NR_{c}R_{c}; wherein, both R_{c} are each independently a hydrogen atom or a C₁₋₁₂ alkyl group), a C₁₋₁₂ alkyl group, and a cycloalkyl group. In some embodiments, the substituent group is a C₁₋₁₂ alkyl group. In another embodiment, the substituent is a cycloalkyl group. In another embodiment, the substituent is a halide group, e.g., fluoride. In another embodiment, the substituent is an oxo group. In another embodiment, the substituent is a hydroxyl group. In another embodiment, the substituent is an alkoxy group. In another embodiment, the substituent is a carboxyl group. In another embodiment, the substituent is an amine group.

In the present application, "optional" or "optionally" (e.g., optionally substituted) means that the event of the situation described thereafter may or may not occur, and the description includes instances in which the event or situation occurs as well as instances in which the event or situation does not occur. For example, "optionally substituted hydrocarbyl" means that the hydrocarbyl group may or may not be substituted, and the description includes both substituted and unsubstituted hydrocarbyl groups.

In the present application, "carbon chain linker" or "carbon chain linking group" refers to the linking group whose main-chain atoms are all carbon atoms, allowing heteroatoms or groups containing heteroatoms in the side chains which substitute for hydrogen atoms connected to main-chain carbon atoms. When a "main-chain atom" is a heteroatom, it can also be called "main-chain heteroatom". For example, A-S-CH₂-B, A-O-CH₂-B, and 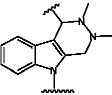 (wherein the atomic spacing is 4) are considered to contain main-chain heteroatoms. Carbon chain linking groups include hydrocarbylene groups and those whose pendant groups contain heteroatoms; the carbon chain linking groups containing heteroatoms in the pendant groups include but are not limited to an oxo group (=O), a thioxo group (=S), an imino group (connected to the main-chain carbon through a carbon-nitrogen double bond), an oxa-hydrocarbon group in the form of an ether bond, a thia-hydrocarbon group in the form of a thioether bond, and an aza-hydrocarbon group in the form of a tertiary amino group, etc. The main chain of the "carbon chain linker" is entirely composed of carbon atoms, and the pendant groups of the carbon chain are allowed to contain heteroatoms, that is, the main chain is constructed by connecting methylene groups or substituted methylene groups. The substituted methylene groups can have one monovalent substituent, two monovalent substituents, or one divalent substituent (e.g., divalent oxygen (=O), or one that forms a three-membered ring 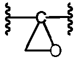 with a divalent methylene group). The substituted methylene groups can have one hydrogen atom being substituted (e.g., -CH(CH₃)-), two hydrogen atoms being respectively substituted (e.g., -(CH₃)C(OCH₃)-), or two hydrogen atoms being simultaneously substituted (e.g., a carbonyl group, a thiocarbonyl group, -C(=NH)-, and -C(=N⁺H₂)-), or a cyclic pendant group (e.g., 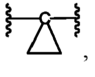 wherein the atomic spacing is 1).

In the present application, a compound or a group can be substituted and heterosubstituted at the same time. For example, a hydrogen atom can be replaced by a nitrophenyl group, and -CH₂-CH₂-CH₂- can be replaced by -CH₂-S-CH(CH₃)-.

In the present application, a "linking bond" without any atom is only for connection, that is, when a group is denoted as a linking bond, the group can be absent.

In the present application, "group" contains at least one atom, referring to the radical formed by a compound losing one or more atoms. With respect to a compound, the remaining group formed by the removal of other groups is also denoted as "residue". The valence of groups is not particularly limited, and examples include a monovalent group, a divalent group, a trivalent group, a tetravalent group, ..., a hectovalent group, etc. Wherein, groups with valence being equal to or greater than two are collectively defined as linking groups. A linking group can also contain only one atom, such as an oxo group and a thio group.

In the present application, "hydrocarbon" refers to a class of compounds that contain only carbon atoms and hydrogen atoms.

In the present application, hydrocarbons are classified into aliphatic hydrocarbons and aromatic hydrocarbons in terms of the type of hydrocarbon groups. Hydrocarbons containing neither phenyl rings nor hydrocarbyl-substituted phenyl rings are defined as aliphatic hydrocarbons. Hydrocarbons containing at least one phenyl ring or hydrocarbyl-substituted phenyl ring are defined as aromatic hydrocarbons. An aromatic hydrocarbon can contain aliphatic hydrocarbyl groups, such as toluene, diphenylmethane, 2,3-dihydroindene, etc.

In the present application, hydrocarbons are classified into saturated hydrocarbons and unsaturated hydrocarbons in terms of the degree of saturation. All aromatic hydrocarbons are unsaturated hydrocarbons. Saturated aliphatic hydrocarbons are also termed alkanes. The degree of saturation of unsaturated aliphatic hydrocarbons is not particularly limited. For example, unsaturated aliphatic hydrocarbons include but are not limited to alkenes (containing carbon-carbon double bonds), alkynes (containing carbon-carbon triple bonds), dienes (containing two conjugated carbon-carbon double bonds), and the like. When the aliphatic moieties of aromatic hydrocarbons are saturated, aromatic hydrocarbons are also termed aralkanes, such as toluene.

In the present application, the structures of hydrocarbons are not particularly limited, including linear structures without pendant groups, branched structures with pendant groups, ring-containing structures, dendritic structures, comb-like structures, hyperbranched structures, etc. Unless otherwise specified, preferable structures include linear structures without pendant groups, branched structures with pendant groups, and ring-containing structures, corresponding to linear hydrocarbons, branched hydrocarbons and cyclic hydrocarbons, respectively. Wherein, hydrocarbons that contain no rings are termed open-chain hydrocarbons, including but not limited to linear structures without pendant groups, and branched structures with pendant groups. Open-chain hydrocarbons belong to aliphatic hydrocarbons. Therefore, linear hydrocarbons are also referred to as linear aliphatic hydrocarbons and branched hydrocarbons are also referred to as branched aliphatic hydrocarbons.

In the present application, "hydrocarbon group" refers to the residue of a hydrocarbon molecule with at least one hydrogen atom removed. According to the number of removed hydrogen atoms, hydrocarbon groups can be classified into monovalent hydrocarbon groups (with one hydrogen atom removed), divalent hydrocarbon groups (with two hydrogen atoms removed), trivalent hydrocarbon groups (with three hydrogen atoms removed), and the like. Accordingly, when n hydrogen atoms are lost, the valence of the formed hydrocarbon group is n compared with the original molecule. Unless otherwise specified, hydrocarbon groups in the present application refer to monovalent hydrocarbon groups. Unless otherwise expressly stated in this specification, the hydrocarbon group is optionally substituted.

In the present application, the source of hydrocarbon group is not particularly limited; for example, hydrocarbon group can be derived from aliphatic hydrocarbons or aromatic hydrocarbons, from saturated hydrocarbons or unsaturated hydrocarbons, from linear hydrocarbons, branched hydrocarbons or cyclic hydrocarbons, or from hydrocarbons or heterohydrocarbons, etc. According to the degree of saturation, hydrocarbon groups can be derived from alkanes, alkenes, alkynes, dienes, etc. Concerning cyclic hydrocarbons, hydrocarbon groups can be derived from alicyclic hydrocarbons, aromatic hydrocarbons, monocyclic hydrocarbons, or polycyclic hydrocarbons. With respect to heterocyclic hydrocarbons, hydrocarbon groups can be derived from aliphatic heterocyclic hydrocarbons or aromatic heterocyclic hydrocarbons.

In the present application, "aliphatic group" refers to the residue of an aliphatic hydrocarbon molecule with at least one hydrogen atom removed. Unless otherwise specified, aliphatic hydrocarbon groups refer to monovalent aliphatic groups in the present application. The aliphatic hydrocarbon group includes saturated aliphatic hydrocarbon groups and unsaturated aliphatic hydrocarbon groups. Unless otherwise expressly stated in this specification, aliphatic hydrocarbon groups are optionally substituted.

In the present application, "alkyl group" refers to a hydrocarbon group obtained from an alkane losing a hydrogen atom at any location, unless otherwise specified, the alkyl group can be linear or branched, substituted or unsubstituted. Specific examples include that a propyl group refers to either a 1-propyl group or an isopropyl group and a propylene group can refer to a 1,3-propylene group, a 1,2-propylene group, or an isopropylidene group. Unless otherwise expressly stated in this specification, alkyl groups are optionally substituted.

In the present application, "unsaturated hydrocarbon group" refers to the hydrocarbon group obtained from an unsaturated hydrocarbon losing hydrogen atoms. The hydrocarbon groups obtained from unsaturated hydrocarbon losing hydrogen atoms bonded to unsaturated carbon atoms can include alkenyl groups, alkynyl groups, dienyl groups, and the like, e.g., propenyl groups and propynyl groups. According to the type of unsaturated bond, the hydrocarbon groups formed by removing hydrogen atoms bonded to saturated carbon atoms of unsaturated hydrocarbons can be termed alkenyl hydrocarbon groups, alkynyl hydrocarbon groups, dienyl hydrocarbon groups, etc., and specifically such as allyl groups or propargyl groups.

In the present application, the "alkenyl" or "alkenyl group" refers to a substituted or unsubstituted alkenyl group with a linear structure or a branched structure, containing two or more carbon atoms (e.g., 2, 3, 4, 5, 6, 7, 8, 9, 10, 11, 12,13, 14, 15, 16, 17, 18, 19, 20 or more carbon atoms) and at least one carbon-carbon double bond. "C₂₋₁₅ alkenyl group' refers to a substituted or unsubstituted alkenyl group with linear structure or branched structure, containing 2 to 15 carbon atoms and at least one carbon-carbon double bond, that is, an alkenyl group can contain one, two, three, four, or more carbon-carbon double bonds. Unless otherwise specified, alkenyl groups include substituted and unsubstituted alkenyl groups in the present application. Unless otherwise expressly stated in this specification, alkenyl groups are optionally substituted.

In the present application, the "alkynyl" or "alkynyl group" refers to an optionally substituted hydrocarbon with a linear structure or a branched structure, containing two or more carbon atoms (such as 2, 3, 4, 5, 6, 7, 8, 9, 10, 11, 12,13, 14, 15, 16, 17, 18, 19, 20, or more carbon atoms) and at least one carbon-carbon triple bond. "C₂₋₁₅ alkynyl group" refers to a substituted or unsubstituted alkynyl group with linear structure or branched structure, containing 2 to 15 carbon atoms and at least one carbon-carbon triple bond, that is, an alkynyl group can contain one, two, three, four, or more carbon-carbon triple bonds. Unless otherwise specified, alkynyl groups include substituted and unsubstituted alkynyl groups in the present application. Unless otherwise expressly stated in this specification, alkynyl groups are optionally substituted.

In the present application, "hydrocarbylene group" or "hydrocarbylene chain" refers to a linear or branched divalent hydrocarbon chain that connects the remainder of a molecule to a free radical, consisting only carbon and hydrogen, being saturated or unsaturated. For example, a hydrocarbylene group with one to twenty-four carbon atoms (C₁₋₂₄ hydrocarbylene groups), a hydrocarbylene group with one to twelve carbon atoms (C₁₋₁₂ hydrocarbylene groups), specifically, a methylene group, an ethylene group, a propylene group, n-butylene group, a vinylene group, a propenylene group, an n-butenylene group, a propynylene group, an n-butynylene group, etc. Unless otherwise explicitly stated in this specification, hydrocarbylene groups are optionally substituted.

In the present application, "alkylene group" is also a divalent alkyl group, including an open-chain alkylene group and a divalent cycloalkyl group. An open-chain alkylene group refers to a divalent alkyl group without a cyclic structure, and a divalent cycloalkyl group refers to a divalent alkyl group containing a cyclic structure. Unless otherwise expressly stated in this specification, alkylene groups are optionally substituted.

In the present application, "molecular weight" reprsents the mass of a compound. The "average molecular weight" represents the mass of a compound component of a general in macroscopic matter, and unless otherwise specified, the "average molecular weight" also refers to the "number average molecular weight" (Mₙ). The number average molecular weight can be used to describe the molecular weight of polydisperse blocks or substances, or that of monodisperse blocks or substances. The measuring unit of "molecular weight" and "average molecular weight" is Dalton (Da), unless otherwise specified. The molecular weight of polyethylene glycol chain can also be measured in "degree of polymerization" which is, specifically, the number of repeat units (oxyethylene units, i.e., EO units) in the compound molecule. Accordingly, "average degree of polymerization", "number average degree of polymerization", or "number of EO units" represents the average value or the number average value of the number of repeating units.

In the present application, the term "about" and "approximately" before a percentage refers to a range of ±0.5%.

In the present application, the terms "stable" and "degradable" are a pair of opposing concepts. For detailed examples of stable groups and degradable groups are given in paragraphs [0134]-[0145] in CN113402405A.

In the present application, "hydroxyl protecting group" includes all the groups that can be used as common hydroxyl protecting groups. A hydroxyl protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a t-butyldimethylsilyl group, an allyl group, an acetal group, or a ketal group. Removal of acetyl groups is generally carried out under basic conditions, most commonly by ammonolysis with NH₃/MeOH or by methanolysis catalyzed by methanol anions. The benzyl group can be easily removed via palladium-catalyzed hydrogenolysis in neutral solution at room temperature, or via a reduction reaction by metallic sodium in ethanol or liquid ammonia. The triphenylmethyl group is generally removed via catalytic hydrogenolysis. The trimethylsilyl groups are generally removed using reagents containing fluoride ions (e.g., tetrabutylammonium fluoride/anhydrous THF, etc.). The t-butyldimethylsilyl ether is relatively stable, which can withstand ester hydrolysis conditions with alcoholic potassium hydroxide and mild reduction conditions (e.g., Zn/CH₃OH and the like), and can be removed by fluoride ions (e.g., Bu₄N⁺F⁻) in THF solution or by aqueous acetic acid at room temperature.

In the present application, "carboxyl protecting group" refers to the protecting group which can be transformed into a carboxyl group via the hydrolysis or the deprotection reaction of the carboxyl protecting group itself. Carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me), and an ethyl group (Et). In the present application, "protected carboxyl group" refers to the group protected by an appropriate carboxyl protecting group, preferably selected from the group consisting of a methoxycarbonyl group, an ethoxycarbonyl group, a t-butoxycarbonyl group, and a benzyloxycarbonyl group. The carboxyl protecting groups can be removed through hydrolysis catalyzed by acids or alkalis, or through pyrolysis reactions occasionally; for example, the t-butyl group can be removed under mildly acidic conditions, and the benzyl groups can be removed by hydrogenolysis. The reagent used for removal of carboxyl protecting groups is selected from the group consisting of TFA, H₂O, LiOH, NaOH, KOH, MeOH, EtOH, and combinations thereof, preferably selected from the group consisting of the combination of TFA and H₂O, the combination of LiOH and MeOH, and the combination of LiOH and EtOH. A protected carboxyl group can undergo deprotection and then produce the corresponding free acid; the deprotection is conducted in the presence of an alkali, and the alkali forms pharmaceutically acceptable salt with the free acid produced via the deprotection.

In the present application, an "amino protecting group" includes all the groups which are used as amino protecting groups generally, such as an aryl C₁₋₆ alkyl group, a C₁₋₆ alkoxy C₁₋₆ alkyl group, a C₁₋₆ alkoxycarbonyl group, an aryloxycarbonyl group, a C₁₋₆ alkylsulfonyl group, an arylsulfonyl group, a silyl group, etc. An amino protecting group is preferably selected from the group consisting of a t-butoxycarbonyl group (Boc), a p-methoxybenzyloxycarbonyl group (Moz), and a 9-fluorenylmethyloxycarbonyl (Fmoc). The reagent used for removal of amino protecting groups is selected from the group consisting of TFA, H₂O, LiOH, NaOH, KOH, MeOH, EtOH, and combinations thereof, preferably selected from the group consisting of the combination of TFA and H₂O, the combination of LiOH and MeOH, and the combination of LiOH and EtOH. The reagent used for removal of the Boc protecting group can be TFA or HCl/EA, preferably TFA. The reagent used for the removal of the Fmoc protecting group can be theN,N-dimethylformamide (DMF) solution containing 20% piperidine.

In the present application, "cation" refers to the corresponding structure bearing a positive charge, either permanently, or non-permanently but in response to certain conditions such as pH. Therefore, the cations include permanent cations and those cationic compounds, groups, or atoms. Permanent cations refer to the corresponding compounds, groups, or atoms that bear positive charges under conditions of any pH value or hydrogen ion activity of their environment; typically, a positive charge is generated by the presence of quaternary nitrogen atom. When a compound carries multiple such positive charges, it can be termed a permanent cation. A cationic substance refers to a compound, group, or atom that is positively charged at a lower pH and uncharged at a higher pH of its environment. Moreover, in non-aqueous environments where pH cannot be determined, a cationic compound, group, or atom is positively charged at high hydrogen ion concentration and uncharged at low concentration or activity of hydrogen ions. It depends on the individual properties of the cationic or polycationic compound, in particular the pKa of the respective cationic group or atom, which is charged or uncharged at the pH or hydrogen ion concentration. In the diluted aqueous environment, the Henderson-Hasselbalch equation can be used to estimate the fraction of positively charged cationic compounds, groups or atoms, which is well-known to those skilled in the art. For example, in some embodiments, if a compound or moiety is cationic, pH of the compound or moiety is preferably about 1 to 9, preferably 4 to 9, 5 to 8, or even 6 to 8, equal to or below 9, equal to or below 8, equal to or below 7, most preferably at physiological pH values, e.g., about 7.3 to 7.4, i.e., positively charged under physiological conditions, especially under normal saline conditions in cells in vivo. In other embodiments, it is preferably that the cationisic compound or moiety is predominantly neutral at physiological pH values, e.g., about 7.0-7.4, but becomes positively charged at lower pH values. In some embodiments, pKa for the cationic compound or moiety is preferablyabout 5 to 7.

In the present application, "cationic component/compound" typically refers to a charged molecule that is positively charged (cation) at a pH of about 1 to 9. In some embodiments, the cationic component/compound is preferably charged at a pH of or below 9 (e.g., 5 to 9), of or below 8 (e.g., 5 to 8), of or below 7 (e.g., 5 to 7), most preferably at a physiological pH (e.g., about 7.3 to 7.4). Thus, cationic peptides, proteins, polysaccharides, lipids, or polymers according to one embodiment of the present application are positively charged under physiological conditions, especially under physiological conditions of cells in vivo.

In the present application, LNP, cationic peptides, proteins, polysaccharides, lipids, or polymers are uncharged, having a neutral charge or being electrically neutral under physiological conditions, especially under the physiological salt conditions of cells in vivo. Cationic peptides or proteins preferably contain a larger amount of cationic amino acids, such as a great number of Arg, His, Lys or Orn than other amino acid residues (in particular, more cationic amino acids than anionic amino acid residues such as Asp or Glu) or components comprising mainly cationic amino acid residues. The expression "cation" can also refer to "polycation" components/compounds, or it can also refer to a cationic lipid that can be positively charged. For example, cationic lipids contain one or more amine groups with a positive charge, preferably cationic lipids are ionizable, allowing them to exist in either a positively charged or neutral form according to pH. The ionization of cationic lipids affects the surface charge of LNPs at different pH conditions. This charge state can affect plasma protein absorption, blood clearance and tissue distribution, as well as the ability to form non-bilayer structures that are critical for intracellular delivery of nucleic acids.

In the present application, "PEGylated lipid" (i.e., PEG-lipid) refers to the molecules containing both lipid and PEG moieties.

In the present application, "neutral lipid" refers to any lipid substance that is uncharged or exists in the form of neutral zwitterion at the chosen pH, preferably phospholipid. A neutral lipid can be synthetic or natural.

In the present application, "steroid lipid" refers to a steroid or a steroid analogue.

In the present application, "amino acid residue" is an amino acid from which, formally, a hydrogen atom has been removed from an amino group and/or from which, formally, a hydroxy group has been removed from a carboxy group and/or from which, formally, a hydrogen atom has been removed from a sulfydryl group and/or with a protected amino group and/or with a protected carboxyl group and/or with a protected sulfydryl group. Imprecisely, an amino acid residue can be described as an amino acid. The source of the amino acid in the present application, unless otherwise specified, is not particularly limited; that is, the amino acid can be either natural or unnatural, or a mixture thereof. The configuration of the amino acid in the present application is not particularly limited, which could be L-type, D-type, or a mixture thereof. In one embodiment of the present application, the amino acid is a hydrophobic amino acid, selected from the group consisting of tryptophan (Trp), phenylalanine (Phe), valine (Val), isoleucine (Ile), leucine (Leu) or tyrosine (Tyr). **In** another embodiment of the present application, amino acids are hydrophilic amino acids, selected from the group consisting of glutamic acid (Glu), aspartic acid (Asp), histidine (His), glutamine (Gln), asparagine (Asn), serine (Ser), threonine (Thr), proline (Pro), glycine (Glys), lysine (Lys) or arginine (Arg), preferably glycine or lysine, more preferably lysine.

**In** the present application, "variant form" refers to a structure that can be transformed into the target reactive group after any process of chemical change selected from the group consisting of oxidation, reduction, hydration, dehydration, electronic rearrangement, structural rearrangement, salt complexation and decomplexation, ionization, protonation, deprotonation, substitution, deprotection, leaving group transformation, etc.

In the present application, "variant form of reactive group" refers to a form which still has activity (remains reactive group) after the reactive group undergoes at least one process of chemical change selected from oxidation, reduction, hydration, dehydration, electronic rearrangement, structural rearrangement, salt complexation and decomplexation, ionization, protonation, deprotonation, substitution, deprotection, leaving group transformation, etc., or an inactive form that has been protected.

In the present application, "micro-modification" refers to a process of chemical modification that can be completed through simple chemical reactions. Simple chemical reactions mainly include deprotection, salt complexation and decomplexation, ionization, protonation, deprotonation, leaving group transformation, etc.

The "variant form with micro-modification" corresponds to the "micro-modification", referring to a structural form that can be transformed into the target reactive group after simple chemical reactions selected from deprotection, salt complexation and decomplexation, ionization, protonation, deprotonation, leaving group transformation, etc.; the leaving group transformation includs the transformation from the form of ester to the form of acyl chloride.

"Any suitable" in "any suitable linking group", "any suitable reactive group", etc. in the present application refers to a structure that conforms to the basic principles of a chemical structure and can enable the preparation method of the present application to be successfully implemented. The chemical structure described in this way can be considered to have a clear, defined range.

When at least two structural types are enumerated, "any combination" of the enumerated structure types means a combination of any two or more of the relevant structural types listed above. The number of structural units is not limited, the number of any structural unit can be zero, one, or greater than one. When the number of structural units of the same type is greater than one, the structural units could be the same or different chemical structures, and the total number of the structral units is at least two. For example, any arbitrary combination of an alkylene group, a divalent cycloalkyl group, a divalent cycloalkenyl group, a divalent cycloalkynyl group, a divalent cyclodienyl group, an arylene group, a carbon-carbon double bond, a carbon-carbon triple bond, a conjugated carbon-carbon double bond, a divalent aliphatic heterocyclic linking group, a divalent aromatic heterocyclic linking group, and a carbon chain linking group with heteroatom-containing pendant groups, can be, e.g., -Ph-CH₂-Ph-(arylene-alkylene-arylene), -CH₂-Ph-CH₂CH₂- (alkylene-arylene-alkylene; wherein,the alkylene group has a quantity of 2, with different chemical structures), or a structure obtained from the phenyl ring of any aforementioned example being replaced with the ring of a hexane, diazepine, or 1-(2-pyridinyl)hexahydro-1H-1,4-diazepine. As another example, cycloalkenyl hydrocarbon group = cycloalkenyl group + hydrocarbylene group = hydrocarbon group substituted by a cycloalkenyl group, and cyclodienyl hydrocarbon group = hydrocarbon group substituted by a cyclodienyl group.In the present application, "N/P ratio" refers to the molar ratio of nitrogen atoms in cationic lipids to phosphates in nucleic acids.

In the present application, "nucleic acid" refers to DNA, RNA or their modified forms, including purine or pyrimidine bases in DNA (adenine "A", cytosine "C", guanine "G", thymine "T"), and purine or pyrimidine bases in RNA (adenine "A", cytosine "C", guanine G", uracil "U").

In the present application, "RNA" refers to ribonucleic acid that may be naturally or non-naturally occurring. For example, an RNA may include modified and/or non-naturally occurring components such as one or more nucleobases, nucleosides, nucleotides, and linkers. An RNA may include a cap structure, a chain terminating nucleoside, a stem loop, a polyA sequence, and/or a polyadenylation signal. An RNA may have a nucleotide sequence encoding a polypeptide of interest. For example, an RNA may be a messenger RNA (mRNA). Translation of an mRNA encoding a particular polypeptide, for example, in vivo translation of an mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA (messenger RNA), singleguiderna RNA (sgRNA), self-amplifying RNA (saRNA), circular RNA (circRNA), cas9 mRNA and mixtures thereof.

In the present application, antisense oligonucleotides and small interfering RNA (siRNA) can inhibit the expression of a target gene and target protein in vitro or in vivo.

In the present application, FLuc mRNA can express luciferase protein which emits bioluminescence in the presence of fluorescein substrate, so FLuc is commonly used in mammalian cell culture to measure gene expression and cell activity.

In the present application, "inhibiting expression of a target gene" refers to the ability of nucleic acids to silence, reduce, or inhibit the expression of a target gene. To examine the extent of gene silencing, a test sample (e.g., a sample of cells in culture expressing the target gene) is contacted with nucleic acids that inhibit the expression of the target gene. The expression of the target gene in the test sample or test animal is compared to the expression of the target gene in a control sample (e.g., a sample of cells in culture expressing the target gene) which is not contacted with or administered the nucleic acids. The expression of the target gene in the control sample may be assigned a value of 100%. In particular embodiments, inhibition of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%.

In the present application, suitable assays for determining the level of target gene expression include but are not limited to dot blots, northern blots, in situ hybridization, ELISA, immunoprecipitation, enzyme function, and phenotypic assays.

In the present application, "transfection" refers to the introduction of a species (e.g., an RNA) into a cell. Transfection may occur, for example, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the present application, the antigen may be a translation product of the provided nucleic acid molecule (preferably mRNA as defined herein). In this context, fragments, variants, and derivatives of peptides and proteins containing at least one epitope are also defined as antigens.

In the present application, "delivery" refers to delivering an entity to the target, for example, delivering drugs and/or therapeutic agents and/or prophylactic agents to subjects, the subjects are tissues and/or cells of humans and/or other animals.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including those from petroleum, animal, vegetable, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral preparations can contain standard carriers, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigmentss), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, vaccines are preventive or therapeutic materials that provide at least one antigen or antigenic function. Antigen or antigenic function can stimulate the body's adaptive immune system to provide an adaptive immune response.

In the present application, treatment refers to the management and care of patients to resist diseases, obstacles, or symptoms, which is intended to delay the development of diseases, obstacles, or symptoms, reduce or alleviate symptoms and complications, and/or cure or eliminate diseases, obstacles, or symptoms. The patients to be treated are preferably mammals, especially humans.

### 1. Amino acid-based cationic lipids

An embodiment of the present application:
An amino acid-based cationic lipid; wherein, the structure is represented by the general formula (1): 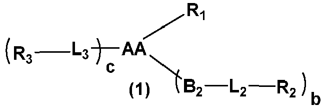
Wherein, AA is a residue of an amino acid or amino acid derivative;
B₂ is independently a linking bond or a C₁₋₂₀ alkylene group at each occurrence;
L₂ and L₃ are each independently a linking bond or a divalent linking group at each occurrence;
R₁ is a C₁₀₋₄₀ aliphatic hydrocarbon group containing one, two or more unsaturated bonds, R₂ is independently a C₁₋₄₀ aliphatic hydrocarbon group or 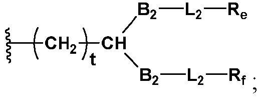 wherein, t is an integer from 0 to 12; Rₑ and R_{f} are each independently a C₁₋₂₀ alkyl group, a C₂₋₂₀ alkenyl group, or a C₂₋₂₀ alkynyl group;
R₃ is, at each occurrence, independently a hydrogen atom, an alkyl group an alkoxy group, a C₃₋₆ carbocyclic group, a nitrogen-containing heterocyclic group, -NR_{d}R_{d}, -SR_{d}, -C(=O)R_{d}, -C(=O)OR_{d}, -OC(=O)R_{d} or a functional group R₀₁ that can interact with bio-related substances; wherein, R_{d} is independently a C₁₋₁₂ alkyl group at each occurrence;
b and c are each independently 1 or 2; when fragments -B₂-L₂-R₂ and/or -L₃-R₃ are protruded from the amino terminus of amino acids and their derivatives, b and c are each independently 1 or 2; when fragments -B₂-L₂-R₂ and/or -L₃-R₃ are protruded from the carboxyl group terminus of amino acids and their derivatives, b and c are each independently 1; when b is 2, two -B₂-L₂-R₂ fragments are the same or different; when c is 2, two -L₃-R₃ fragments are the same or different; when c is 2, two -L₃-R₃ fragments are the same or different;
or a salt, tautomer, stereoisomer, or solvate thereof.

### 1.1. Residues of amino acids or amino acid derivatives, AA

In the present application, AA is a residue of an amino acid or amino acid derivative.

In a specific embodiment of the present application, the aforementioned amino acid or amino acid derivative is preferably selected from the group consisting of arginine, aspartic acid, asparagine, cysteine, glutamic acid, glutamine, histidine, lysine, methionine, phenylalanine, serine, threonine, tryptophan, tyrosine, and an amino acid derivative of any of the aforementioned amino acids, more preferably selected from the group consisting of lysine, glutamic acid and histidine, more preferably selected from the group consisting of the following structures and the corresponding forms having 1 to 3 protected terminal groups: 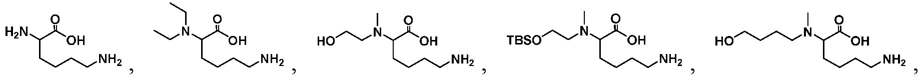 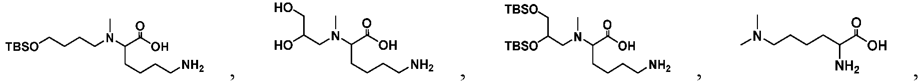 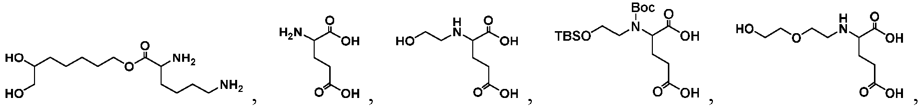 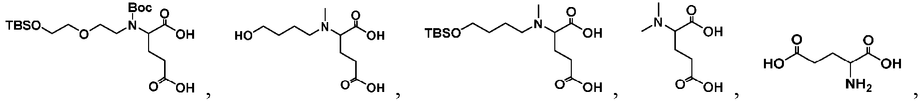 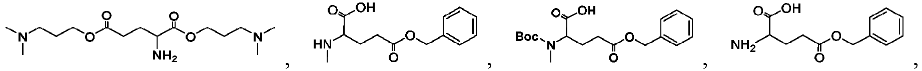 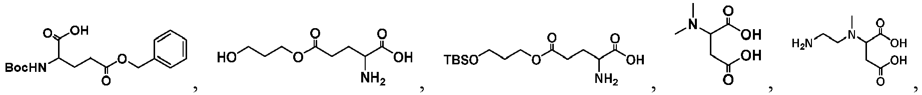 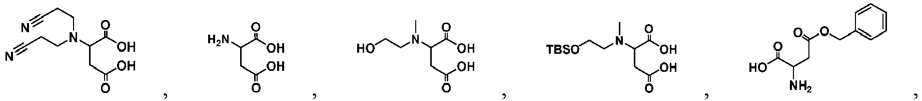 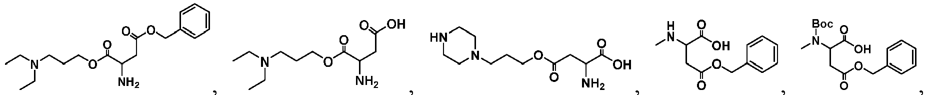 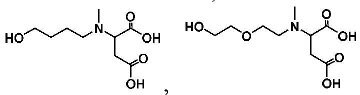 and

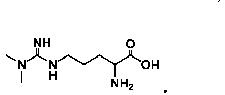

In a specific embodiment of the present application, AA is preferably 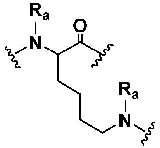 or 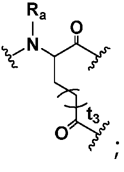 wherein, Rₐ is independently selected from the group consisting of a linking bond, H, a methyl group, an ethyl group, a propyl group, and an isopropyl group at each occurrence; or, AA is the case where one or two carbonyl groups in any of the above structures are independently capped by an oxygen atom or a secondary amine group: 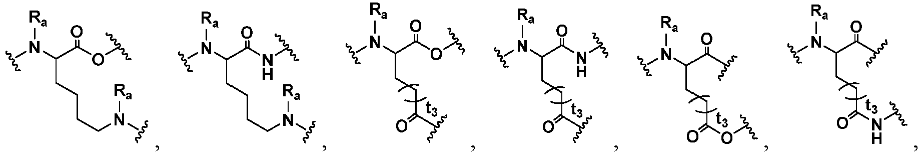 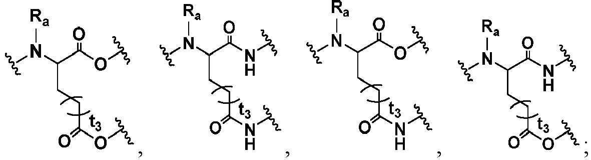 AA is more preferably selected from the group consisting the following structures: 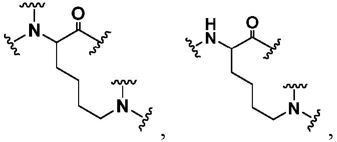 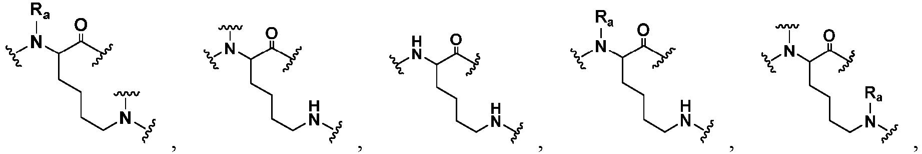 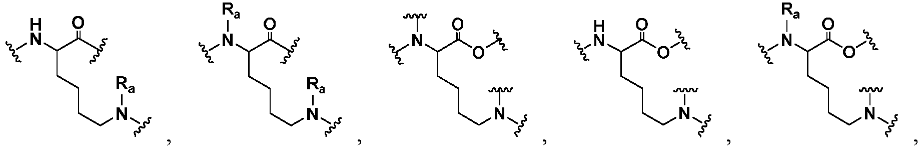 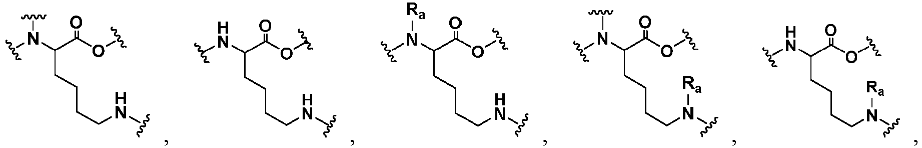 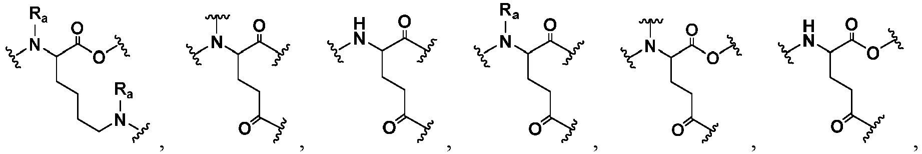 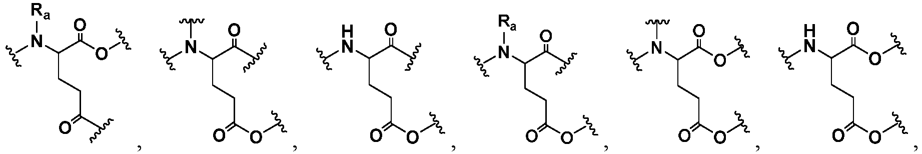 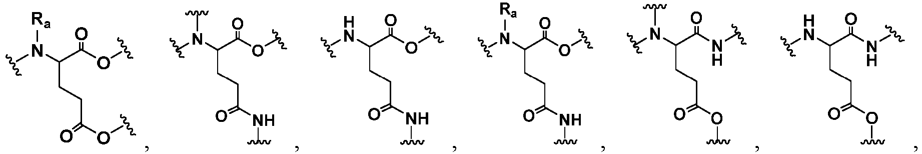 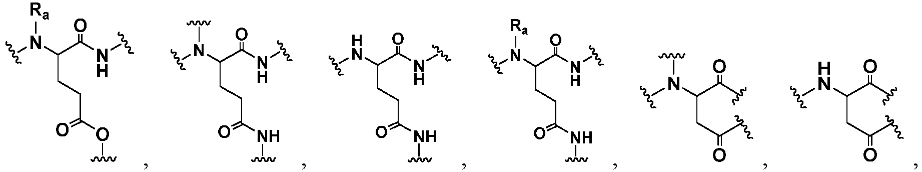 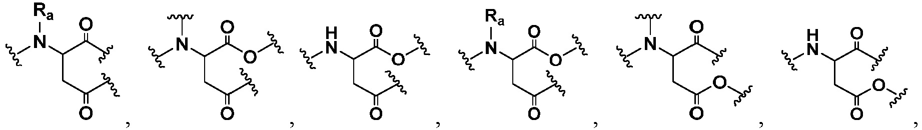 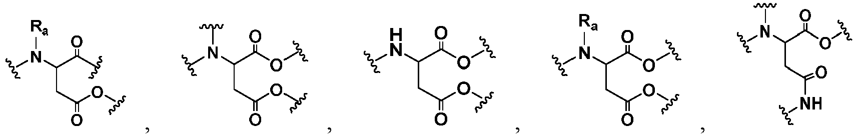 and

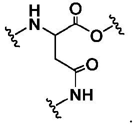

### 1.2. B₂

In the present application, B₂ is independently a linking bond or a C₁₋₂₀ alkylene group at each occurrence.

In a specific embodiment of the present application, B₂ is independently a linking bond or a C₁₋₂₀ alkylene group at each occurrence; the C₁₋₂₀ alkylene group is selected from the group consisting of a methylene group, an ethylene group, a propylene group, a butylene group, a pentylene group, a hexylene group, a heptylene group, an octylene group, a nonylene group, a decylene group, a undecylene group, a dodecylene group, a tridecylene group, a tetradecylene group, a pentadecylene group, a hexadecylene group, a heptadecylene group, an octadecylene group, a nonadecylene group and an eicosylene group; more preferably, B₂ is independently a linking bond or a C₂₋₁₀ alkylene group; the C₁₋₂₀ alkylene group and C₂₋₁₀ alkylene group are optionally substituted by 0 to 5 identical or different C₁₋₁₂ alkyl groups, cycloalkyl groups, heterocyclyl groups or hydroxyl groups; wherein, the C₁₋₁₂ alkyl group is preferably a methyl group, an ethyl group, or a propyl group.

### 1.3. R₁, R₂, R₃

### 1.3.1. R₁

In the present application, R₁ is a C₁₀₋₄₀ aliphatic hydrocarbon group containing one, two or more unsaturated bonds.

In one specific embodiment of the present application, R₁ is a C₁₀₋₄₀ linear aliphatic hydrocarbon group containing 1 to 3 unsaturated bonds, preferably a linear C₁₀₋₂₀ aliphatic hydrocarbon group containing one or two unsaturated bonds; more preferably, R₁ is selected from the group consisting of the following structures: 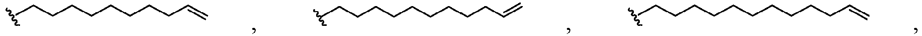 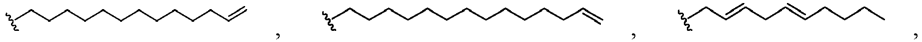 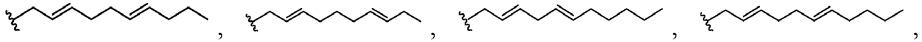 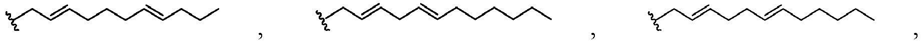 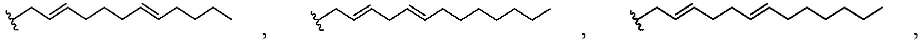 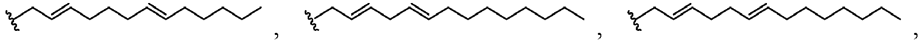 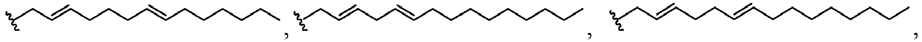 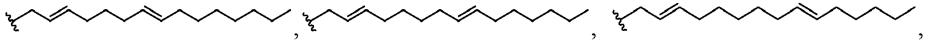 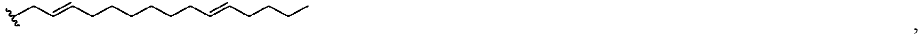 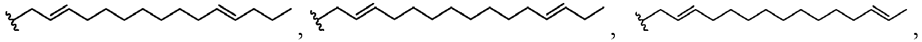 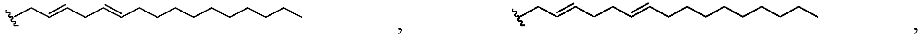 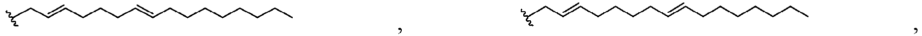 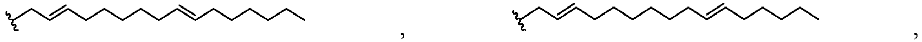 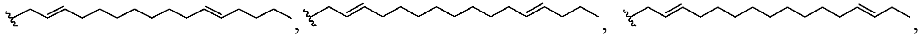 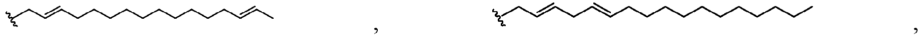 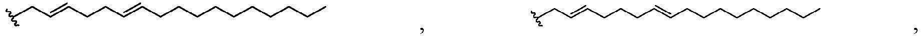 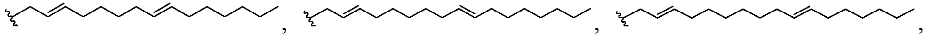 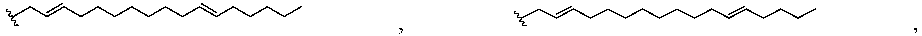 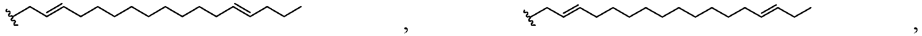 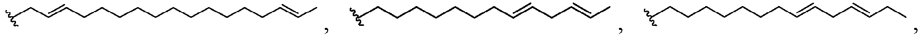 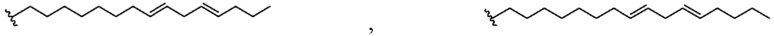 and

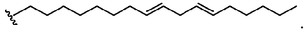

In a specific embodiment of the present application, R₁ is preferably a branched aliphatic hydrocarbon group containing two or more unsaturated bonds, preferably a branched C₁₅₋₄₀ aliphatic hydrocarbon group containing two or more unsaturated bonds; preferably, R₁ is selected from the group consisting of the following structures: 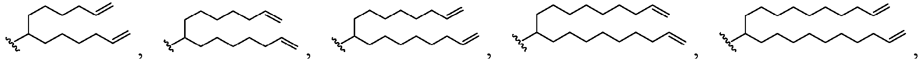 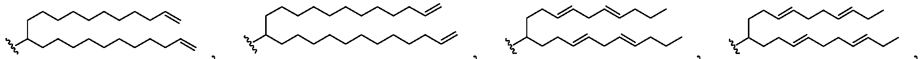 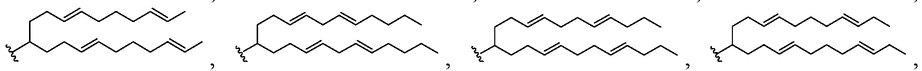 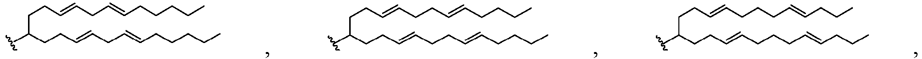 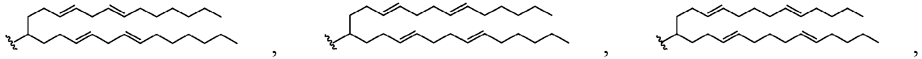 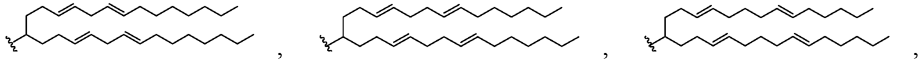 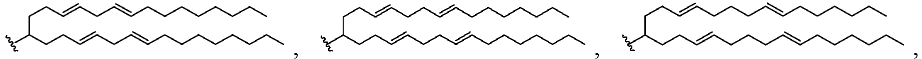 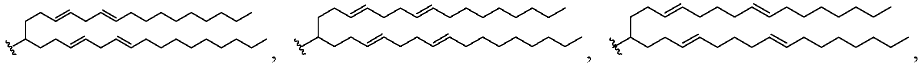 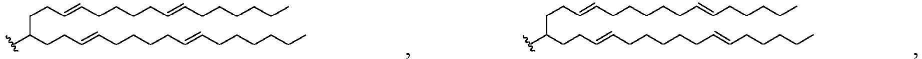 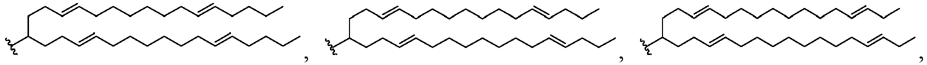 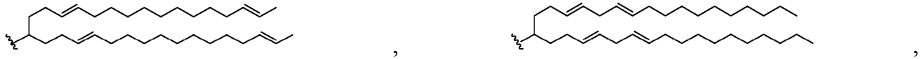 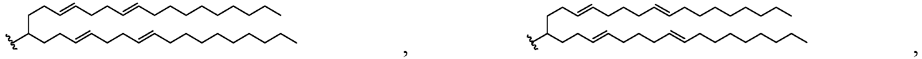 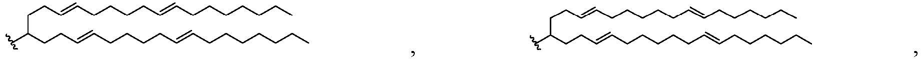 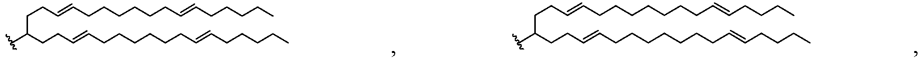 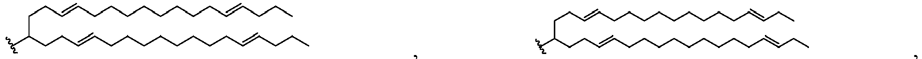 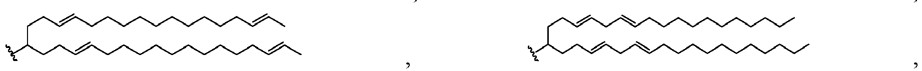 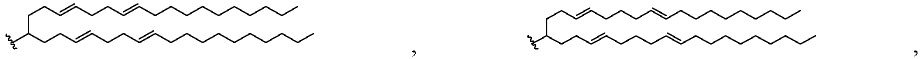 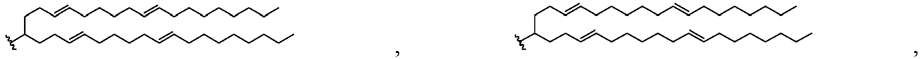 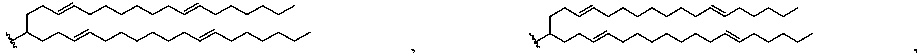 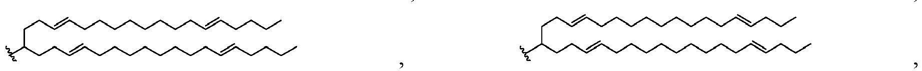 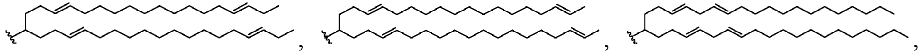 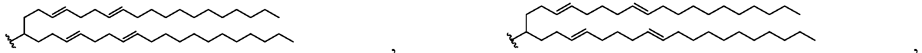 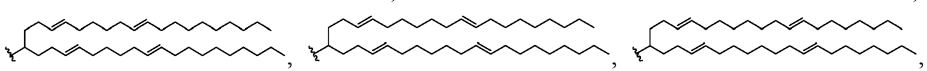 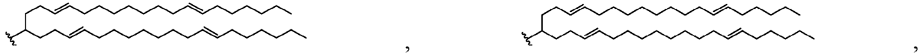 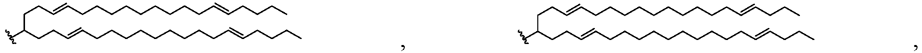 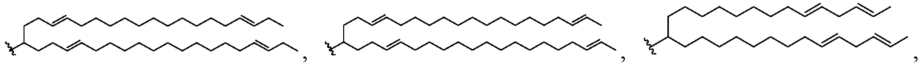 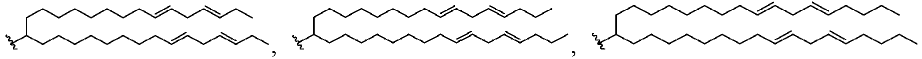 and

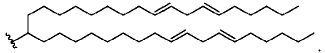

### 1.3.2. R₂

In the present application, R₂ is independently a linear alkyl group, a branched alkyl group, a linear alkenyl group, a branched alkenyl group, a linear alkynyl group, a branched alkynyl group, or 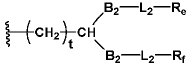 at each occurrence.

In a specific embodiment of the present application, the linear alkyl group is preferably a C₁₋₂₅ linear alkyl group, more preferably independently a C₁₋₁₇ linear alkyl group which is specifically selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group and a heptadecyl group.

In one specific embodiment of the present application, the branched alkenyl group or branched alkynyl group is represented as 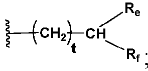 wherein, Rₑ and R_{f} are each independently a C₁₋₂₀ alkyl group, a C₂₋₂₀ alkenyl group or a C₂₋₂₀ group; t is an integer from 0 to 12; more preferably, Rₑ and R_{f} are each independently selected from the group consisting of a pentyl group, a hexyl group, a heptyl group, an octyl group, a nonyl group, a decyl group, an undecyl group, a dodecyl group, and an octadeca-6,9-dienyl group; more preferably, the branched alkyl group, branched alkenyl group or branched alkynyl group is selected from the group consisting of the following structures: 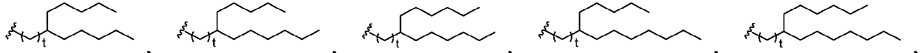 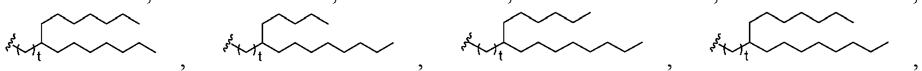 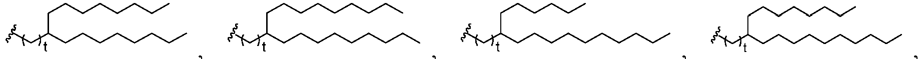 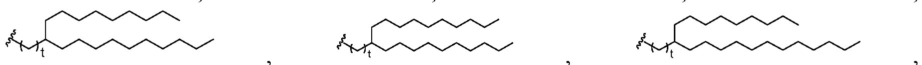 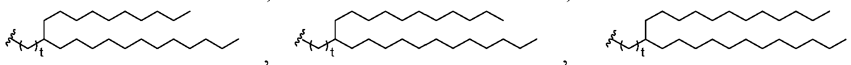 and

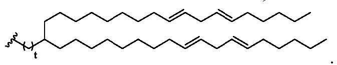

In one specific embodiment of the present application, 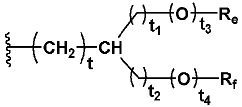 is selected from the group consisting of the following structures: 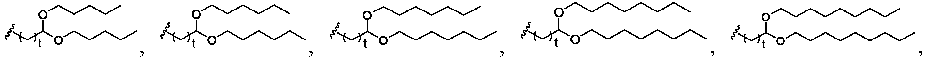 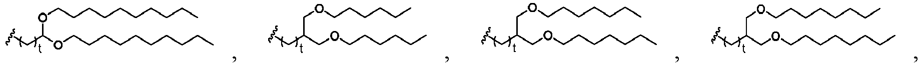 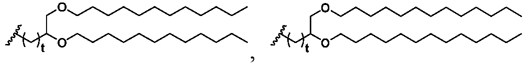 and

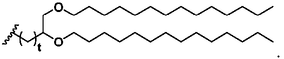

### 1.3.3. R₃

In the present application, R₃ is a hydrogen atom, an alkyl group, an alkoxy group, a C₃₋₆ carbocyclic group, a nitrogen-containing heterocyclyl group, -NR_{d}R_{d}, -SR_{d}, -C(=O)R_{d}, -C(=O)OR_{d}, -OC(=O)R_{d} or a functional group R₀₁ capable of interacting with bio-related substances; wherein, R_{d} is independently a C₁₋₁₂ alkyl group at each occurrence.

In a specific embodiment of the present application, R₃ is independently preferably selected from the group consisting of a hydrogen atom, -R_{d}, -OR_{d}, -NR_{d}R_{d}, -C(=O)R_{d}-, -C(=O)OR_{d}, -OC(=O)R_{d}, -OC(=O)OR_{d} and R₀₁ at each occurrence; the R₃ is more preferably independently of any of the following cases:
Case (1): R₃ is independently selected from the group consisting of a hydrogen atom, an alkyl group, an alkoxy group, -C(=O)OR_{d}, -OC(=O)R_{d}, -OC(=O)OR_{d}, an epoxy group, a hydroxyl group, a protected hydroxyl group, a sulfhydryl group, a protected sulfhydryl group, a carboxyl group, a protected carboxyl group, an amino group, a protected amino group, an aldehyde group, a protected aldehyde group, an active ester group, a carbonate group, a urethane groups, an isocyanate group, an isothiocyanate group, a succinimidyl group, a maleimide group, a protected maleimide group, a dimethylamino group, an alkenyl group, an enoate group, an azido group, a cyano group, a dithiopyridyl group, an α-haloacetyl alkynyl group, an alkynyl group, a folate group, a rhodamine group, a biotin group, a monosaccharide group, a polysaccharide group, 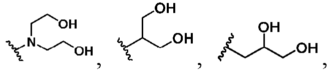 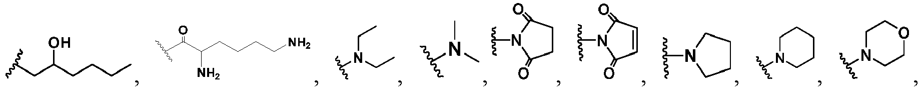 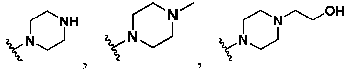 and 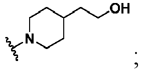
Case (2): R₃ is a functional group with therapeutic targeting properties; R₃ is preferably a residue of folic acid, N-acetylgalactosamine, or functional derivative thereof, more preferably selected from the group consisting of the following structures:

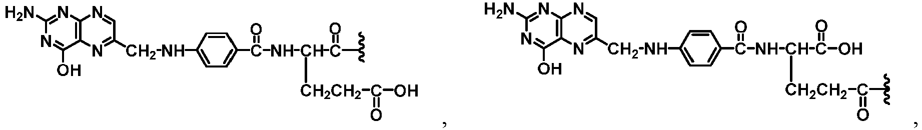

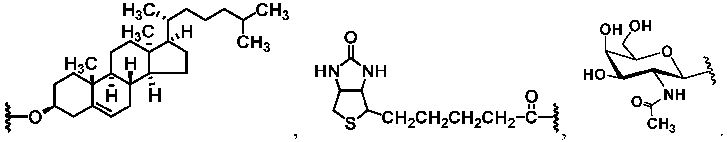

### 1.4.1. L₂

In the present application, L₂ is independently a linking bond or a divalent linking group at each occurrence.

In a specific embodiment of the present application, L₂ is preferably, independently selected from the group consisting of a linking bond, -OC(=O)-, -C(=O)O-, -OC(=O)O-, -C(=O)-, -O-, -NH-, -O(CR_{c}R_{c})ₛO-, -S-, -C(=O)S-, -SC(=O)-, -NR_{c}C(=O)-,-C(=O)NR_{c}-, -NR_{c}C(=O)NR_{c}-, -OC(=O)NR_{c}-, -NR_{c}C(=O)O-, -SC(=O)NR_{c}- and -NR_{c}C(=O)S- at each occurrence; wherein, R_{c} is independently a hydrogen atom or C₁₋₁₂ alkyl group at each occurrence, and s is an integer of 2 to 4; L₂ is more preferably selected from the group consisting of a linking bond, -OC(=O)-, -C(=O)O-, -OC(=O)O-, -C(=O)-, -O-, -O(CH₂)ₛO-, -S-, -C(=O)S-, -SC(=O)-, -NHC(=O)-, -C(=O)NH-, -NHC(=O)NH-, -OC(=O)NH-, -NHC(=O)O-, -SC(=O)NH- and -NHC(=O)S-; more preferably, L₂ is independently selected from the group consisting of a linking bond, -C(=O)-, -O-, -NH-, -OC(=O)-, -C(=O)O-, -NHC(=O)- and -C(=O)NH- at each occurrence.

### 1.4.2. L₃

In the present application, L₃ is independently a linking bond or a divalent linking group at each occurrence.

In a specific embodiment of the present application, L₃ is preferably, independently selected from the group consisting of a linking bond, -O-, -NH-, -C(=O)-, -(CH₂)ₜ-, -(CH₂)ₜO-, -O(CH₂)ₜ-, -(CH₂)ₜNH-, -(CH₂)ₜC(=O)-, -(CH₂)ₜO(CH₂)ₜ-, -C(=O)O(CH₂)ₜ-, -(CH₂)ₜC(=O)O- and -(CH₂)ₜOC(=O)- at each occurrence.

### 1.5. Fragments -L₃-R₃ and -B₂-L₂-R₂

### 1.5.1. -L₃-R₃

In a specific embodiment of the present application, the -L₃-R₃ fragment is preferably selected from the group consisting of the following structures: 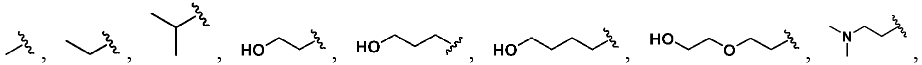 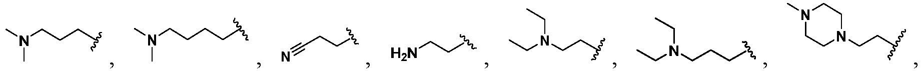 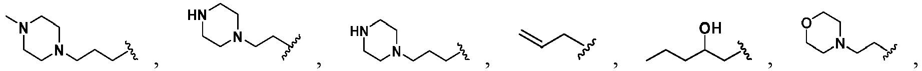 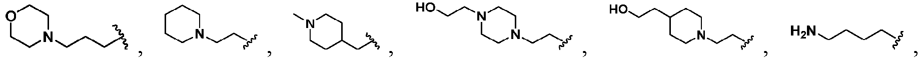 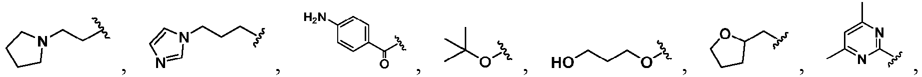 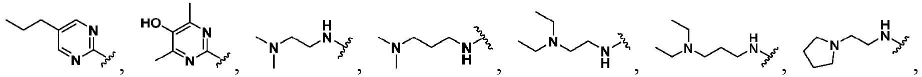 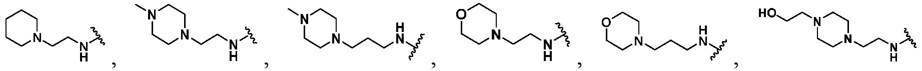 and

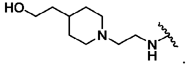

### 1.5.2. B₂-L₂-R₂

In one specific embodiment of the present application, -B₂-L₂-R₂ is preferably independently selected from the group consisting of the following structures at each occurrence: 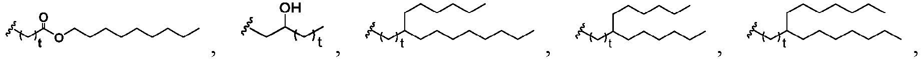 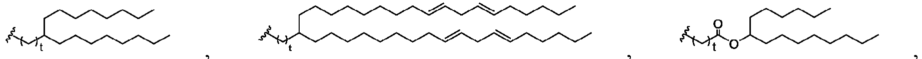 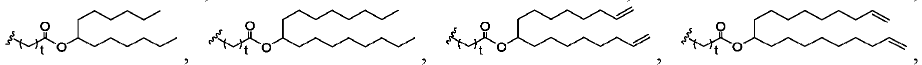 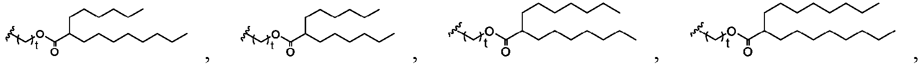 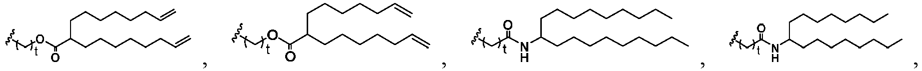 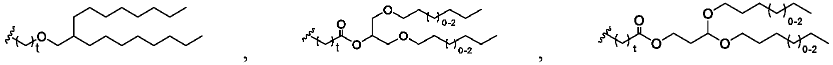 and

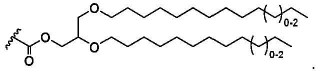

### 1.6. Examples of general structural formulas

In a specific embodiment of the present application, the structure of the amino acid-based cationic lipid is preferably selected from the group consisting of the following general formulas: 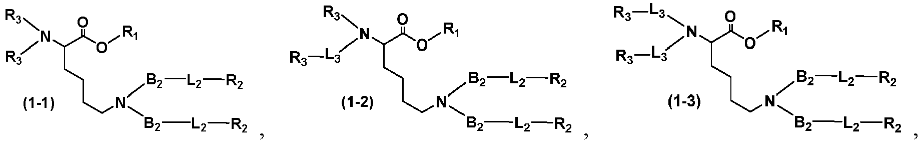 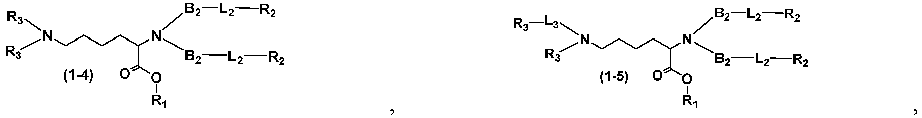 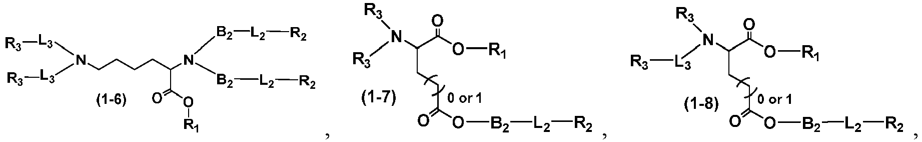 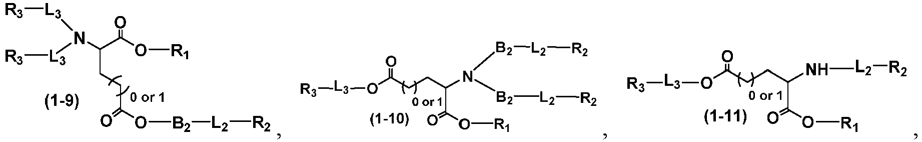 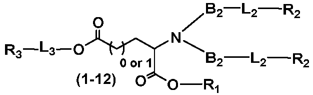 and 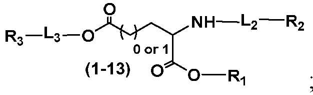 wherein, B₂, L₂ and L₃ are not linking bonds; more preferably, L₂ is independently selected from the group consisting of -C(=O)-, -C(=O)O-, -OC(=O)-, -OC(=O)O- -C(=O)NH-, -NHC(=O)-, -OC(=O)NH-, and -NHC(=O)O- at each occurrence; more preferably, L₂ is independently selected from the group consisting of -C(=O)-, -C(=O)O-, -OC(=O) -, and -OC(=O)O- at each occurrence.

### 1.7. Examples of specific structures

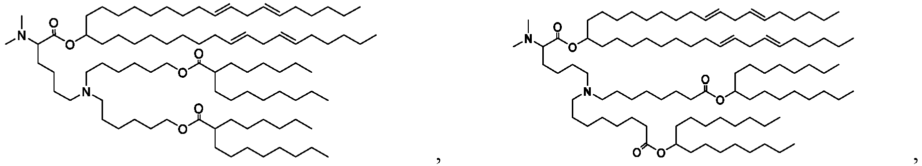

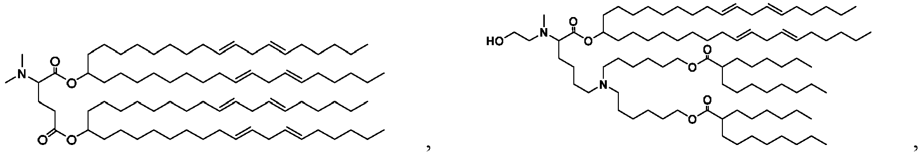

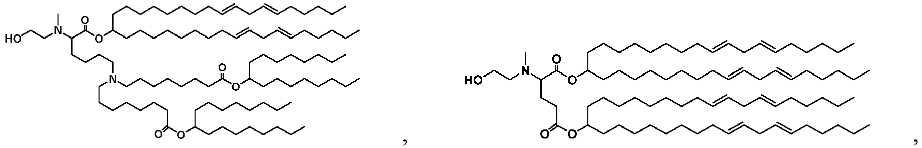

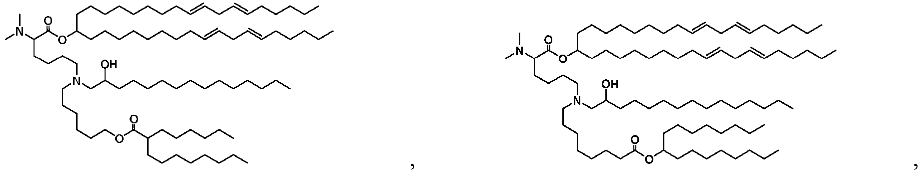

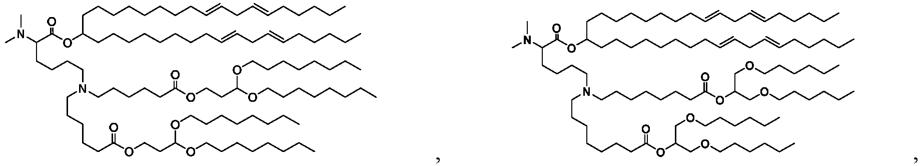

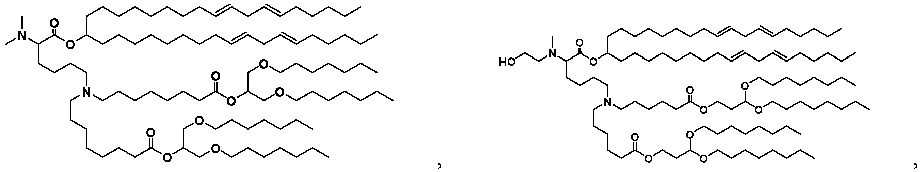

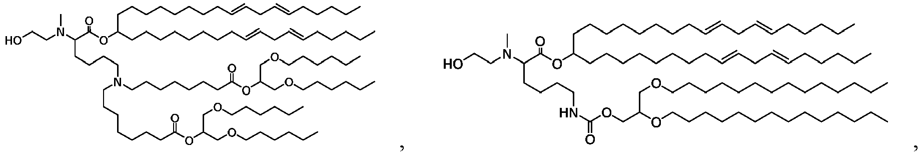

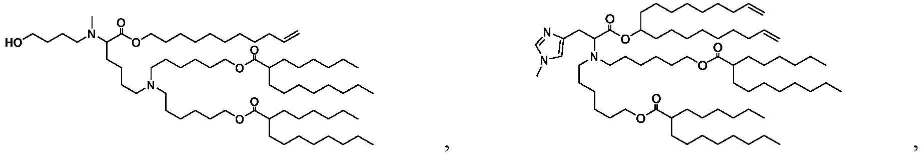

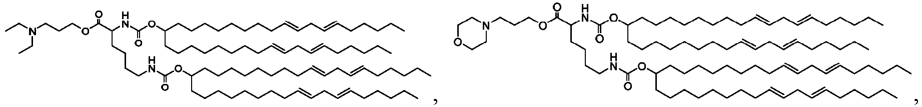

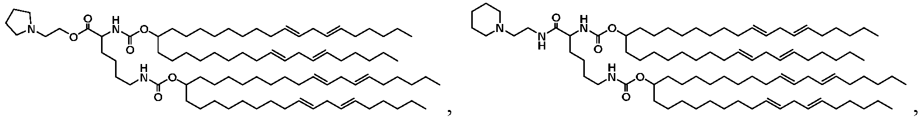

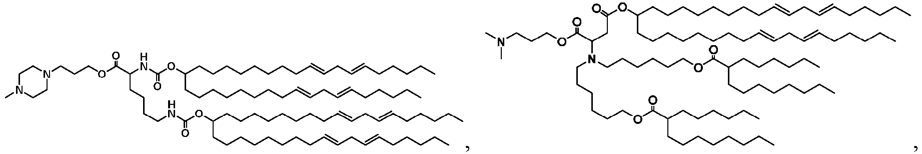

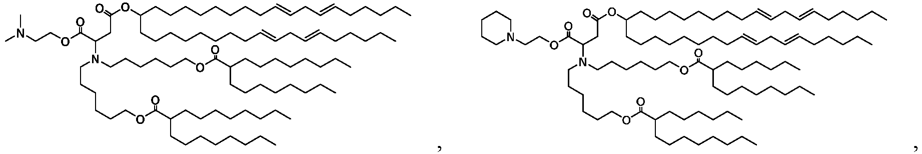

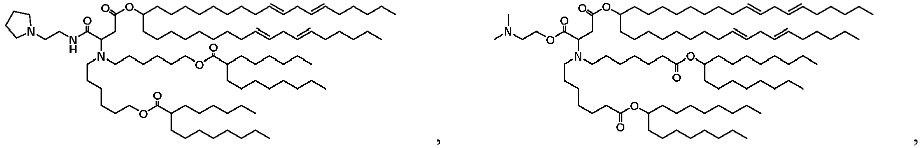

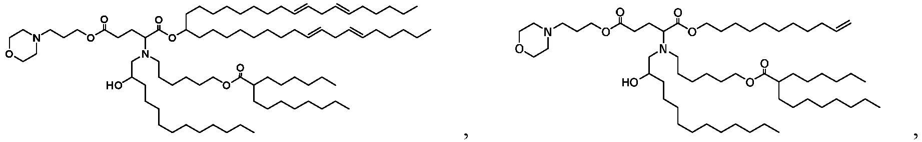

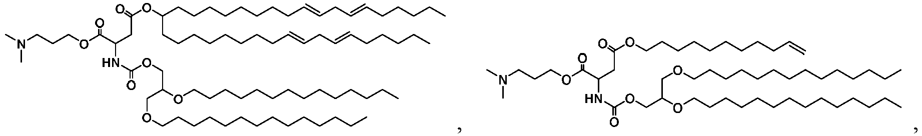

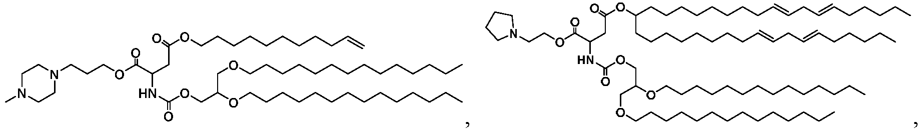

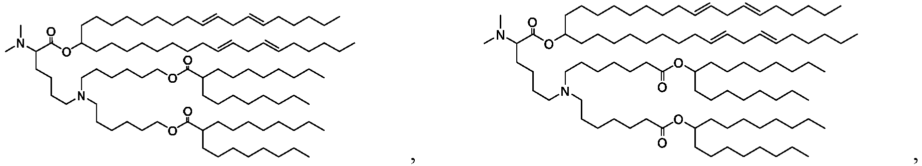

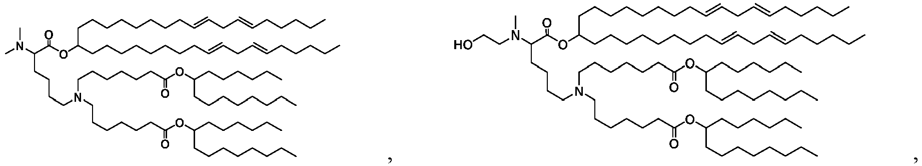

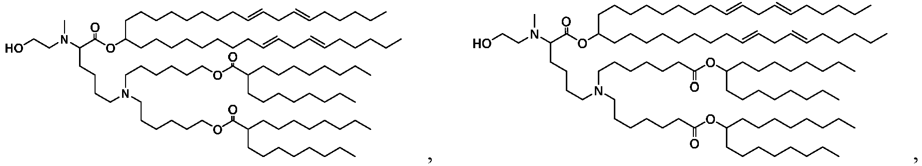

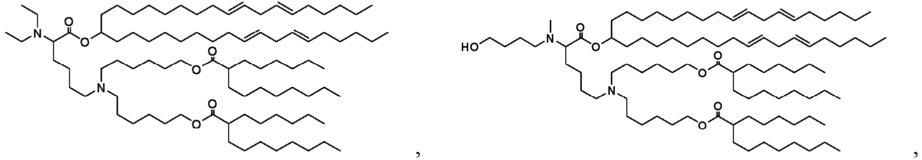

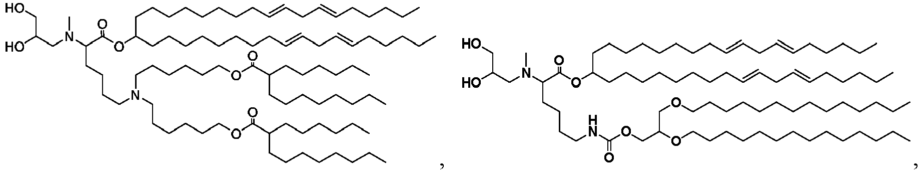

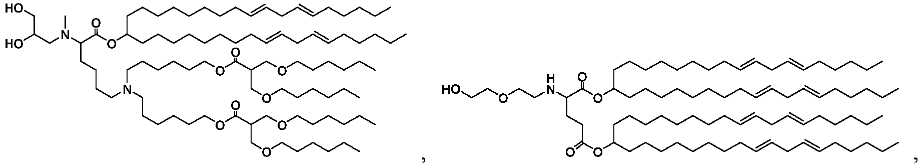

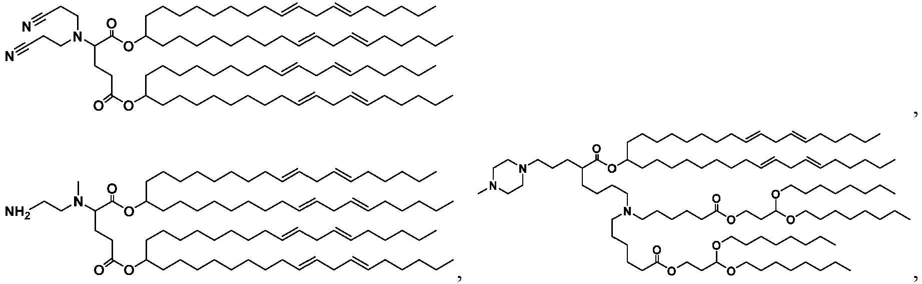

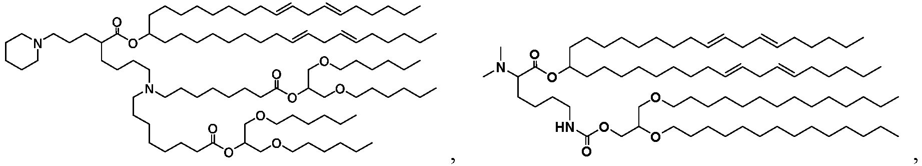

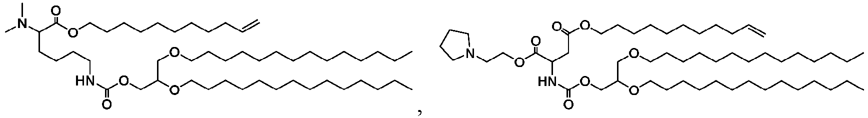

and

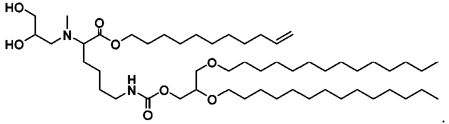

### 2. Preparation of amino acid-based cationic lipids

In the present application, the preparation of any amino acid-based cationic lipid described above may be prepared by methods including but not limited to the following:

### 2.1. Method-1:

Step 1: one molecule of A-1 (AA₁) is reacted with two molecules of the same or different A-2 (R₃'-F₁) to obtain a small molecule intermediate A-3 ( 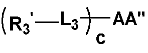 ) containing a divalent linking group L₃; wherein, the small molecule A-1 is an amino acid or an amino acid derivative containing 1 to 3 identical or different amino acidend groups, and the amino acid end groups are protected or unprotected, including but not limited to amino groups, carboxyl groups, hydroxyl groups, sulfhydryl groups, protected amino groups, protected carboxyl groups, protected hydroxyl groups, and protected sulfhydryl groups; the small molecule A-2 contains a reactive group F₁, which can react with the amino acid end group of AA₁ to obtain a divalent linking group L₃, preferably -OH, -COOH, -NH2, or -Br, etc., and the R₃' terminus contains a reactive group R₀₁ or a variant form with micro-modification containing R₀₁; the variant form with micro-modification refers to the structural form which can form the target reactive group after simple chemical reaction processes such as deprotection, salt complexation and decomplexation, ionization, protonation, deprotonation, and leaving group transformation. AA" is an amino acid derivative residue containing two identical or different amino acid end groups, and the amino acid end groups are protected or unprotected, including but not limited to an amino group, a carboxyl group, a hydroxyl group, a sulfhydryl group, a protected amino group, a protected carboxyl group, a protected hydroxyl group, and a protected sulfhydryl group. c is one or two, depending on the type of terminal group of AA₁ reacting with F₁ and the quantity of R₃'-F₁. When the terminal group of AA₁ reacting with F₁ is a carboxyl group or a hydroxyl group, c is one, and when it is an amino group, c is one or two; and, when c is two, the two -L₃-R₃' fragments can be the same or different, that is, when F₁ is reacting with the amino end group of AA₁, A-1 can react with different A-2 successively to obtain A-3 with c being two and two different -L₃-R₃' fragments, or one molecule of A-1 and two molecules of the same A-2 can react in a single-step or by a step-by-step reaction to obtain A-3 with c being two and two same -L₃-R₃' fragments;
Step 2: the small molecule A-4 (R₂-F₂) is reacted with the small molecule A-5 (F₃-B₂-F_{N}) to obtain a small molecule intermediate A-6 (R₂-L₂-B₂-F_{N}) containing a divalent linking group L₂, a reactive group F_{N} at one end and R₂ at the other end; wherein, the small molecule A-4 contains a reactive group F₂; the small molecule A-5 contains the heterofunctional pairs F₃ and F_{N}; F₂ can react with F₃ to obtain a divalent linking group L₂, and F_{N} is a reactive group that can react with amino groups, preferably -F, -Cl, -Br, or 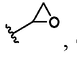 , etc.;
Step 3: one or two molecules of the same or different small molecule intermediate A-6 (R₂-L₂-B₂-F_{N}) is reacted with the small molecule intermediate A-3 ( 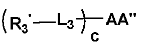 ) to obtain the amino acid derivative A-7 ( 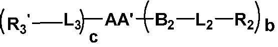 ); wherein, AA' is an amino acid derivative residue containing an amino acid end group, the amino acid end group is a protected or unprotected group, including but not limited to an amino group, a carboxyl group, a hydroxyl group, a sulfhydryl group, a protected amino group, a protected carboxyl group, a protected hydroxyl group, and a protected sulfhydryl group; b is one or two, depending on the type of the terminal group of AA" reacting with F_{N} and the quantity of R₂-L₂-B₂-F_{N}; when the terminal group of AA" reacting with F_{N} is a carboxyl group or a hydroxyl group, b is one, and when it is an amino group, b is one or two; when b is two, the two -L₆-B₂-L₂-R₂ fragments can be the same or different, that is, when F_{N} reacts with the amino end group of AA", A-3 can react with different A-6 successively to obtain A-7 with b being two and two different -L₆-B₂-L₂-R₂ fragments, or one molecule of A-3 and two same molecules of A-6 can undergo a single-step or stepwise reaction to obtain A-7 with b being two and two same -L₆-B₂-L₂-R₂ fragments;
Step 4: the amino acid derivative A-7 ( 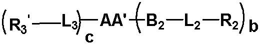 ) is reacted with the small molecule intermediate A-8 (R₁-OH, the small-molecule alcohol derivative obtained by the reaction of the bromide R₁-Br with metallic magnesium under anhydrous, oxygen-free conditions, also be available by purchase) to obtain the amino acid-based cationic lipid A-9' 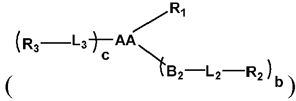 ; AA is an amino acid or amino acid derivative residue;

when R₃' is equal to R₃, the resulting structure A-9' corresponds to the structure represented by the general formula (1);
when R₃' is not equal to R₃, A-9' is micro-modified at the terminal group to obtain A-9, which corresponds to the structure represented by the general formula (1); the terminal micro-modification is selected from the following chemical reactions: deprotection, salt complexation and decomplexation, ionization, protonation, deprotonation, leaving group transformation;
wherein, the definitions of L₂, L₃, B₂, R₃, R₁, R₂, b, and c are consistent with those described in general formula (1), and will not be repeated here.

The aforementioned small-molecule starting materials A-1, A-2, A-3, A-4, A-5, A-8, etc., may be obtained by purchase or by synthesis; for example, the small molecule A-3 in Example 4 is 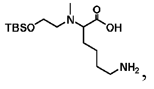 which can be synthesized by using N-methyllysine 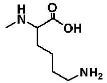 and TBS-protected bromoethane 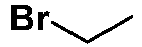 as starting materials; the small molecule A-3 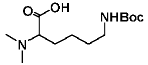 in Example 1 can be obtained by purchase and then by protection of amino group with Boc, and the Step 1 can be omitted.
Step 1 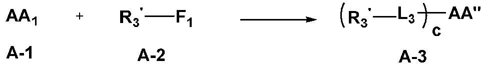
Step 2 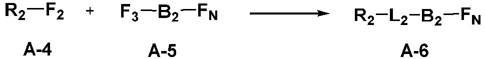
Step 3 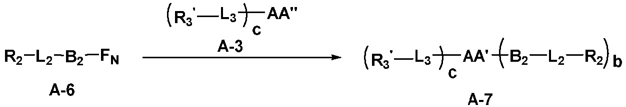
Step 4

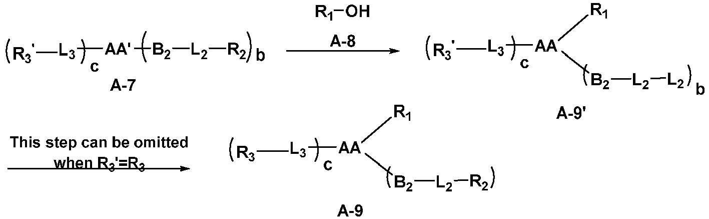

In this method, the two amino acid end groups contained in the AA" of A-3 ( 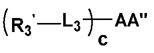 ) of Step 3 can also react with A-8 (R₁-OH) at the same time to obtain A-10'; meanwhile, both L₂ and B₂ are linking bonds, R₁ and R₂ are the same, and the definitions of L₂, L₃, B₂, R₃, R₁, R₂, b, and c are consistent with those described in the general formula (1), and will not be repeated here;
when R₃' is equal to R₃, the resulting structure A-10' corresponds to the structure represented by the general formula (1);
when R₃' is not equal to R₃, A-10' is micromodified at the end to obtain A-10, which corresponds to the structure shown in general formula (1); the terminal micro-modification is selected from the following chemical reactions: deprotection, salt complexation and decomplexation, ionization, protonation, deprotonation, leaving group transformation.

In the aforementioned preparation steps, a reaction involving multiple molecules can be carried out in a single step or in steps.

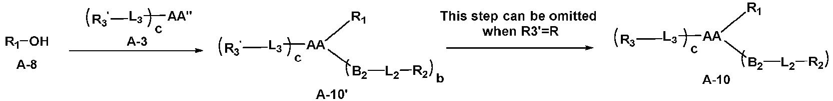

The R₂ of the reaction starting material R₂-F₂ in the aforementioned preparation method can be an aliphatic hydrocarbon derivative residue 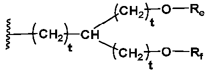 ; wherein, t is independently an integer of 0 to 12 at each occurrence; Rₑ and R_{f} are each independently a C₁₋₁₅ alkyl group, a C₂₋₁₅ alkenyl group or a C₂₋₁₅ alkynyl group. More specifically, R₁-F₁ can be 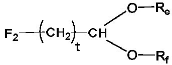 , which can be obtained through purchase or synthesis, and the synthesis may utilize the aldehyde alcohol addition, such as the addition reaction between one molecule of 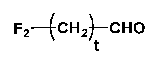 and two molecules of Rₑ-OH, which obtains 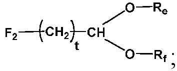 wherein Rₑ and R_{f} are the same; R₁-F₁ can also be 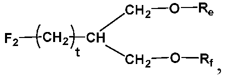 which can be obtained through purchase or by a reaction between 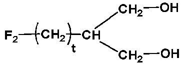 and a related alkylating reagent; wherein the alkylating reagent is preferably a halide; for example, 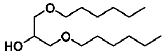 can be obtained by the reaction between one molecule of glycerol with a TBS-protected hydroxyl group and two molecules of bromohexane and then deprotection.

### 2.1.1. Specific methods

### 2.1.1.1. Specific method-1:

Step 1: one molecule of A-1 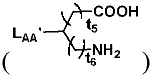 is reacted with one or two molecules of the same or different A-2 (R₃'-F₁) to obtain a small molecule intermediate A-3 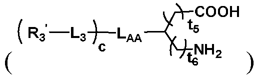 containing a divalent linking group L₃; wherein the small molecule A-1 is an amino acid or amino acid derivative containing two or three identical or different amino acid end groups; the amino acid end groups are protected or unprotected, including but not limited to an amino group, a carboxyl group, a hydroxyl group, a sulfhydryl group, a protected amino group, a protected carboxyl group, a protected hydroxyl group, and a protected sulfhydryl group; L_{AA}' is an amino acid derivative residue containing one amino acid end group; the amino acid end group is protected or unprotected, including but not limited to an amino group, a carboxyl group, a hydroxyl group, a sulfhydryl group, a protected amino group, a protected carboxyl group, a protected hydroxyl group, and a protected sulfhydryl group; the small molecule A-2 contains a reactive group F₁ which can react with the amino acid end group of L_{AA}' to obtain a divalent linking group L₃, preferably -OH, -COOH, -NH₂, or -Br, etc., and the R₃' end contains a reactive group R₀₁ or a variant form with micro-modification of R₀₁; the variant form with micro-modification refers to a group that can be transformed into R₀₁ after any chemical process of deprotection, salt complexation and decomplexation, ionization, protonation, deprotonation, or leaving group transformation; L_{AA} is -NH(CH₂)ₘ- , -OC(=O)(CH₂)ₜ₅-, or -NHC(=O)(CH₂)ₜ₅-; the left side of L_{AA} is connected to L₃, m is an integer of 0 to 4, and t₅ and t₆ are integers of 0 to 2; A-3 can be 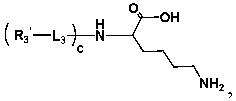 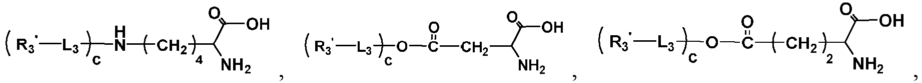 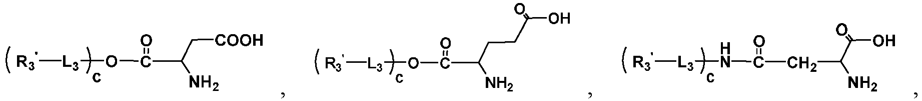 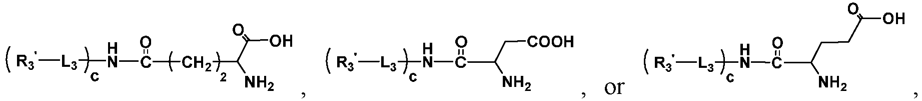 etc., and its amino terminus and carboxyl terminus may be protected or unprotected, such as S1-3 in Example 1, S4-1 in Example 4, S14-1 in Example 14, etc.; c is one or two, depending on the type of the terminal group of L_{AA}' that reacts with F₁ and the quantity of R₃'-F₁; when the terminal group of L_{AA}' reacting with F₁ is a carboxyl group or a hydroxyl group, c is one, and when it is an amino group, c is one or two; and, when c is two, the two -L₃-R₃' fragments can be the same or different; that is, when F₁ is reacting with amino end group of L_{AA}', A-1 can react with different A-2 successively to obtain A-3 with c being 2 and two different -L₃-R₃' fragments, or one molecule of A-1 can react with two molecules of A-2 in a single step or multiple steps to obtain A-3 with c being 2 and two same -L₃-R₃' fragments;
Step 2: amino acid derivative A-3 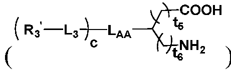 undergoes an esterification reaction with one molecule of alcohol derivative A-4 (R₁-OH) to obtain intermediate A-5 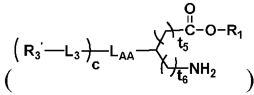 ; wherein, A-4 can be obtained from the bromide R₁-Br under the action of metallic magnesium or by purchase, such as S1-2 in Example 1, S14-4 in Example 14, S15-3 in Example 15, etc.;
Step 3: one or two molecules of the same or different bromide A-6 ( 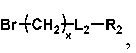 , x is an integer of 0 to 7, e.g., S1-6 in Example 1, S7-1 and S1-6 in Example 7, S2-3 in Example 8, etc.) or succinimide derivative A-7 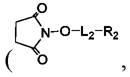 such as S13-3 in Example 13) is reacted with A-5 to obtain amino acid lipids A-8' 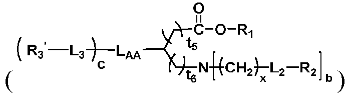 or A-9' 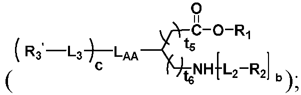 wherein, b is 2, and two -(CH₂)ₓ-L₂-R₂ fragments may be the same or different, which depends on the quantity and type of A-6 that reacts with A-5; that is, if A-5 reacts with different A-6 successively, the A-8' containing two different -(CH₂)ₓ-L₂-R₂ fragments will be obtained, and if A-5 reacts with two identical molecules of A-6 in a single step or steps, the A-9' containing two same -(CH₂)ₓ-L₂-R₂ fragments will be obtained;
The R₂ in the reaction starting material A-6 in the aforementioned preparation method can be an etherified aliphatic hydrocarbon derivative residue 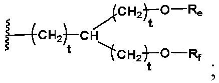 wherein, t is independently an integer of 0 to 12 at each occurrence; Rₑ and R_{f} are each independently a C₁-C₁₅ alkyl group, a C₂-C₁₅ alkenyl group or a C₂-C₁₅ alkynyl group. More specifically, R₁-F₁ can be 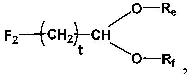 which can be obtained through purchase or synthesis, and the synthesis can utilize the aldehyde alcohol addition, such as the addition reaction between one molecule of 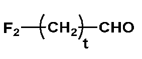 and two molecules of Rₑ-OH, which leads to the formation of 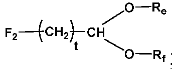 wherein, Rₑ and R_{f} are the same; for example, S9-5 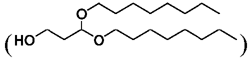 in Example 9 is obtained by the reaction of a molecule of 3-hydroxypropionaldehyde containing a TBS-protected hydroxyl group and two molecules of 1-octanol and then by deprotection; R₁-F₁ may also be 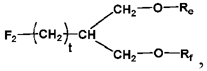 which can be obtained through purchase or synthesis, and the synthesis may include the reaction between 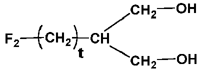 and a related alkylating reagent; wherein the alkylation reagent is preferably a halide, e.g., S10-4 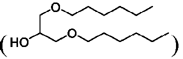 in Example 10 is obtained by the reaction between one molecule of glycerol containing a TBS-protected hydroxyl group and two molecules of bromohexane and then deprotection.

When R₃' is equal to R₃, the resulting structure A-8' or A-9' corresponds to the structure represented by the general formula (1); wherein, 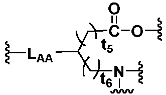 corresponds to the AA core of the general formula (1);
when R₃' is not equal to R₃, A-8' or A-9' is modified at the end to obtain the structure of A-8 or A-9 corresponding to general formula (1); the terminal micro-modification is selected from the following chemical reactions: deprotection, salt complexation and decomplexation, ionization, protonation, deprotonation, and leaving group transformation;
wherein, the definitions of L₃, R₃, R₁, R₂, b, and c are consistent with those described in general formula (1), and will not be repeated here. The aforementioned small-molecule starting materials A-1, A-2, A-3, A-4, A-6, etc. may be obtained by purchase or by synthesis; for example, the small molecule A-3 in Example 4 is 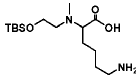 , which can be synthesized by using N-methyllysine 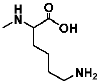 and bromoethane 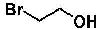 with a TBS-protected hydroxyl group as starting materials; the small molecule A-3 in Example 1 is 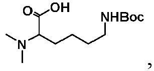 which can be obtained by protection of an amino group with Boc after purchase, and the Step 1 can be omitted.
   Step 1 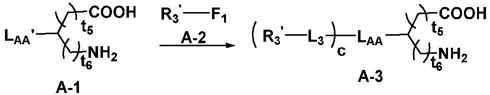
   Step 2 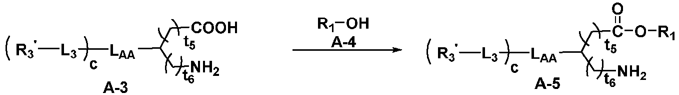
   Step 3

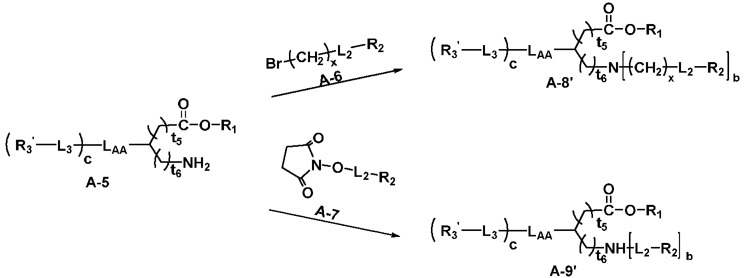

### 2.1.1.2. Specific method-2:

Step 1: one molecule of B-1 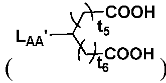 is reacted with one or two molecules of the same or different B-2 (R₃'-F₁) to form a small molecule intermediate B-3 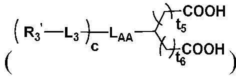 containing a divalent linking group L₃; wherein the small molecule B-1 is an amino acid or amino acid derivative containing 2 or 3 identical or different amino acid end groups, and the amino acid end groups are protected or unprotected, including but not limited to an amino group, a carboxyl group, a hydroxyl group, a protected amino group, a protected carboxyl group, and a protected hydroxyl group; L_{AA}' is an amino acid derivative residue containing one amino acid end group, and the amino acid end group is protected or unprotected, including but not limited to an amino group, a carboxyl group, a hydroxyl group, a protected amino group, a protected carboxyl group, and a protected hydroxyl group; the small molecule B-2 contains a reactive group F₁ which can react with the amino acid end group of L_{AA}' to form a divalent linking group L₃, preferably -OH, -COOH, -NH₂, or -Br, etc., and the R₃' terminus contains a reactive group R₀₁ or a variant form with micro-modification containing R₀₁; the variant form with micro-modification refers to a group that can be transformed into R₀₁ after any chemical process of deprotection, salt complexation and decomplexation, ionization, protonation, deprotonation, or leaving group transformation; L_{AA} is -NH-, and the left side of L_{AA} is connected to L₃; B-3 can be 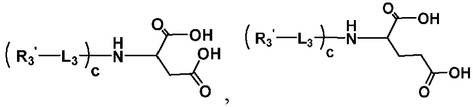 , ..., and the carboxyl termial group of B-3 may be protected or unprotected, such as S3-1 in Example 3, S6-1 in Example 6, etc.; whether c is one or two depends on the type of the terminal group of L_{AA'} reacting with F₁ and the quantity of R₃'-F₁; if it is a secondary amino group of L_{AA}' that reacts with F₁, then c is 1, and if it is a primary amino group, then c is one or two; and, if c is 2, the two -L₃-R₃' fragments can be the same or different, that is, if F₁ reacts with a primary amino group of L_{AA}', B-1 can react with different B-2 successively to obtain B-3 containing two different -L₃-R₃' fragments with c being 2, or one molecule of B-1 may react with two molecules of the same B-2 in a single step or multiple steps to obtain B-3 containing two same -L₃-R₃' fragments with c being 2;
Step 2: the amino acid derivative B-3 ( 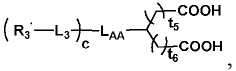 such as S1-3 in Example 3, S6-1 in Example 6, etc.) and two molecules of alcohol derivative B-4 (R₁-OH) undergo esterification reaction to obtain an amino acid lipid B-5' 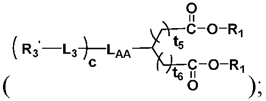 wherein, B-4 can be generated from the bromide R₁-Br under the action of metallic magnesium or can be obtained by purchase, such as S1-2 in Example 3;
   when R₃' is equal to R₃, the resulting structure B-5' corresponds to the structure represented by the general formula (1); wherein, 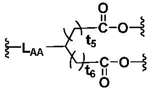 corresponds to the AA core of the general formula (1), R₁ is the same as R₂, and b is 1;
   when R₃' is not equal to R₃, B-5' is micro-modified at the end to obtain the structure of B-5 corresponding to general formula (1); the terminal micro-modification is selected from the following chemical reactions: deprotection, salt complexation and decomplexation, ionization, protonation, deprotonation, leaving group transformation;
   wherein, the definitions of L₃, R₃, R₁, R₂, and c are consistent with those described in general formula (1), which will not be repeated here. The aforementioned small-molecule starting materials B-1, B-2, B-3, B-5, etc. may be obtained by purchase or by synthesis; for example, the small molecule B-3 in Example 6 is 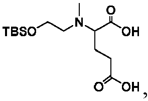 which can be synthesized autonomously by using N-methylglutamic acid and bromoethane 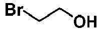 with a TBS-protected hydroxyl group as starting materials; the small molecule B-3 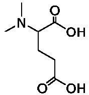 in Example 3 is available by purchase, and the Step one can be omitted.
      Step 1 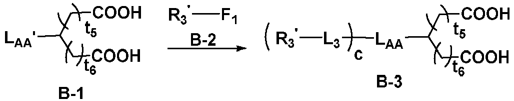
      Step 2

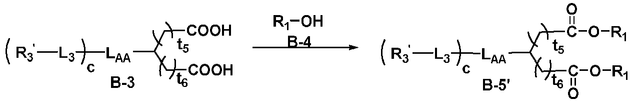

### 2.2. Description of relevant starting materials and/or steps in the preparation process

### 2.2.1. Condensing agent, oxidizing agent, and reducing agent

In the present application, the condensing agent is not particularly limited, preferably *N*,*N*-dicyclohexylcarbodiimide (DCC), 1-ethyl-(3-dimethylaminopropyl)carbodiimide hydrochloride (EDC·HCl), 2-(7-azobenzotriazole-*N*,*N*,*N'*,*N'*-tetramethyluronium hexafluorophosphate (HATU), or 2-(1H-benzotriazol-1-yl)-1,1,3,3-tetramethyluronium hexafluorophosphate (HBTU), and most preferably DDC. Generally, the molar equivalent of the condensing agent is 1 to 20 folds of that of the carboxylic acid, preferably 5 to 10 folds, and suitable catalysts such as 4-dimethylaminopyridine can be added to the reaction.

In the present application, the oxidizing agent used in reactions is not particularly limited as long as it is a compound or a combination of multiple compounds capable of increasing the valence of the substrate, preferably phenyliodine(III) bis(trifluoroacetate), 1,4-benzoquinone, benzyl trimethyl ammonium tribromide, pyridinium dichromate, ozone, oxygen, hydrofluoric acid, sodium hypochlorite, cobaltic acetate, cobalt acetate, manganous acetate, palladium(II) acetate, cupric acetate, monoperoxyphthalic acid, iodine, N-iodosuccinimide, iodoxybenzene, 2-iodylbenzoic acid, dimethyldioxirane, dimethyl sulfoxide-oxalyl chloride, DDQ, dichlorotris(triphenylphosphine)ruthenium, manganese dioxide, (diacetoxyiodo)benzene, periodic acid, sodium periodate, sodium periodate-osmium tetraoxide, potassium permanganate, sodium perborate, perbenzoic acid, dibenzoyl peroxide, nickel peroxide, hydrogen peroxide, cumyl hydroperoxide, 1-butyl hydroperoxide, peracetic acid, *m*-chloroperbenzoic acid, *N*-chlorosuccinimide, pyridinium chlorochromate, palladium chloride-cupric chloride, urea hydrogen peroxide adduct, triphenylcarbenium tetrafluoroborate, tributyltin oxide, cobalt trifluoride, vanadium oxytrifluoride, chromium trioxide, manganese triacetate, TEMPO, diammonium cerium nitrate, bromine, pyridine *N*-oxide, silver oxide, *O*-ethylperoxycarbonic acid, manganese acetyllacetonate, vanadyl acetylacetonate, aluminium isopropoxide, peroxymonosulfate, dichloroiodobenzene, the like, or any combination thereof, and more preferably oxygen, sodium hypochlorite, hydrogen peroxide, dichloroiodobenzene, peroxymonosulfate, the like, or any combination thereof; the molar equivalent of the oxidizing agent is 1 to 50 folds of that of the hydroxyl group of the intermediate compound, preferably 1 to 20 folds, and more preferably 5 to 10 folds.

In the present application, the reducing agent is not particularly limited as long as it can reduce the Schiff base formed by the reaction of an amine with an aldehyde or ketone to an amino group, preferably sodium borohydride, sodium cyanoborohydride, lithium aluminum hydride, borane, diborane, diisobutylaluminum hydride, diisopinocampheylborane, lithium borohydride, zinc borohydride, borane-pyridine, borane-methyl sulfide, borane-tetrahydrofuran, the like, or any combination thereof, and more preferably sodium cyanoborohydride; the molar equivalent of the reducing agent is 1 to 50 folds of that of the amino group to be modified, preferably 1 to 20 folds, and more preferably 5 to 10 folds.

In the present application, the reaction temperature is 0 to 200 °C, preferably 0 to 100 °C, and more preferably 0 to 25 °C; the reaction time is preferably 10 min to 48 h, and more preferably 30 min to 24 h. The obtained product can be purified by purification methods such as extraction, recrystallization, adsorption treatment, precipitation, reverse precipitation, membrane dialysis, and supercritical extraction.

In the present application, the reaction solvent can be absent or an aprotic solvent including toluene, benzene, xylene, acetonitrile, ethyl acetate, diethyl ether, tert-butyl methyl ether, tetrahydrofuran, chloroform, dichloromethane, dimethylsulfoxide, dimethylformamide, and dimethylacetamide; the aprotic solvent is preferably tetrahydrofuran, dichloromethane, dimethylsulfoxide, or dimethylformamide.

In the present application, the bases used in reactions are inorganic bases or organic bases, preferably organic bases (such as triethylamine, pyridine, 4-dimethylaminopyridine, imidazole or diisopropyl ethylamine), preferably triethylamine or pyridine. The molar equivalent of base is 1 to 50 folds of that of carboxylic acids, preferably 1 to 10 folds, and more preferably 2 to 3 folds.

### 2.2.2. "Protection" and "deprotection" of relevant groups involved in the reaction process

In the present application, the reaction process also involves the "protection" and "deprotection" processes of relevant groups. To prevent a functional group from affecting the reaction, the functional group is usually protected. In addition, when there are two or more functional groups and only the target functional group needs to react, the other functional groups should therefore be protected. The protecting group not only protects the functional group stably but also needs to be removed easily as needed. Therefore, in organic synthesis, it is important to remove only the protecting group bonded to the specified functional group under appropriate conditions.

In the present application, the definitions of "carboxyl protection group" and "amino protection group" are consistent with those in the "Detailed description of the application" section, and will not be repeated herein.

In the present application, the hydroxyl groups protected by hydroxyl protecting groups are not particularly limited, e.g., alcoholic hydroxyl groups, phenolic hydroxyl groups, and the like. The amino groups protected by amino protecting groups are not particularly limited, such as those from primary amines, secondary amines, hydrazines, and amides. Amino groups in the present application are not particularly limited, including but not limited to primary amino groups, secondary amino groups, tertiary amino groups, and quaternary ammonium ions.

In the present application, the deprotection of protected hydroxyl groups is related to the types of hydroxyl protecting groups. The types of hydroxyl protecting groups are not particularly limited; for example, benzyl groups, silyl ethers, acetals, and tert-butyl groups can be used to protect terminal hydroxyl groups, and the corresponding deprotection methods include the following:

### A: Deprotection of benzyl protecting groups

The deprotection of benzyl groups can be achieved via hydrogenation using a hydrogenative reducing agent and a hydrogen donor. As used herein, the water content should be less than 1% to facilitate the reaction.

The hydrogenative reduction catalyst is not particularly limited, preferably palladium or nickel, and its carrier is not particularly limited, preferably alumina or carbon, and more preferably carbon. The amount of palladium used is 1 to 100 wt% of that of compounds containing protected hydroxyl groups, preferably 1 to 20 wt%.

The reaction solvent is not particularly limited, as long as it allows the reagents and the products to be dissolved. Preferable solvents include methanol, ethanol, ethyl acetate, tetrahydrofuran, and acetic acid, wherein methanol is preferable. The hydrogen donor is not particularly limited, preferably hydrogen gas, cyclohexene, 2-propanol, ammonium formate, or the like. The reaction temperature is preferably 25 to 40 °C. The reaction time is not particularly limited, which is negatively correlated with the amount of catalyst used and preferably 1 to 5 hours.

### B: Deprotection of silyl ether protecting group

Compounds used for this type of hydroxyl protection include trimethylsilyl ether, triethylsilyl ether, tert-butyldimethylsilyl ether, tert-butyldiphenylsilyl ether, and the like. The deprotection of such silyl ethers uses compounds containing fluoride ions, wherein the compound is preferably tetrabutylammonium fluoride, tetraethylammonium fluoride, hydrofluoric acid, or potassium fluoride, and more preferably tetrabutylammonium fluoride or potassium fluoride. The amount of the fluorine-containing reagent is 5 to 20 folds of that of the protected hydroxyl group and preferably 8 to 15 folds of that of the initiator. When the amount of the fluorine-containing reagent used is less than 5 times the molar equivalents of protected hydroxyl groups, the deprotonation might not be complete. When the amount of deprotecting reagent used exceeds 20 times the molar equivalents of the protected hydroxyl groups, the excess reagent or compound tends to cause difficulty in the purification process and result in side reactions in subsequent steps. The reaction solvent is not particularly limited as long as it can dissolve the reagents and the products, preferably an aprotic solvent, and more preferably tetrahydrofuran or dichloromethane. The reaction temperature is preferably 0 to 30 °C; when it is lower than 0 °C, the reaction rate is relatively slow, and the protecting group cannot be completely removed.

### C: Deprotection of t-butyl protecting groups

The deprotection of tert-butyl groups is carried out under an acidic condition, and the pH of the solution is preferably 0 to 4. The acid is not particularly limited but is preferably acetic acid, phosphoric acid, sulfuric acid, hydrochloric acid, or nitric acid, and more preferably hydrochloric acid. The reaction solvent is not particularly limited as long as it can dissolve the reagents and the products, preferably water. The reaction temperature is preferably 0 to 30 °C.

### 2.2.3. Alkylation reaction

In the present application, the alkylation reactions are preferably those based on hydroxyl groups, sulfhydryl groups, or amino groups, corresponding to the formation of ether bonds, thioether bonds, and secondary or tertiary amino groups, respectively. Specific examples are as follows:

### 2.2.3.1. Alkylation reaction of substrate alcohols with sulfonates or halides

The amine intermediate can be obtained via the nucleophilic substitution of the substrate alcohol with a sulfonate derivative or a halide under a basic condition. Wherein, the amount of the sulfonate or halide is 1 to 50 and preferably 1 to 5 molar equivalents per molar equivalent of the substrate alcohol. When the amount of the sulfonate or halide is less than one molar equivalent per molar equivalent of the substrate alcohol, the substitution may not be complete, causing difficulty in the purification process. When the amount of the sulfonate or halide exceeds 50 times the molar equivalents of the substrate alcohol, the excess reagents can cause problems with purification and can blend into subsequent steps, resulting in increased side reactions and making the purification difficult.

The resulting product is a mixture of ether intermediate and excess sulfonate or halide, and can be purified by anion exchange resin, permeation, ultrafiltration, etc. Wherein, the anion exchange resin is not particularly limited as long as the target product can undergo ion exchange and adsorption on the resin, preferably the ion exchange resin of a tertiary amine or quaternary ammonia salt based on dextran, agarose, polyacrylate, polystyrene, poly(diphenylethylene), or the like. The solvents used for permeation and ultrafiltration are not limited, generally water or organic solvents, of which there is no special reatriction on the organic solvent, as long as the product can be dissolved in it, preferably dichloromethane, or chloroform, etc.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base such as triethylamine, pyridine, 4-dimethylaminopyridine, imidazole, and diisopropylethylamine, or an inorganic base such as sodium carbonate, sodium hydroxide, sodium bicarbonate, sodium acetate, potassium carbonate, and potassium hydroxide, preferably an organic base, and more preferably triethylamine or pyridine. The amount of the base is 1 to 50 times the molar equivalents of the sulfonate or halide, preferably 1 to 10 times, and more preferably 3 to 5 times.

### 2.2.3.2. Alkylation reaction of substrate amines with sulfonate or halide

The amine intermediate can be obtained via the nucleophilic substitution of the substrate amine with a sulfonate derivative or a halide under a basic condition. Wherein, the amount of the sulfonate or halide is 1 to 50 and preferably 1 to 5 molar equivalents per molar equivalent of the substrate amine. When the amount of the sulfonate or halide is less than 1 molar equivalent per molar equivalent of the substrate amine, the substitution may not be complete, causing difficulty in the purification process. When the amount of the sulfonate or halide exceeds 50 molar equivalents per molar equivalent of the substrate amine, the excess reagents can cause problems with purification and can blend into subsequent steps, resulting in increased side reactions and making the purification difficult.

The resulting product is a mixture of amine intermediate and excess sulfonate or halide, which can be purified by a purification means such as column chromatography, anion exchange resin, permeation, ultrafiltration, and the like. Wherein, the anion exchange resin is not particularly limited as long as the target product can undergo ion exchange and adsorption on the resin, preferably the ion exchange resin of a tertiary amine or quaternary ammonia salt based on dextran, agarose, polyacrylate, polystyrene, poly(diphenylethylene), or the like. The solvents used for permeation and ultrafiltration are not limited, generally water or organic solvents. The organic solvent is not particularly limited as long as the product can be dissolved in it, preferably dichloromethane, chloroform, or the like.

The reaction solvent is not limited, preferably an aprotic solvent such as toluene, benzene, xylene, acetonitrile, ethyl acetate, tetrahydrofuran, chloroform, dichloromethane, dimethylsulfoxide, dimethylformamide, and dimethylacetamide, and more preferably dimethylformamide, dichloromethane, dimethylsulfoxide, or tetrahydrofuran.

The base can be an organic base such as triethylamine, pyridine, 4-dimethylaminopyridine, imidazole, and diisopropylethylamine, or an inorganic base such as sodium carbonate, sodium hydroxide, sodium bicarbonate, sodium acetate, potassium carbonate, and potassium hydroxide, preferably an organic base, and more preferably triethylamine or pyridine. The amount of the base is 1 to 50 times the molar equivalents of the sulfonate or halide, preferably 1 to 10 times, and more preferably 3 to 5 times.

### 2.2.3.3. Alkylation reaction of substrate amines with aldehyde derivatives

The substrate amine reacts with an aldehyde derivative to obtain an imine intermediate, which is followed by obtaining an intermediate by reducting reagents. Wherein, the amount of the aldehyde derivative is 1 to 20 times that of substrate amines, preferably 1 to 2 times, and more preferably 1 to 1.5 times. When the amount of aldehyde exceeds 20 times that of the substrate amine, the excess reagent can cause difficulty in the purification process and may be mixed into subsequent steps, resulting in increased difficulty of purification. When the amount of aldehyde is less than 1 times that of the substrate amine, the reaction may not be complete, causing the purification process to be more difficult. Wherein, the resulting product can be obtained after purification by means such as cation exchange resin, permeation, ultrafiltration, and the like. The cation exchange resin is not particularly limited as long as it can undergo ion exchange with quaternary ammonium cations to realize the isolation. The solvents used for permeation and ultrafiltration are not limited, generally water or organic solvents. The organic solvent is not particularly limited as long as the product can be dissolved in it, preferably dichloromethane, chloroform, or the like.

The reaction solvent is not limited, preferably an organic solvent such as methanol, ethanol, water, toluene, benzene, xylene, acetonitrile, ethyl acetate, tetrahydrofuran, chloroform, dichloromethane, dimethylsulfoxide, dimethylformamide, dimethylacetamide, and the like, and more preferably water or methanol.

The reduction reagent is not particularly limited as long as the imine can be reduced to an amine, preferably sodium borohydride, lithium aluminum hydride, sodium cyanoborohydride, Zn/AcOH, or the like, and more preferably sodium cyanoborohydride. The molar amount of the reduction reagent used is generally 0.5 to 50 folds and preferably 1 to 10 folds of that of aldehyde derivatives.

### 2.2.4. The linear end-functionalization

The method for linear end-functionalization is not particularly limited but related to the type of the final functional group or the protected form thereof. The method mainly includes the functionalization of the terminal hydroxyl group and the conversion of a reactive group into the target functional group or the protected form thereof.

The method for functionalization of the terminal hydroxyl group is described herein, which is by converting the terminal hydroxyl group of A into a group from classes A~J and protected forms thereof via functionalization. A specific preparation method is described in paragraphs [0960] to [1205] of the document CN104530417A. The general formula of the reaction is as follows:

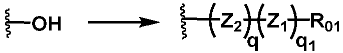

Wherein, q and q₁ are each independently 0 or 1; Z₁ and Z₂ are each independently a divalent linking group; R₀₁ is the functional group capable of reacting with bio-related substances.

Conversion of reactive groups into the target functional groups or protected forms thereof can be achieved by any of the following approaches:
Approach 1: direct modification. The target functional group or its protected form can be obtained via direct modification of a reactive group. The direct modification includes, for example, the conversion of a carboxyl group to an acyl halide group, a hydrazide group, an ester group, a thioester group, or a dithioester group, the conversion of a hydroxyl group, a sulfhydryl group, an alkynyl group, an amino group, a carboxyl group, or the like to the corresponding protected form, and the modification of a hydroxyl group, an amino group, or the like with an anhydride, etc.

Approach 2: coupling reaction between two reactive groups. The coupling reaction uses a heterofunctional reagent containing a reactive group and the target functional group or its protected form as a starting material and introduces the target functional group or its protected form through the reaction between one of the reactive groups and the A-terminal reactive group. The modes and methods of the reaction between two reactive groups are not particularly limited, wherein the reaction conditions are related to the types of divalent linking groups formed via the reaction. The available prior art such as alkylation reaction, addition reaction of alkenes, addition reaction of alkynes, combination of Schiff-base reaction and reduction reaction, condensation reaction, and the like, can be used herein. Wherein, the alkylation reaction is preferably based on a sulfhydryl group or an amino group, corresponding to the formation of a thioether bond, and a secondary or tertiary amino group, respectively. Wherein, the condensation reaction includes but is not limited to those forming an ester bond, a thioester bond, an amide bond, an imine bond (-C=N-), a hydrazone bond, a carbamate bond, or the like. The target functional group or its protected form can also be introduced via click reactions using materials such as a heterofunctional reagent containing both the target functional group or its protected form and a reactive group selected from the group consisting of an azido group, an alkynyl group, an alkenyl group, a trithioester group, a sulfhydryl group, a dienyl group, a furyl group, a 1,2,4,5-tetrazinyl group, a cyanate group, and the like. The reaction between two reactive groups is accompanied by the formation of new bonds. Typical representatives of the newly formed divalent linking groups include amide bond, urethane bond, ester bond, secondary amino bond, thioether bond, triazole group, and the like.

Approach 3: the target functional group or its protected form is obtained by a combination of direct modification and coupling reactions.

In the present application, starting materials in every preparation method can be synthesized or purchased.

The intermediates and end-products prepared in the present application can be purified by the purification method including but not limited to extraction, recrystallization, adsorption treatment, precipitation, reverse precipitation, membrane dialysis, supercritical extraction, and the like. The characterization of the structure and the molecular weight of end-products can use methods including but not limited to NMR, electrophoresis, UV-visible spectrophotometer, FTIR, AFM, GPC, HPLC, MALDI-TOF, circular dichroism spectroscopy, mass spectrometry, etc.

### 3.1. Lipid compositions

In the present application, provided herein is a lipid composition containing any amino acid-based cationic lipid whose structure is represented by the general formula (1).

In one specific embodiment of the present application, it is preferred that the lipid composition contains not only a cationic lipid of a structure represented by the general formula (1) but also contains one or more types of lipids selected from the group consisting of phospholipid, steroidal lipid and PEGylated lipid, and the lipid composition is selected from any of the following cases:
Case (1): also contains a phospholipid;
Case (2): also contains a steroid lipid;
Case (3): also contains a PEGylated lipid;
Case (4): also contains a phospholipid and a steroid lipid;
Case (5): also contains a phospholipid and a PEGylated lipid;
Case (6): also contains a steroid lipid and a PEGylated lipid;
Case (7): also contains a phospholipid, a steroid lipid and a PEGylated lipid;
more preferably, the lipid composition also contains a neutral lipid, a steroid lipid and a PEGylated lipid, simultaneously.

In a specific embodiment of the present application, phospholipids in the lipid composition are preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-O-octadecenyl-sn-glycero-3-phosphatidylcholine (18:0 Diether PC), 1-oleoyl-2-cholesterylhemisuccinyl-sn-glycero-3-phosphocholine (OChemsPC), 1-O-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine (LPE), and combinations thereof.

In a specific embodiment of the present application, the steroid lipid in the lipid compositions is preferably selected from the group consisting of cholesterol, fecal sterol , sitosterol, ergosterol, campesterol, stigmasterol, rapeseed sterol , lycopene, ursolic acid, α-tocopherol, and combinations thereof.

In a specific embodiment of the present application, the PEGylated lipid in the lipid composition is preferably selected from the group consisting of 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine and PEG2000-2,3-dimyristoylglycerol (PEG-DMG), and combinations thereof.

In a specific embodiment of the present application, the PEGylated lipid in the lipid composition is preferably selected from the group consisting of the following structures and combinations thereof: 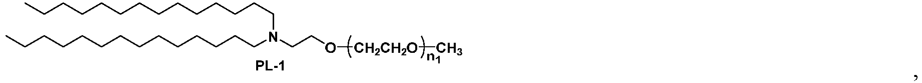 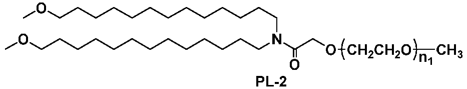 and 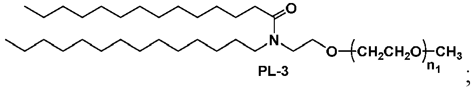 wherein n₁ is an integer of 25 to 300. More preferably, n₁ is selected from the group consisting of 44, 45, 46, 47, and 48.

In one specific embodiment of the present application, any of the aforementioned lipid compositions preferably contain 20-80% amino acid-based cationic lipid represented by the formula (1), 5-15% phospholipid, 25-55% steroid lipid, and 0.5-10% PEGylated lipid; wherein, the percentage is the molar percentage of each lipid in the total lipid in a solution containing solvent.

In a specific embodiment of the present application, in any of the aforementioned lipid compositions, the molar percentage of cationic lipid in the total lipid in a solution containing solvent is preferably 30-65%, more preferably about 35%, 40%, 45%, 46%, 47%, 48%, 49%, 50%, or 55%.

In a specific embodiment of the present application, in any of the aforementioned lipid compositions, the molar percentage of phospholipid in the total lipid in a solution containing solvent is preferably about 7.5-13%, more preferably about 8%, 9%, 10%, 11%, or 12%.

In a specific embodiment of the present application, in any of the aforementioned lipid compositions, the molar percentage of steroid lipid in the total lipid in a solution containing solvent is preferably 35-50%, more preferably about 40%, 41%, 42%, 43%, 44%, 45%, 46%, 47%, 48%, 49%, or 50%.

In a specific embodiment of the present application, in any of the aforementioned lipid compositions, the molar percentage of PEGylated lipid in the total lipid in a solution containing solvent is preferably 0.5-5%, more preferably 1-3%, more preferably about 1.5%, 1.6%, 1.7%, 1.8%, or 1.9%.

### 3.2. Preparation of lipid compositions

In the present application, the lipid composition can be prepared by the following methods, including but not limited to ethanol injection, microfluidic method, T-tube mixing method, and periplasmic extrusion method, preferably ethanol injection and microfluidic method.

### 4. Lipid pharmaceutical compositions and formulations thereof

### 4.1. Lipid pharmaceutical compositions

In one embodiment of the present application, a lipid pharmaceutical composition contains any aforementioned lipid composition and a drug; wherein, the lipid composition contains any of the previously described amino acid-based cationic lipids with the structure represented by general formula (1), and the drug is selected from the group consisting of a nucleic acid drug, ga ene vaccine, an antitumor drug, a small-molecule drug, a peptide drug and a protein drug.

In a specific embodiment of the present application, in a lipid pharmaceutical composition, the nucleic acid drug is selected from the group consisting of RNA, DNA, antisense nucleic acid, plasmid, interfering nucleic acid, aptamer, antagomir and ribozyme, the RNA is selected from the group consisting of mRNA, saRNA, circRNA, miRNA and siRNA; preferably, the nucleic acid drug is is selected from the group consisting of DNA, mRNA, miRNA and siRNA.

In a specific embodiment of the present application, the lipid pharmaceutical composition is preferably used as a drug, selected from the group consisting of the following drugs: an antineoplastic agent, an antiviral agent, an antifungal agent and a vaccine.

In one specific embodiment of the present application, the drug preferably includes, but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, tioteppa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytosine arabinoside, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitic agents, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, quinizarin, mithramycin, 1-dihydrotestosterone, glucocorticoid, procaine, tetracaine, lidocaine, puromycin, and maytansinoid.

In a specific embodiment of the present application, the N/P ratio of the lipid composition to the nucleic acid is preferably (0.1~100):1, more preferably (0.2~30):1, and most preferably (0.5~20):1.

### 4.2. Formulations of Lipid pharmaceutical compositions

In a specific embodiment of the present application, the drug in the lipid pharmaceutical composition is a nucleic acid drug, and the working solution is preferably deionized water, ultrapure water, phosphate buffer or normal saline, more preferably phosphate buffer or normal saline, and most preferably normal saline; preferred lipid composition: working solution = (0.05~20) g: 100 mL, more preferably (0.1~10) g: 100 mL, most preferably (0.2~5) g: 100 mL.

In a specific embodiment of the application, a formulation of lipid pharmaceutical composition contains the aforementioned lipid pharmaceutical composition and a pharmaceutically acceptable diluent or excipient; the diluent or excipient is preferably deionized water, ultrapure water, phosphate buffer or normal saline, more preferably phosphate buffer or normal saline, most preferably normal saline.

In the present application, the preparation of formulation of lipid pharmaceutical composition comprises the following steps:
(1) equilibrate the lipid composition in the diluent or excipient;
(2) add the nucleic acid drug to the equilibrated mixture of the balanced lipid composition and diluent or excipient for complexing;
wherein, the equilibrium time is preferably 0.1~12 h, more preferably 0.2~6 h, and more preferably 0.5~3 h; the composite time is preferably 0.1~12 h, more preferably 0.2~5 h, and more preferably 0.5~2 h.

### 5. Liposomes or lipid nanoparticles and the preparation thereof

### 5.1. Liposomes or lipid nanoparticles

In a specific embodiment of the present application, a liposome or lipid nanoparticle contains any lipid pharmaceutical composition described above.

In a specific embodiment of the present application, the aforementioned lipid nanoparticle is preferably an LNP pharmaceutical composition, an LPP pharmaceutical composition, or a PNP pharmaceutical composition; preferably an LNP pharmaceutical composition; more preferably an LNP-nucleic acid pharmaceutical composition; more preferably an LNP-mRNA pharmaceutical composition.

### 5.2. Preparation of liposomes or lipid nanoparticles

In a specific embodiment of the present application, liposomes can be prepared by the following methods including but not limited to thin-film dispersion method, ultrasonic dispersion method, reverse-phase evaporation method, freeze-drying method, freeze-thaw method, double emulsion method and injection method, preferably thin-film dispersion method, ultrasonic dispersion method and/or reverse-phase evaporation method.

In a specific embodiment of the present application, lipid nanoparticles can be prepared by the following methods including but not limited to microemulsion method, double emulsion method, high-shear homogenization and ultrasonication, thin-film hydration extrusion method, and microfluidic method.

In a specific embodiment of the present application, liposomes are prepared by the thin-film dispersion method that includes the following steps:
Step (1): weigh cationic lipids, steroid lipids, neutral lipids, and PEGylated lipids, fully dissolve them in organic solvents, shake them well, remove the organic solvents by reduced pressure rotary evaporation to obtain an oil film, and further dry with a vacuum pump to remove the organic solvents;
Step (2): add phosphate buffer with dissolved cryoprotectant, and use water-bath ultrasonication to obtain a translucent emulsion;
Step (3): add the emulsion to a high-pressure homogenizer for overpressure, and then add the overpressurized emulsion to the liposome extruder for film-passing to obtain cationic liposomes;
Step (4): optionally, dry the liposomes in a freeze dryer to obtain a liposome powder;

wherein, the organic solvent is preferably dichloromethane, chloroform, and/or methanol, more preferably chloroform or methanol; the rotational speed of reduced pressure rotary evaporation is preferably 30 to 300 rpm, more preferably 50 to 200 rpm, and most preferably 100 to 170 rpm; the temperature of vacuum rotary evaporation is preferably 10 to 200 °C, more preferably 20 to 200 °C, and most preferably 40 to 80 °C;
the time of the drying with a vacuum pump is preferably 1 to 72 h, more preferably 5 to 48 h, and most preferably 15 to 36 h;
the mass concentration of the cryoprotectant dissolved in phosphate buffer is preferably 0.1 to 80%, more preferably 1 to 50%, and more preferably 5 to 20%;
the frequency of the water-bath ultrasonication is preferably 10 to 300 kHz, more preferably 30 to 200 kHz, and most preferably 60 to 150 kHz;
the time of the water-bath ultrasonication is preferably 0.1 to 5 h, more preferably 0.2 to 2 h, and most preferably 0.25 to 1 h;
the pressure of the high-pressure homogenizer is preferably 50 to 240 MPa, more preferably 80 to 200 MPa, and most preferably 100 to 150 MPa;
the times of the overpressure of the high-pressure homogenizer is preferably any integer from 1 to 50, more preferably any integer from 3 to 20, and most preferably any integer from 5 to 10;
the pressure of the liposome extruder is preferably 50 to 300 MPa, more preferably 80 to 250 MPa, and most preferably 120 to 200 MPa;
the times of the film-passing of liposome extruder is preferably any integer from 1 to 50, more preferably any integer from 3 to 30, and most preferably any integer from 5 to 20;
the time of the drying in a freeze dryer is preferably 1 to 120 h, more preferably 5 to 72 h, and most preferably 10 to 36 h.

In a specific embodiment of the present application, In the preparation method of liposomes, the ratio of liposome to phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, lipid nanoparticles are preferably prepared by the microfluidic method, and the steps are as follows:
Step (1): dissolve each lipid component in the organic solvent to obtain the lipid composition dissolved in the organic phase; the organic phase is preferably ethanol;
Step (2): add the nucleic acid drug to a buffer to obtain an aqueous phase solution; the aqueous phase is preferably a citrate buffer or sodium acetate buffer;
Step (3): the organic phase solution and aqueous phase solution are mixed by a microfluidic device to obtain the lipid nanoparticle composition, and then purified by ultrafiltration or the like to remove organic solvents and free nucleic acid molecules.

The following specific examples are further descriptions of the preparation methods of amino acid-based cationic lipids, lipid compositions, and formulations of lipid pharmaceutical composition, and the biological activity assays for lipid pharmaceutical compositions; the specific examples are disclosed to further illustrate the application, but should not be regarded as a limitation of the scope of the present application. Wherein, in the embodiments of preparing cationic lipids, the structures of end products are characterized by NMR, and the molecular weight is confirmed by mass spectrometry.

### Example 1: Cationic lipid (E1-1)

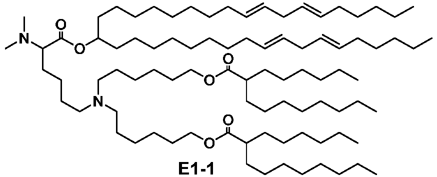

The preparation process is as follows:
Step a: freshly activated magnesium granules (0.40 g, 16.6 mmol) were added to a clean round-bottom flask equipped with a magnetic stir bar, an addition funnel, and a reflux condenser. The device was degassed and rinsed with argon gas, and 2 mL of anhydrous ether was added to the flask through a syringe. The bromide 18-bromooctadedeca-6,9-diene (S1-1, 4.37 g, 13.3 mmol) was dissolved in anhydrous ether (50 mL) and added to the drop funnel. About 1 mL of this ether solution was added to the magnesium granules with vigorous agitation. An exothermic reaction was noted (to confirm/accelerate formation of the Grignard reagent, 1 mg of iodine was added, and decolorization was immediately observed, and thereby the formation of Grignard reagent formation was confirmed), and the ether began to reflux. The remaining bromide solution was added dropwise while maintaining the reaction under gentle reflux by cooling the flask in water. After the addition was complete, the reaction mixture was kept at 35 °C for 1 h and then cooled in an ice bath. **Ethyl formate** (0.44 g, 6.0 mmol) was dissolved in anhydrous ether (8 mL),then the solution was transferred to the addition funnel, and then added dropwise to the reaction mixture while stirring. An exothermic reaction was observed and the reaction mixture began to reflux. After the reaction was initiated, the ether solution of the remaining formate ester was rapidly added in the form of a liquid stream, and the reaction mixture was stirred for another 1 h at room temperature. The reaction was quenched by adding 5 mL of acetone dropwise followed by ice water (10 mL). The reaction mixture was treated with aqueous H₂SO₄ (10% volume, 50 mL) until the solution became homogeneous, and the solution was stood for stratification. The aqueous phase was extracted with ether (20 mL*2). The combined ether layer was dried with anhydrous magnesium sulfate, concentrated to obtain a crude product, and then purified by column chromatography to obtain colorless oily compound **S1-2** (2.46 g).
Step b: under nitrogen atmosphere, *N,N*-dicyclohexylcarbodiimide (**DCC**, 1.00 g, 4.8 mmol) was added to a round-bottom flask containing **S1-2** (2.46 g), Boc-protected N,N-dimethyllysine (**S1-3**, 0.60 g, 2.2 mmol) and 4-dimethylaminopyridine (**DMAP,** 67.10 mg, 0.55 mmol) dissolved in dichloromethane (20 mL), and the reaction was conducted for 16 h at room temperature. After the reaction, the precipitate was removed by filtration, the filtrate was concentrated, and the resulting residue was purified by column chromatography to obtain compound **S1-4** (1.48 g).
Step c: removing the Boc protecting group. In a dry and clean round-bottom flask, a solution of trifluoroacetic acid/dichloromethane (1:2, v/v) was prepared, and the dichloromethane solution of **S1-4** (1.18 g, 1.5 mmol) in was slowly added dropwise under ice bath conditions and reacted for 2 h at room temperature. After the reaction, the reaction solution was concentrated, diluted with purified water, and extracted with dichloromethane. The organic phase was dried with anhydrous magnesium sulfate, and filtered, and the filtrate was concentrated and recrystallized to obtain compound **S1-5** (0.92 g, 90.1%).
Step d: Under the protection of nitrogen, compound **S1-5** (0.69 g, 1.0 mmol) was dissolved in acetonitrile (20 mL). 6-bromohexyl-2-hexyldecanoate (**S1-6**, 1.05 g, 2.5 mmol, **S1-6** was prepared by the reaction between 2-hexyldecanoic acid 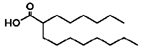 and 6-bromohexanol 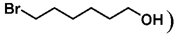 and *N,N*-diisopropylethanamine (**DIPEA,** 0.18 g, 2.0 mmol) were added successively while stirring slowly and reacted at room temperature for about 20 h. After the end of the reaction, the reaction solution was concentrated, then dissolved with dichloromethane, and extracted sequentially with 0.6 M hydrochloric acid/10% sodium chloride solution and saturated sodium bicarbonate solution. The organic phases were combined, dried with anhydrous magnesium sulfate, and filtered, and the filtrate was concentrated, and purified by column chromatography to obtain cationic lipid **E1-1** (1.11 g). ¹H NMR (400 MHz, CDCl₃) δ: 5.35-5.24 (m, 8H), 4.92-4.80 (m, 1H), 4.02 (t, 4H), 3.05 (t, 1H), 2.76-2.68 (m, 4H), 2.36-2.26 (m, 14H), 2.03-1.97 (m, 8H), 1.69-1.14 (m, 110H), 0.89 (t, 18H). MS (ESI): m/z=1362.30 ([M+H]⁺).

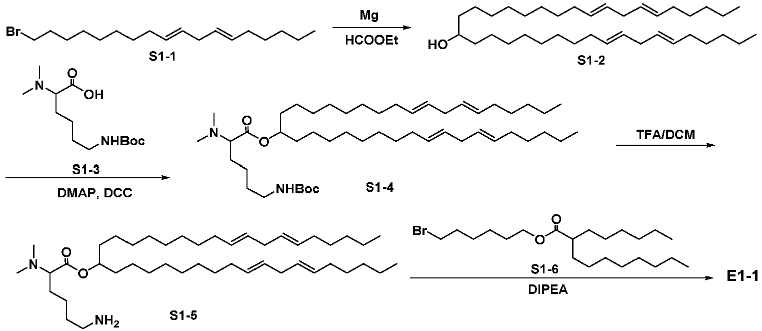

### Example 2: Cationic lipid (E2-1)

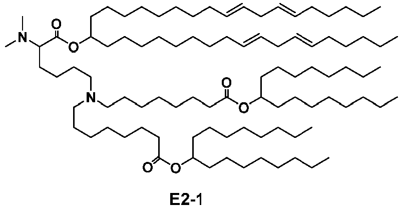

The preparation process is as follows:
Step a: **DCC** (1.81 g, 8.8 mmol) was added to a round-bottom flask containing 8-bromononanoic acid (**S2-1**, 0.89 g, 4.0 mmol), 9-heptadecanol (**S2-2**, 1.23 g, 4.8 mmol) and **DMAP** (0.12 g, 1.0 mmol) dissolved in dichloromethane (30 mL), and the reaction was conducted for 16 h at room temperature. After the reaction, the precipitate was removed by filtration, the filtrate was concentrated, and the resulting residue was purified by column chromatography to obtain 8-bromo-octanoic acid 1-octylnonyl ester (**S2-3**, 1.52 g).
Step b: under nitrogen protection, compound **S1-5** (0.55 g, 0.8 mmol) was dissolved in acetonitrile (20 mL), and then **S2-3** (0.92 g, 2.0 mmol) and **DIPEA** (0.14 g, 1.6 mmol) were added sequentially under slow stirring, and the reaction was strred for about 20 h at room temperature. After the end of the reaction, the reaction solution was concentrated, then dissolved with dichloromethane, and then extracted sequentially with 0.6 M hydrochloric acid/10% sodium chloride solution and saturated sodium bicarbonate solution. The organic phases were combined, dried with anhydrous magnesium sulfate, and filtered, the filtrate was concentrated and purified by column chromatography to obtain cationic lipid **E2-1** (0.95 g). ¹H NMR (400 MHz, CDCl₃) δ: 5.42-5.25 (m, 8H), 4.93-4.81 (m, 3H), 3.08 (t, 1H), 2.83-2.68 (m, 4H), 2.49-2.30 (m, 12H), 2.26 (t, 4H), 2.07-2.00 (m, 8H), 1.50-1.19 (m, 122H), 0.88 (t, 18H). MS (ESI): m/z=1446.39 ([M+H]⁺).

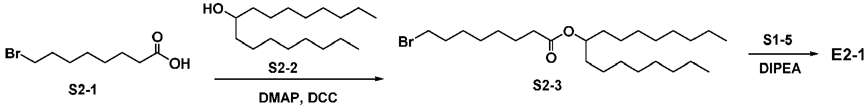

### Example 3: Cationic lipid (E3-1)

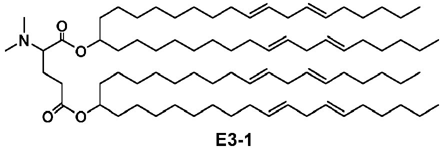

The preparation process is as follows:
**S1-2** (1.98 g, 3.8 mmol) and *N,N*-dimethyl glutamic acid (**S3-1**, 0.26 g, 1.5 mmol) were dissolved in anhydrous dichloromethane, respectively. After stirring well, the two component solutions were combined. **DMAP** (18.30 mg, 0.15 mmol), 1-ethyl-(3-dimethylaminopropyl) carbodiimide hydrochloride **(EDCI,** 0.72 g, 3.8 mmol) and **DIPEA** (0.68 g, 7.5 mmol) were added to the mixture sequentially, and the reaction was stirred for 9 h at room temperature. After the end of the reaction, the reaction solution was diluted with dichloromethane (20 mL), then washed twice with saturated sodium carbonate solution (10 mL*2), washed twice with 10 mL aqueous solution, and then washed once with saturated saline. The organic phase was dried with anhydrous sodium sulfate, and the solvent was concentrated to obtain a crude product. The crude product was separated and purified by column chromatography. The target eluent was collected and concentrated to obtain cationic lipid **E3-1** (0.95 g). ¹H NMR (400 MHz, CDCl₃) δ: 5.45-5.26 (m, 16H), 4.92-4.82 (m, 2H), 3.06 (t, 1H), 2.83-2.68 (m, 8H), 2.43 (s, 6H), 2.26 (t, 2H), 2.03-1.97 (m, 16H), 1.71-1.23 (m, 82H), 0.89 (t, 12H). MS (ESI): m/z=1197.11 ([M+H]⁺).

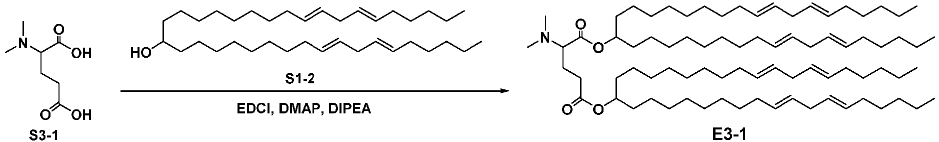

### Example 4: Cationic lipid (E4-2)

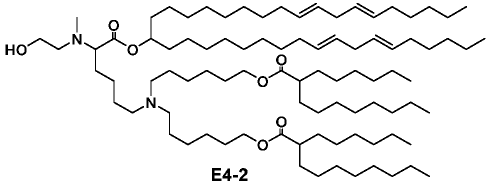

The preparation process is as follows:
Step a: under the protection of nitrogen, TBS-N-hydroxyethyl-N-methyllysine (**S4-1**, 0.64 g, 2.0 mmol, **S4-1** was obtained by the reaction between N-methyllysine 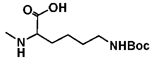 with a Boc protected amino group with 2-bromoethanol 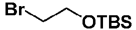 with a TBS protected hydroxyl group, and then removing the Boc group) was dissolved in acetonitrile (30 mL), and **S1-6** (2.10 g, 5.0 mmol) and **DIPEA** (0.36 g, 4.0 mmol) were added sequentially under slow stirring and the reaction was stirred for about 20 h at room temperature. After the end of the reaction, the reaction solution was concentrated and dissolved with dichloromethane, and then extracted sequentially with 0.6 M hydrochloric acid/10% sodium chloride solution and saturated sodium bicarbonate solution. The organic phases were combined, dried with anhydrous magnesium sulfate, filtered and concentrated, and purifiedby column chromatography to obtain **S4-2** (1.63 g).
Step b: under nitrogen atmosphere, **DCC** (0.59 g, 2.9 mmol) was added to a round-bottom flask containing compounds **S4-2** (1.29 g, 1.3 mmol), **S1-2** (0.82 g, 1.6 mmol) and **DMAP** (39.65 mg, 0.3 mmol) dissolved in dichloromethane, and the reaction was conducted for 16 h at room temperature. After the reaction, the precipitate was removed by filtration, the filtrate was concentrated, and the resulting residue was purified by column chromatography to obtain **E4-1** (1.61 g).
Step c: the above compound **E4-1** (1.20 g, 0.8 mmol) was dissolved in THF (20 mL), and added into a nitrogen-protected flask, and tetrabutylammonium fluoride solution **(TBAF,** 20 mL, 1 M) was added to react overnight to remove the TBS protection. After the reaction, the reaction solution was concentrated and extracted, and the organic phases were combined, dried over anhydrous sodium sulfate, filtered, and the filtrate was concentrated, and purified by column chromatography to obtain cationic lipid **E4-2** (0.98 g, 88.4%).¹H NMR (400 MHz, CDCl₃) δ: ¹H NMR (400 MHz, CDCl₃) δ: 5.40-5.32 (m, 8H), 4.90 (t, 1H), 4.05 (t, 4H), 3.60-3.55 (m, 2H), 3.22 (t, 1H), 2.85-2.67 (m, 6H), 2.43-2.28 (m, 11H), 2.08-2.03 (m, 8H), 1.44-1.22 (m, 110H), 0.87 (t, 18H).MS (ESI): m/z=1414.29 ([M+Na]⁺).

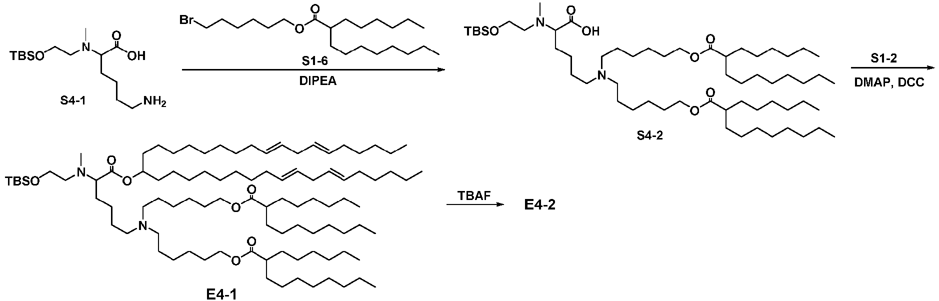

### Example 5: Cationic lipid (E5-2)

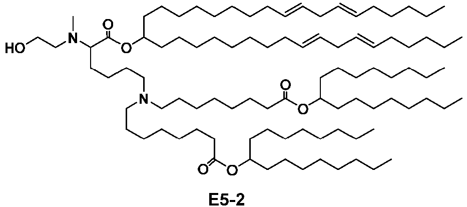

The preparation process is as follows:
Step a: under nitrogen protection, compound **S4-1** (0.64 g, 2.0 mmol) was dissolved in acetonitrile (30 mL), and **S2-3** (2.30 g, 5.0 mmol) and **DIPEA** (0.36 g, 4.0 mmol) were added sequentially under slow stirring. The reaction was stirred for about 20 h at room temperature. After the reaction, the reaction solution was concentrated and dissolved with dichloromethane, and then extracted sequentially with 0.6 M hydrochloric acid/10% sodium chloride solution and saturated sodium bicarbonate solution. The organic phases were combined, dried with anhydrous magnesium sulfate, filtered and the filtrate was concentrated, and purified by column chromatography to obtain **S5-1** (1.77 g) purification.
Step b: under a nitrogen atmosphere, **DCC** (0.59 g, 2.9 mmol) was added to a round-bottom flask containing **S5-1** (1.40 g, 1.3 mmol), **S1-2** (0.82 g, 1.6 mmol) and **DMAP** (39.65 mg, 0.3 mmol) dissolved in dichloromethane (30 mL) and the reaction was conducted for 16 h at room temperature. After the reaction, the precipitate was removed by filtration, the filtrate was concentrated, and the resulting residue was purified by column chromatography to obtain **E5-1** (1.70 g).
Step c: the above compound **E5-1** (1.27 g, 0.8 mmol) was dissolved in THF (20 mL) and added into in a nitrogen-protected flask, and **TBAF** solution (20 mL, 1 M) was added to react overnight to remove TBS protection. After the reaction, the reaction solution was concentrated and extracted, and the organic phases were combined, dried with anhydrous sodium sulfate, and filtered, the filtrate was concentrated, and purified by column chromatography to obtain cationic lipid **E5-2** (1.11 g, 88.1%). ¹H NMR (400 MHz, CDCl₃) δ: ¹H NMR (400 MHz, CDCl₃) δ: 5.42-5.29 (m, 8H), 4.93-4.83 (m, 3H), 3.57 (t, 2H), 3.22 (t, 1H), 2.81-2.70 (m, 6H), 2.47-2.17 (m, 13H), 2.08-1.99 (m, 8H), 1.69-1.08 (m, 122H), 0.88 (t, 18H), MS (ESI): m/z=1498.39 ([M+Na]⁺).

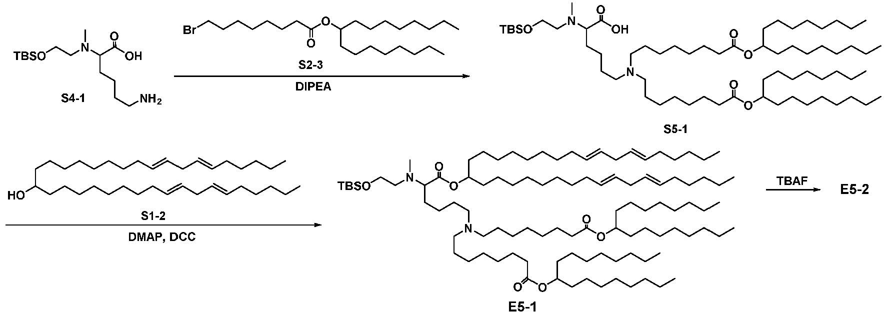

### Example 6: Cationic lipid (E6-2)

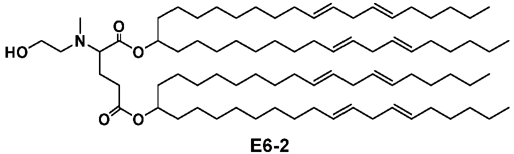

The preparation process is as follows:
Step a: **S1-2** (2.64 g, 5.0 mmol) and TBS-N-hydroxyethyl-N-methylglutamic acid (**S6-1**, 0.64 g, 2.0 mmol) were dissolved in anhydrous dichloromethane, respectively. After stirring well, the two component solutions were combined. **DMAP** (24.40 mg, 0.2 mmol), **EDCI** (0.96 g, 5.0 mmol) and **DIPEA** (0.90 g, 10.0 mmol) were added to the mixture sequentially, and the reaction was stirred at room temperature for 9 h. After the reaction, the reaction solution was diluted with dichloromethane (20 mL), then washed twice with saturated sodium carbonate solution (10 mL*2), washed twice with 10 mL aqueous solution, and washed once with saturated saline. The organic phase was dried with anhydrous sodium sulfate, and the solvent was concentrated to obtain a crude product. The crude product was separated and purified by column chromatography, the target eluent was collected and concentrated to obtain the product **E6-1** (1.43 g).
Step b: the above compound **E6-1** (1.07 g, 0.8 mmol) was dissolved in THF (20 mL) and added into a nitrogen-protected flask, then **TBAF** solution (20 mL, 1 M) was added to react overnight and remove TBS protection. After the reaction, the reaction solution was concentrated and extracted. The organic phases were combined, dried with anhydrous sodium sulfate, and filtered, and the filtrate was concentrated, and purified by column chromatography to obtain cationic lipid **E6-2** (0.87 g, 88.4%). ¹H NMR (400 MHz, CDCl₃) δ: 5.45-5.26 (m, 16H), 4.92-4.81 (m, 2H), 3.58 (t, 2H), 3.06 (t, 1H), 2.85-2.69 (m, 10H), 2.43 (s, 3H), 2.26 (t, 2H), 2.03-1.97 (m, 16H), 1.71-1.22 (m, 82H), 0.88 (t, 12H). MS (ESI): m/z=1227.13 ([M+H]⁺).

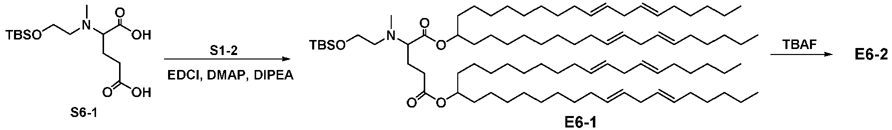

### Example 7: Cationic lipid (E7-1)

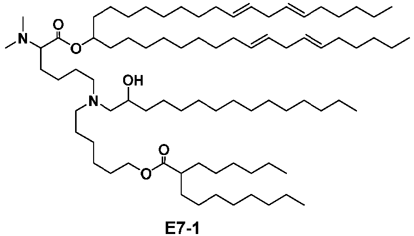

The preparation process is as follows:
Step a: 1,2-epoxytetradecane (**S7-1**, 0.32 g, 1.5 mmol) and **S1-5** (1.03 g, 1.5 mmol) were dissolved in 30 mL of acetonitrile, then calcium trifluoromethanesulfonate **(Ca(OTf)₂,** 0.25 g, 0.8 mmol) was added to the solution. After stirring the reaction mixture at room temperature until TLC showed that the reaction was complete, acetonitrile was evaporated and water (30 mL) was added, and then extracted with dichloromethane (10 mL*3). The organic layer was combined, dried with anhydrous Na₂SO₄, filtered and evaporated. The crude product was separated and purified by column chromatography, the target eluent was collected, and concentrated to obtain cationic lipid **S7-2** (1.27 g, 94.7%).
Step b: under the protection of nitrogen, the above compound **S7-2** (0.90 g, 1.0 mmol) was dissolved in acetonitrile (20 mL), and **S1-6** (0.52 g, 1.3 mmol) and **DIPEA** (0.09 g, 1.0 mmol) were added sequentially under slow stirring. The reaction was stirred for about 20 h at room temperature. After the reaction, the reaction solution was concentrated and then dissolved with dichloromethane, and extracted sequentially with 0.6 M hydrochloric acid/10% sodium chloride solution and saturated sodium bicarbonate solution. The organic phase was combined, dried with anhydrous magnesium sulfate, filtered and concentrated, and purified by column chromatography to obtain cationic lipid **E7-1** (1.02 g). ¹H NMR (400 MHz, CDCl₃) δ: 5.35-5.24 (m, 8H), 4.92-4.80 (m, 1H), 4.02 (t, 2H), 3.55-3.53 (m, 1H), 3.05 (t, 1H), 2.76-2.68 (m, 4H), 2.40 (s, 6H), 2.36-2.26 (m, 7H), 2.03-1.97 (m, 8H), 1.71-1.22 (m, 102H), 0.88 (t, 15H). MS (ESI): m/z=1250.20 ([M+H]⁺).

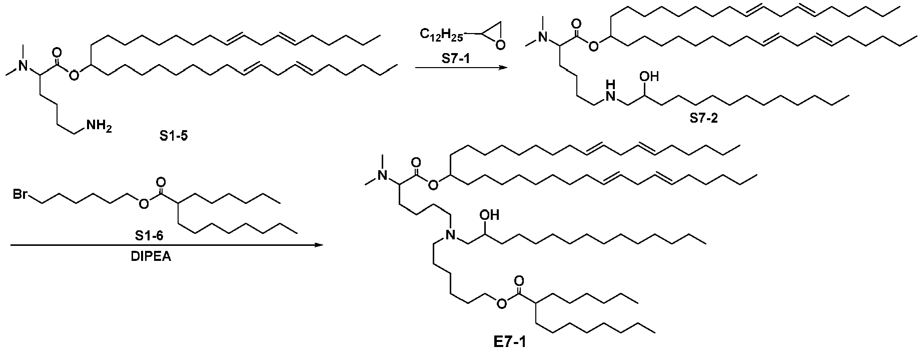

### Example 8: Cationic lipid (E8-1)

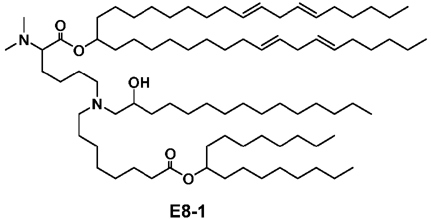

The preparation is as follows:
Under the protection of nitrogen, the above compound **S7-2** (0.90 g, 1.0 mmol) was dissolved in acetonitrile (20 mL), and **S2-3** (0.58 g, 1.3 mmol) and **DIPEA** (0.09 g, 1.0 mmol) were added sequentially under slow stirring. The reaction was stirred for about 20 h at room temperature. After the reaction, the reaction solution was concentrated and then dissolved with dichloromethane, and extracted sequentially with 0.6 M hydrochloric acid/10% sodium chloride solution and saturated sodium bicarbonate solution. The organic phases were combined, dried with anhydrous magnesium sulfate, filtered and concentrated, and cationic lipid **E8-1** (1.06 g) was obtained by column chromatography purification. ¹H NMR (400 MHz, CDCl₃) δ: 5.36-5.24 (m, 8H), 4.92-4.82 (m, 2H), 3.55-3.52 (m, 1H), 3.06 (t, 1H), 2.76-2.68 (m, 4H), 2.40 (s, 6H), 2.36-2.26 (m, 8H), 2.03-1.98 (m, 8H), 1.71-1.22 (m, 108H), 0.88 (t, 15H). MS (ESI): m/z=1292.25 ([M+H]⁺).

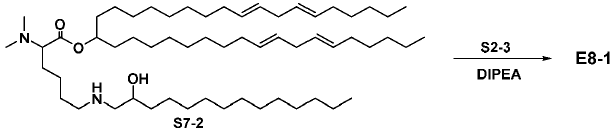

### Example 9: Cationic lipid (E9-1)

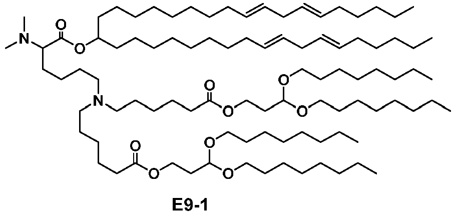

The preparation is as follows:
Step a: 1,3-propanediol (**S9-1**, 9.50 g, 50 mmol) with a TBS-protected hydroxyl group was dissolved in 300 mL of dichloromethane solution, and pyridinium chlorochromate (**PCC**, 16.13 g, 75.0 mmol) was added to the solution. After stirring at 15°C for at least 2 h, **S9-2** (6.02 g) was obtained by filtration, reduced pressure concentration, and silica gel column chromatography purification.
Step b: the above compound **S9-2** (5.64 g, 30.0 mmol) and 1-octanol (**S9-3**, 9.75 g, 75.0 mmol) were dissolved in 200 mL of dichloromethane solution, and p-toluenesulfonic acid monohydrate **(TsOH·H₂O,** 1.14 g, 6.0 mmol) and **anhydrous sodium sulfate** (10.65 g, 75.0 mmol) were added to the solution. After stirring at 15°C for at least 24 h, the reaction solution was filtered, concentrated under reduced pressure, and the crude product was purified by column chromatography to obtain compound **S9-4** (2.84 g).
Step c: the above product **S9-4** (2.16 g, 5.0 mmol) was dissolved in THF (20 mL) and added to a nitrogen-protected flask, and **TBAF** (20 mL, 1 M) was added for overnight reaction to remove the TBS protection. After the reaction, the reaction solution was concentrated, extracted, and the organic phases were combined, dried with anhydrous sodium sulfate, filtered and concentrated, and purified by column chromatography to obtain the compound **S9-5** (1.40 g, 88.6%).
Step d: under nitrogen atmosphere, **DCC** (0.91 g, 4.4 mmol) was added to a round-bottom flask containing **S9-5** (0.76 g, 2.4 mmol), **S9-6** (0.39 g, 2.0 mmol), and **DMAP** (61.00 mg, 0.5 mmol) dissolved in dichloromethane (20 mL) and the reaction was conducted for 16 h at room temperature. After the reaction, the precipitate was removed by filtration, the filtrate was concentrated, and the resulting residue was purified by silica gel column chromatography to obtain **S9-7** (0.81 g).
Step e: under nitrogen protection, compound **S1-5** (0.34 g, 0.5 mmol) was dissolved in acetonitrile (20 mL), and **S9-7** (0.62 g, 1.3 mmol) and **DIPEA** (0.09 g, 1.0 mmol) were added sequentially under slow stirring. The reaction was stirred for about 20 h at room temperature. After the reaction, the reaction solution was concentrated and then dissolved with dichloromethane, and extracted sequentially with 0.6 M hydrochloric acid/10% sodium chloride solution and saturated sodium bicarbonate solution. The organic phases were combined, dried with anhydrous magnesium sulfate, filtered and concentrated, and purified by column chromatography to obtain cationic lipid **E9-1** (0.60 g). ¹H NMR (400 MHz, CDCl₃) δ: 5.45-5.29 (m, 8H), 4.93-4.83 (m, 1H), 4.64 (t, 2H), 4.20 (t, 4H), 3.52-3.36 (m, 8H), 3.06 (t, 1H), 2.83-2.68 (m, 4H), 2.49-2.30 (m, 12H), 2.26 (t, 4H), 2.07-1.98 (m, 12H), 1.71-1.21 (m, 106H), 0.88 (t, 18H). MS (ESI): m/z=1510.36 ([M+H]⁺).

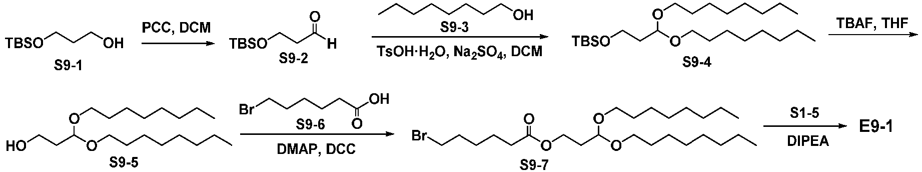

### Example 10.1: Cationic lipid (E10-1)

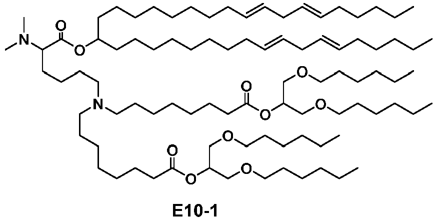

The preparation process is as follows:
Step a: under nitrogen atmosphere, glycerol **(S10-1,** 2.06 g, 10.0 mmol) with a TBS-protected hydroxyl group, potassium carbonate (**K₂CO₃**, 4.14 g, 30.0 mmol) and bromohexane **(S10-2,** 1.80 g, 11.0 mmol) were dissolved in 80 mL of DMF, and the mixture was stirred at 110°C for 16 h, and the reaction solution was poured into water (50 mL) for precipitation and filtration after confirming the completion of the reaction by thin layer chromatography, and further separated and purified by column chromatography to obtain compound **S10-3** (3.35 g, 89.3%).
Step b: the above product **S10-3** (1.88 g, 5.0 mmol) was dissolved in THF (20 mL) and added to a nitrogen-protected flask, and **TBAF** (20 mL, 1 M) was added for overnight reaction to remove the TBS protection. After the reaction, the reaction solution was concentrated, and extracted, and the organic phases were combined, dried with anhydrous sodium sulfate, filtered and concentrated, and purified by column chromatography to obtain compound **S10-4** (1.14 g, 87.9%).
Step c: under nitrogen atmosphere, **DCC** (0.91 g, 4.4 mmol) was added to a round-bottom flask containing **S10-4** (0.62 g, 2.4 mmol), 8-bromooctanoic acid (**S10-5,** 0.45 g, 2.0 mmol) and **DMAP** (61.00 mg, 0.5 mmol) dissolved in dichloromethane (20 mL), and the reaction was conducted for 16 h at room temperature. After the reaction, the precipitate was removed by filtration, the filtrate was concentrated, and the resulting residue was purified by silica gel column chromatography to obtain **S10-6** (0.76 g).
Step d: under nitrogen protection, compound **S1-5** (0.34 g, 0.5 mmol) was dissolved in acetonitrile (20 mL), and **S10-6** (0.58 g, 1.3 mmol) and **DIPEA** (0.09 g, 1.0 mmol) were added successively while stirring slowly. The reaction was stirred for about 20 h at room temperature. After the reaction, the reaction solution was concentrated and dissolved with dichloromethane, and then extracted with 0.6M hydrochloric acid/10% sodium chloride solution and saturated sodium bicarbonate solution, successively. The organic phases were combined, dried with anhydrous magnesium sulfate, filtered and concentrated, and purified by column chromatography to obtain the cationic lipid **E10-1** (0.59 g). ¹H NMR (400 MHz, CDCl₃) δ: 5.41-5.25 (m, 8H), 5.14-5.08 (m, 2H), 4.93-4.85 (m, 1H), 3.59-3.49 (m, 8H), 3.48-3.36 (m, 8H), 3.08 (t, 1H), 2.84-2.66 (m, 4H), 2.46-2.26 (m, 16H), 2.06-1.99 (m, 8H), 1.63-1.18 (m, 98H), 0.87 (t, 18H). MS (ESI): m/z=1454.30 ([M+H]⁺).

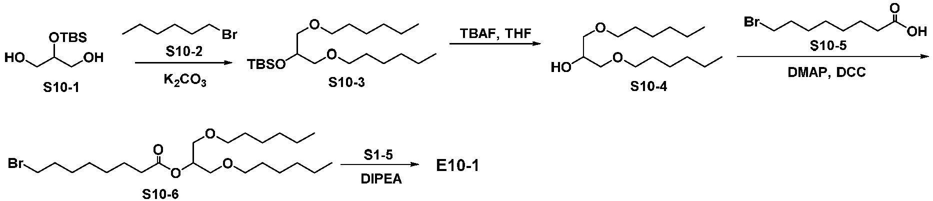

### Example 10.2: Cationic lipid (E10-2)

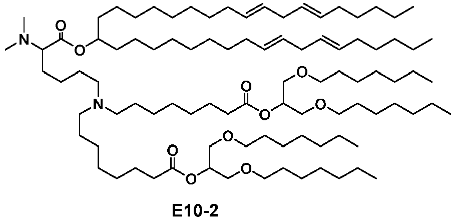

Referring to Example **10.1,** the starting material **S10-2** was replaced with 1-bromoheptane ( 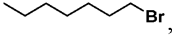 1.97 g, 11.0 mmol), and the same reaction steps for preparation were followed to obtain cationic lipid **E10-2** (0.62 g). ¹H NMR (400 MHz, CDCl₃) δ: 5.43-5.24 (m, 8H), 5.14-5.08 (m, 2H), 4.92-4.85 (m, 1H), 3.59-3.48 (m, 8H), 3.48-3.36 (m, 8H), 3.06 (t, 1H), 2.85-2.66 (m, 4H), 2.46-2.26 (m, 16H), 2.08-1.98 (m, 8H), 1.63-1.18 (m, 106H), 0.89 (t, 18H). MS (ESI): m/z=1510.37 ([M+H]⁺).

### Example 11: Cationic lipid (E11-2)

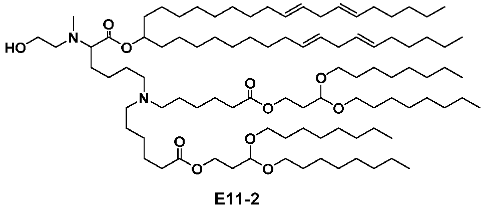

The preparation process is as follows:
Step a: under nitrogen protection, compound **S4-1** (0.96 g, 3.0 mmol) was dissolved in acetonitrile (50 mL), and **S9-7** (3.71 g, 7.5 mmol) and **DIPEA** (0.54 g, 6.0 mmol) were added sequentially under slow stirring. The reaction was stirred for about 20 h at room temperature. After the reaction, the reaction solution was concentrated and then dissolved with dichloromethane, and sequentially extracted with 0.6 M hydrochloric acid/10% sodium chloride solution and saturated sodium bicarbonate solution. The organic phases were combined, dried with anhydrous magnesium sulfate, filtered and concentrated, and purified by column chromatography to obtain compound **S11-1** (2.74 g).
Step b: under nitrogen atmosphere, **DCC** (0.91 g, 4.4 mmol) was added to a round-bottom flask containing **S1-2** (1.27 g, 2.4 mmol), **S11-1** (2.18 g, 2.0 mmol) and **DMAP** (61.00 mg, 0.5 mmol) dissolved in dichloromethane (50 mL) and the reaction was conducted for 16 h at room temperature. After the reaction, the precipitate was removed by filtration, the filtrate was concentrated, and the residue obtained was purified by silica gel column chromatography to obtain **E11-1** (2.67 g).
Step c: the above product **E11-1** (1.66 g, 1.0 mmol) was dissolved in THF (20 mL), placed in a nitrogen-protected flask, and **TBAF** (20 mL, 1 M) was added to react overnight to remove the TBS protection. After the reaction, the reaction solution was concentrated and extracted, and the organic phases were combined, dried with anhydrous sodium sulfate, filtered and concentrated, and purified by column chromatography to obtain cationic lipid **E11-2** (1.35 g, 87.6%). ¹H NMR (400 MHz, CDCl₃) δ: 5.44-5.29 (m, 8H), 4.91-4.83 (m, 1H), 4.63 (t, 2H), 4.20 (t, 4H), 3.60-3.55 (m, 2H), 3.52-3.36 (m, 8H), 3.06 (t, 1H), 2.83-2.68 (m, 6H), 2.48-2.30 (m, 9H), 2.25 (t, 4H), 2.07-1.99 (m, 12H), 1.71-1.21 (m, 106H), 0.88 (t, 18H). MS (ESI): m/z=1540.37 ([M+H]⁺).

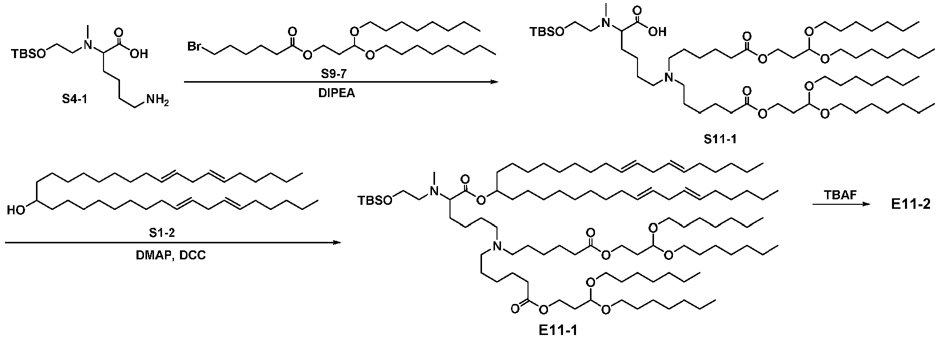

### Example 12: Cationic lipid (E12-2)

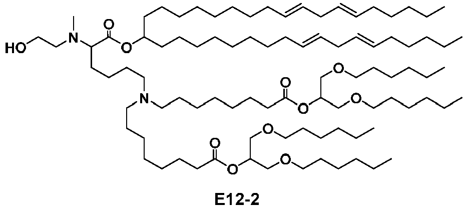

The preparation process is as follows:
Step a: under nitrogen protection, compound **S4-1** (0.96 g, 3.0 mmol) was dissolved in acetonitrile (50 mL), and **S10-6** (3.50 g, 7.5 mmol) and **DIPEA** (0.54 g, 6.0 mmol) were added sequentially under slow stirring. The reaction was stirred for about 20 h at room temperature. After the reaction, the reaction solution was concentrated and then dissolved with dichloromethane, and extracted sequentially with 0.6 M hydrochloric acid/10% sodium chloride solution and saturated sodium bicarbonate solution. The organic phases were combined, dried with anhydrous sodium sulfate, filtered and concentrated, and purified by column chromatography to obtain compound **S12-1** (2.61 g).
Step b: under nitrogen atmosphere, **DCC** (0.91 g, 4.4 mmol) was added to a round-bottom flask containing **S1-2** (1.27 g, 2.4 mmol), **S12-1** (2.17 g, 2.0 mmol), and **DMAP** (61.00 mg, 0.5 mmol) dissolved in dichloromethane (30 mL) and reacted for 16 h at room temperature. After the reaction, the precipitate was removed by filtration, the filtrate was concentrated, and the resulting residue was purified by silica gel column chromatography to obtain **E12-1** (2.58 g).
Step c: the above product **E12-1** (1.60 g, 1.0 mmol) was dissolved in THF (20 mL) and added to a nitrogen-protected flask, and **TBAF** (20 mL, 1 M) was added to react overnight to remove the TBS protection. After the reaction, the reaction solution was concentrated, and extracted, and the organic phases were combined, dried with anhydrous sodium sulfate, filtered and concentrated, and purified by column chromatography to obtain the cationic lipid **E12-2** (1.29 g, 86.9%). ¹H NMR (400 MHz, CDCl₃) δ: 5.43-5.25 (m, 8H), 5.14-5.08 (m, 2H), 4.93-4.85 (m, 1H), 3.60-3.49 (m, 10H), 3.48-3.36 (m, 8H), 3.08 (t, 1H), 2.84-2.67 (m, 6H), 2.45-2.26 (m, 13H), 2.06-1.98 (m, 8H), 1.68-1.18 (m, 98H), 0.87 (t, 18H). MS (ESI): m/z=1484.31 ([M+H]⁺).

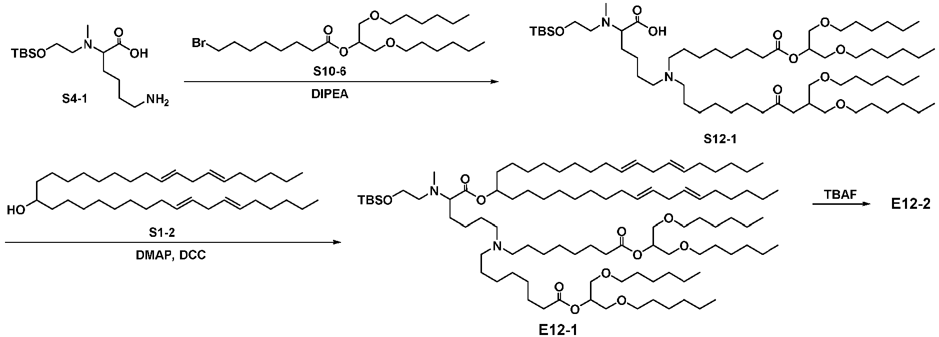

### Example 13: Cationic lipid (E13-2)

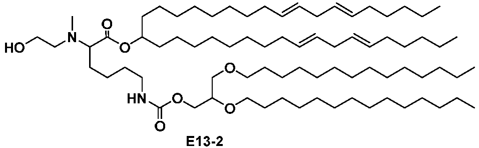

The preparation process is as follows:
Step a: 1,2-Di-O-tetradecyl-sn-glycerol (**S13-1**, 2.43 g, 5.0 mmol, **S13-1** was obtained by the reaction between the glycerol with a TBS protected hydroxyl group 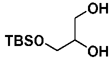 and bromo-tetradecane 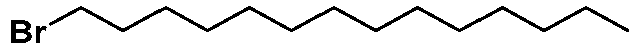 and then removing the TBS protecting group, and the specific operation method referred to the Step a in Example 10) and *N,N'*-succinimide carbonate (**DSC, S13-2**, 1.92 g, 7.5 mmol) were added to dichloromethane (50 mL) and stirred in an ice bath. Triethylamine (**TEA,** 2.1 mL, 15.0 mmol) was added to the stirred solution, and then the reaction mixture was stirred overnight at room temperature. The progress of the reaction was monitored by TLC. After the reaction, the reaction mixture was diluted with dichloromethane. The organic layer was washed with water (50 mL) and sodium bicarbonate aqueous solution (50 mL), successively, and then concentrated, further purified by column chromatography, concentrated, and vacuum-dried to obtain compound **S13-3** (2.72 g)
Step b: **S4-1** (1.15 g, 3.6 mmol) was dissolved in dichloromethane (30 mL), **S13-3** (1.88 g, 3.0 mmol) and **TEA** (0.75 mL, 5.4 mmol) were added sequentially, and the reaction was stirred at room temperature overnight. After the reaction, the reaction solution was concentrated to obtain a crude product. The crude product was purified by column chromatography, and concentrated, and dried with an oil pump to obtain compound **S13-4** (1.96 g).
Step c: under nitrogen atmosphere, **DCC** (0.91 g, 4.4 mmol) was added to a round-bottom flask containing **S1-2** (1.27 g, 2.4 mmol), **S13-4** (1.66 g, 2.0 mmol) and **DMAP** (61.00 mg, 0.5 mmol) dissolved in dichloromethane (30 mL), and the reaction was conducted for 16 h at room temperature. After the reaction, the precipitate was removed by filtration, the filtrate was concentrated, and the resulting residue was purified by silica gel column chromatography to obtain **E13-1** (2.17 g).
Step d: the above product **E13-1** (1.34 g, 1.0 mmol) was dissolved in THF (20 mL) and added to a nitrogen-protected flask, and **TBAF** (20 mL, 1 M) was added to react overnight to remove the TBS protection. After the reaction, the reaction solution was concentrated, and extracted, and the organic phases were combined, dried with anhydrous sodium sulfate, filtered and concentrated, and purified by column chromatography to obtain the cationic lipid **E13-2** (1.05 g, 85.7%). ¹H NMR (400 MHz, CDCl₃) δ: 5.35-5.24 (m, 8H), 4.92-4.80 (m, 1H), 4.13-4.01 (m, 2H), 3.59-3.33 (m, 9H), 3.17-3.00 (m, 3H), 2.76-2.68 (m, 6H), 2.43-2.28 (m, 3H), 2.03-1.97 (m, 8H), 1.71-1.21 (m, 94H), 0.88(t, 12H). MS (ESI): m/z=1226.13 ([M+H]⁺).

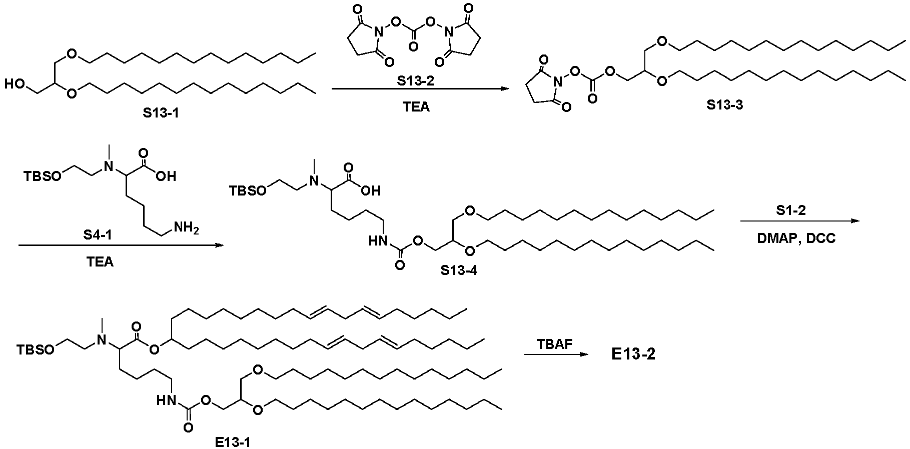

### Example 14: Cationic lipid (E14-2)

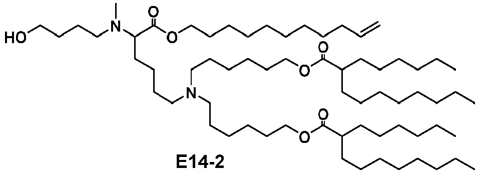

The preparation process is as follows:
Step a: under nitrogen protection, TBS-N-hydroxybutyl-N-methyllysine (**S14-1**, 1.04 g, 3.0 mmol, **S14-1** was obtained by the reaction between N-methyllysine 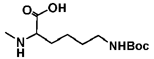 with a Boc protected amino group and 4-bromobutanol 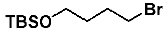 with a TBS protected hydroxyl group, and then removing the Boc group) was dissolved in acetonitrile (50 mL), and 6-bromohexyl-2-hexyldecanoate **(S14-2,** 3.14 g, 7.5 mmol, **S14-2** was prepared by the reaction between 2-hexyldecanoic acid 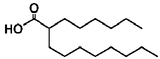 and 6-bromohexanol 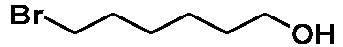 ) and **DIPEA** (0.54 g, 6.0 mmol) were added sequentially under slow stirring. The reaction was stirred for about 20 h at room temperature. After the reaction, the reaction solution was concentrated, and dissolved with dichloromethane, and then extracted sequentially with 0.6 M hydrochloric acid/10% sodium chloride solution and saturated sodium bicarbonate solution. The organic phases were combined, and dried with anhydrous magnesium sulfate, filtered and concentrated, and purified by column chromatography to obtain **S14-3** (2.51 g).
Step b: under nitrogen atmosphere, **DCC** (0.91 g, 4.4 mmol) was added to a round-bottom flask containing compound **S14-3** (2.05 g, 2.0 mmol), 10-undecen-1-ol (**S14-4**, 0.41 g, 2.4 mmol) and **DMAP** (61.00 mg, 0.5 mmol) dissolved in dichloromethane (30 mL), and reacted for 16 h at room temperature. After the reaction, the precipitate was removed by filtration, the filtrate was concentrated, and the resulting residue was purified by column chromatography to obtain compound **E14-1** (1.97 g).
Step c: the above compound **E14-1** (1.76 g, 1.5 mmol) was dissolved in THF (20 mL) and added to a nitrogen-protected flask, and **TBAF** solution (20 mL, 1 M) was added to react overnight to remove the TBS protection. After the reaction, the reaction solution was concentrated and extracted. The organic phases were combined, dried with anhydrous sodium sulfate, filtered and concentrated, and purified by column chromatography to obtain cationic lipid **E14-2** (1.39 g, 87.3%). ¹H NMR (400 MHz, CDCl₃) δ: 5.86-5.75 (m, 1H), 5.05-4.81 (m, 2H), 4.05 (t, 6H), 3.60-3.55 (m, 2H), 3.18-2.99 (m, 3H), 2.76-2.68 (m, 2H), 2.43-2.26 (m, 11H), 1.74-1.22 (m, 88H), 0.87 (t, 12H). MS (ESI): m/z=1061.97 ([M+H]⁺).

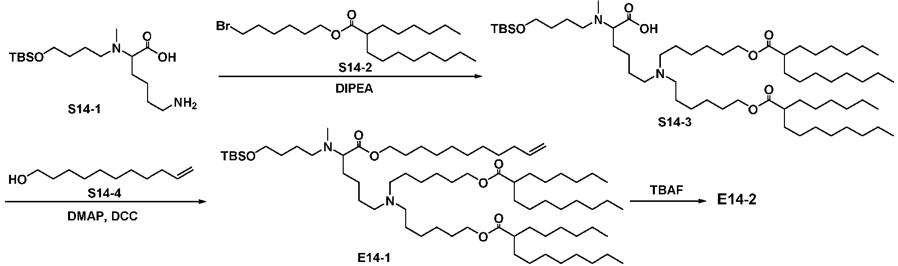

### Example 15: Cationic lipid (E15-1)

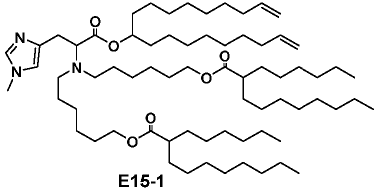

The preparation process is as follows:
Step a: under nitrogen protection, 1-methylhistidine (**S15-1**, 0.51 g, 3.0 mmol) was dissolved in acetonitrile (50 mL), **S14-2** (3.14 g, 7.5 mmol) and **DIPEA** (0.54 g, 6.0 mmol) were added sequentially under slow stirring, and the reaction was stirred for about 20 h at room temperature. After the reaction, the reaction solution was concentrated and dissolved with dichloromethane, and then extracted sequentially with 0.6 M hydrochloric acid/10% sodium chloride solution and saturated sodium bicarbonate solution. The organic phases were combined, dried with anhydrous magnesium sulfate, filtered and concentrated, and purified by column chromatography to obtain **S15-2** (2.08 g).
Step b: under nitrogen atmosphere, **DCC** (0.91 g, 4.4 mmol) was added to a round-bottom flask containing compounds **S15-2** (1.69 g, 2.0 mmol), **S15-3** (0.67 g, 2.4 mmol) and **DMAP** (61.00 mg, 0.5 mmol) dissolved in dichloromethane (30 mL), and the reaction was conducted for 16 h at room temperature. After the reaction, the precipitate was removed by filtration, the filtrate was concentrated, and the resulting residue was purified by column chromatography to obtain cationic lipid **E15-1** (1.79 g). ¹H NMR (400 MHz, CDCl₃) δ: 7.60 (s, 1H), 6.89 (s, 1H), 5.86-5.75 (m, 2H), 5.05-4.81 (m, 5H), 4.64 (t, 1H), 4.02 (t, 4H), 3.71 (s, 3H), 3.22-3.09 (m, 2H), 2.43-2.28 (m, 6H), 2.11-1.93 (m, 4H), 1.70-1.25 (m, 88H), 0.89 (t, 12H). MS (ESI): m/z=1108.99 ([M+H]⁺).

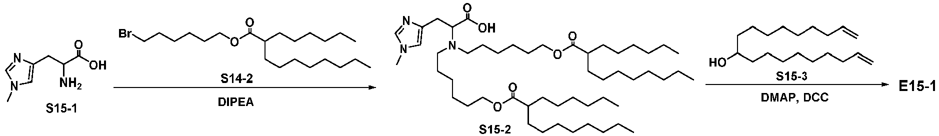

### Example 16: Preparation of LNP-mRNA pharmaceutical composition and assays for its physicochemical properties

### Example 16.1: Preparation of LNP-mRNA pharmaceutical composition

In the present embodiment, multiple sets of LNP-mRNA pharmaceutical compositions containing Fluc-mRNA were prepared for comparison. The phospholipids contained in each group were DSPC, and the steroid lipids contained were cholesterol. The PEGylated lipids contained in the experimental groups L-1 to L-15 were all PEG2k-DMG (abbreviated as DMG), and the PEGylated lipid contained in the experimental group L-16 was PL-3 ( 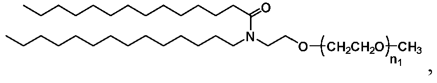 prepared with reference to the method disclosed in CN115515924A); wherein, the cationic lipid of the control group L-0 was an amino acid-based cationic lipid in the prior art (abbreviated as CL-1, prepared with reference to the method disclosed in CN104168887A, and the structure was represented by 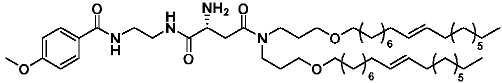 ); the cationic lipids of the experimental group series (L-1~L-16) were amino acid-based cationic lipids containing unsaturated bonds prepared in the embodiment of the present application, specifically as shown in Table 1.

The preparation method of LNP-mRNA pharmaceutical composition is as follows:
Step a: the cationic lipid, DSPC, cholesterol, and PEG-lipid were dissolved in ethanol at a molar ratio of 48:9:42:1.5 to obtain an ethanol phase solution;
Step b: Fluc-mRNA was added to 10~50 mM citrate buffer (pH=4) to obtain an aqueous solution;
Step c: the ethanol phase solution and aqueous phase solution were mixed (1:3 v/v) to prepare LNP-mRNA and washed by multiple DPBS ultrafiltration to remove ethanol and free molecules, and finally, filtered through a sterile filter of 0.2 µm to obtain the LNP-mRNA pharmaceutical compositions.

### Example 16.2: Assays for physicochemical properties of LNP-mRNA pharmaceutical compositions

Determination of encapsulation rate: encapsulation rate of LNP-mRNA compositions was determined using the Quant-iT RiboGreen RNA quantification kit. The results showed that the lipid compositions of the present application (L-1~L-15) had high nucleic acid encapsulation rates for nucleic acid drugs, all in the range of 88%-95%, and most of the encapsulation rates were in the range of 90%-95%. The results are shown in Table 1 below. The results showed that the lipid compositions prepared from amino acid-based cationic lipids in each experimental group could effectively encapsulate mRNA, showing a better encapsulation rate than the existing amino acid-based cationic lipids, and there were also differences in the encapsulation rate among various amino acid-based cationic lipids.

Determination of particle size: in the present embodiment, the particle size of LNP-mRNA was determined by dynamic light scattering (DLS), and the results were shown in Table 1 below. The measured LNP-mRNA had high dimensional uniformity, and the PDI were all smaller than 0.3. The particle sizes of the LNP-mRNA prepared with the lipid composition of the present application were in the range of 90-110 nm.

**Table 1: Summary table of particle size and encapsulation rate of the formulation of each lipid composition and the prepared LNP-mRNA**

| **LNP-mRNA** | **L-0** | **L-1** | **L-2** | **L-3** | **L-4** | **L-5** |
|---|---|---|---|---|---|---|
| **Cationic lipid CL** | CL-1 | E1-1 | E2-1 | E3-1 | E4-2 | E5-2 |
| **PEGylated lipid PL** | DMG | | | | | |
| **Encapsulation rate /%** | 88.37 | 95.21 | 94.38 | 94.73 | 93.20 | 92.37 |
| **Particle size /nm** | 101.59 | 93.84 | 92.05 | 91.33 | 93.58 | 94.51 |

| **LNP-mRNA** | **L-6** | **L-7** | **L-8** | **L-9** | **L-10** | **L-11** |
|---|---|---|---|---|---|---|
| **Cationic lipid CL** | E6-2 | E7-1 | E8-1 | E9-1 | E10-2 | E11-2 |
| **PEGylated lipid PL** | DMG | | | | | |
| **Encapsulation rate /%** | 93.65 | 89.47 | 91.73 | 93.26 | 95.11 | 92.10 |
| **Particle size /nm** | 96.30 | 98.42 | 96.25 | 93.37 | 92.17 | 94.81 |

| **LNP-mRNA** | **L-12** | **L-13** | **L-14** | **L-15** | **L-16** | |
|---|---|---|---|---|---|---|
| **Cationic lipid CL** | E12-2 | E13-2 | E14-2 | E15-1 | E1-1 | |
| **PEGylated lipid PL** | DMG | | | | PL-3 | |
| **Encapsulation rate /%** | 91.42 | 89.20 | 91.35 | 90.07 | 93.72 | |
| **Particle size /nm** | 101.35 | 92.15 | 97.21 | 95.88 | 95.71 | |

### Example 17: Biological activity assays of formulation of LNP-mRNA pharmaceutical composition

### (1) Assays for cytotoxicity (biocompatibility)

The cytotoxicity of the formulation of LNP-mRNA pharmaceutical composition of the present application was tested by MTT assay. The formulation of LNP-mRNA pharmaceutical composition was dissolved in the medium to prepare the required concentration, and if necessary, an appropriate amount of co-solvent could be added. Hela cells were used as a cell model, and 100 µL/well of cell suspension was seeded into 96-well plate at a density of 1×10⁴ cells/well. After inoculation, the cells were incubated in a cell culture incubator at 37 °C, 4% CO₂ for 24 h and then the culture medium was discarded. Then, 100 µL medium containing 0.1-0.3 ug/well mRNA (LNP-mRNA pharmaceutical composition prepared in Example 16) was added into each well of the experimental group; and 100 µL of fresh medium was added to the blank control group. Each group had six replicate wells for each concentration (0.1 ug, 0.15 ug, 0.20 ug, 0.25 ug and 0.3 ug). After 24 h co-incubation of the formulation of LNP-mRNA pharmaceutical composition with Hela cells, 20 µL of PBS buffer containing 5 mg/mL MTT was added to each well. After 4 h incubation of MTT with cancer cells, the mixture of medium and MTT buffer was discarded, followed by adding 150 µL DMSO per well to dissolve the purple formazan crystals formed in living cells. After sufficient shaking, the absorbance was tested with a microplate reader. Calculations based on the measured absorbance values were carried out. The results showed that compared with the blank control group, the cell viability rate of the formulation of LNP-mRNA pharmaceutical composition prepared by the present application was greater than 95%, indicating that the formulation of LNP-mRNA pharmaceutical composition of the present application had good biocompatibility.

### (2) Serum stability evaluation

LNP-mRNA pharmaceutical compositions were added to the culture medium containing 10% fetal bovine serum (FBS) with agitation at 37 °C. Samples were taken at regular intervals to determine the particle size change of LNP-mRNA, and the serum stability of the LNP-mRNA pharmaceutical composition formulation was analyzed by testing its particle size change. The experimental results showed that within 7 days, the particle size changes of the control groups and the experimental groups were all less than 10%; especially the particle size changes of the experimental groups L-1, L-2, L-4, L-5, L-10, and L-15 were less than 5%, indicating that the formulation of LNP-mRNA pharmaceutical composition prepared by cationic lipids of the present application had good serum stability.

### (3) Study of cell transfection activity

To investigate the mRNA transfection rate at the cellular level of each group of LNP-mRNA pharmaceutical compositions prepared in Example 16 of the present application, luciferase bioluminescence was used for testing. The LNP-mRNA pharmaceutical composition was dissolved in a culture medium to prepare the required dose. Hela cells were used as the cell model, and cell suspensions of 100 µL/well were seeded in black-edged, clear-bottomed 96-well plate at a density of 6000 cells/well. After inoculation, the cells were cultured in a cell culture incubator for 24 h. The cells were then dosed with 0.2 ug mRNA per well, and the control group was added with the corresponding dose of free Fluc-mRNA. After 24 h of transfection, the old medium was replaced with a fresh medium containing D-fluorescein sodium substrate (1.5 mg/mL). After 5 min of incubation, bioluminescence was detected using a microplate reader. The stronger the fluorescence the more Fluc-mRNA was transported into the cytoplasm and translated to the corresponding fluorescent protein, as shown in Table 2. Among them, the relative value of fluorescence intensity was the ratio of the fluorescence intensity value of each group to the fluorescence intensity of the control group. The results showed that the LNP-mRNA pharmaceutical compositions prepared by the present application had excellent in vitro transfection effects, that was, the LNPs in the experimental groups were all effective nucleic acid delivery carriers, and they were superior to the L-0 group prepared by the prior art amino acid-based cationic lipids. Among them, the relative fluorescence values of the experimental groups L-1, L-2, L-9, L-10 and L-15 were higher, which might be because the compound contained ionizable tertiary amine structures, multiple degradable ester bonds, and unsaturated aliphatic chains, simultaneously; wherein, the ionizable tertiary amine that has generated a partial positive charge can combine with negatively charged nucleic acids, the degradable ester bonds can promote the endosomal escape of LNP-mRNA so that the mRNA can be released into the cytoplasm to exert therapeutic effects, and the unsaturated aliphatic chains can increase the membrane fluidity to enhance the cellular uptake. Compared with other experimental groups, although the encapsulation rates of the experimental groups L-2 and L-6 (containing only unsaturated aliphatic chains) were not very low, their relative fluorescence intensities were relatively diminished; this suggests that having more unsaturated aliphatic chains is not always better, while the amino acid-based cationic lipids containing both unsaturated aliphatic chains and degradable saturated aliphatic chains will exhibit higher transfection efficiency.

**Table 2: Results of Cell Transfection Assays**

| **Number** | **Relative Fluorescence Value** | **Number** | **Relative Fluorescence Value** | **Number** | **Relative Fluorescence Value** |
|---|---|---|---|---|---|
| **Blank** | 1 | **L-6** | 5.8 | **L-13** | 7.3 |
| **L-0** | 3.3 | **L-7** | 7.5 | **L-14** | 7.7 |
| **L-1** | 9.5 | **L-8** | 8.6 | **L-15** | 9.2 |
| **L-2** | 10.2 | **L-9** | 9.6 | **L-16** | 9.4 |
| **L-3** | 5.3 | **L-10** | 9.3 | | |
| **L-4** | 8.2 | **L-11** | 7.4 | | |
| **L-5** | 7.9 | **L-12** | 8.1 | | |

### (4) Study of transfection activity in animals

Babl/c mice (6- to 8-week-old female) were administered LNP-mRNA pharmaceutical compositions (L-2, L-9, L-16) by tail vein injection at a dose of 10 µg mRNA/mouse, and a small animal living body fluorescence imaging was performed 6 h, 12 h, and 24 h post-dose, respectively. After imaging at the last time point, the mice were euthanized, and the main organs (heart, liver, spleen, lung, kidney) and the muscles at injection sites were subjected to imaging. 0.2 mL of sodium D fluorescein (15 mg/mL) was injected intraperitoneally 10-15 minutes before imaging. The results showed that the LNP-mRNA pharmaceutical composition prepared by the amino acid-based cationic lipid of the present application also had excellent transfection efficiency in vivo, and was mainly distributed in the liver and spleen. The small animal fluorescence imaging and organ distribution of L-2 are shown in Figure 1 (with the organs presented from left to right being the heart, liver, spleen, lung, and kidney).

Those described above are only embodiments of the present application and are not for limitation. Any modification of equivalent structures or equivalent routes according to the present application, which may be applied in other related art directly or indirectly, should be included in the scope of the present application.

For those skilled in the art, without departing from the spirit and scope of the present application, and without unnecessary experimentation, the present application can be implemented in a wider range under equivalent parameters, concentrations, and conditions. While the present application has given particular examples, it should be understood that the present application can be further modified. In conclusion, under the principles of the present application, the present application is intended to cover any alterations, uses, or improvements of the present application, including changes departing from the scope disclosed in this application but made using conventional techniques known in the art.

## Claims

1. An amino acid-based cationic lipid, wherein the structure is represented by the general formula (1): 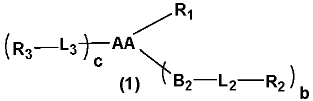
wherein, AA is a residue of an amino acid or amino acid derivative;
B2 is independently a linking bond or a C1-20 alkylene group at each occurrence;
L2 and L3 are each independently a linking bond or a divalent linking group at each occurrence;
R1 is a C10-40 aliphatic hydrocarbon group containing one, two or more unsaturated bonds; R2 is independently a C1-40 aliphatic hydrocarbon group or 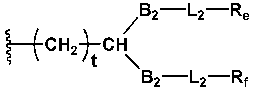 at each occurrence; wherein, t is an integer from 0 to 12; Re and Rf are each independently a C1-20 alkyl group, a C2-20 alkenyl group, or a C2-20 alkynyl group;
R3 is, at each occurrence, independently a hydrogen atom, an alkyl group, an alkoxy group, a C3-6 carbocyclic group, a nitrogen-containing heterocyclic group, -NRdRd, -SRd, -C(=O)Rd, -C(=O)ORd, -OC(=O)Rd or a functional group R01 that can interact with bio-related substances; wherein, Rd is independently a C1-12 alkyl group at each occurrence;
b and c are each independently 1 or 2; when fragments -B2-L2-R2 and/or -L3-R3 are protruded from the amino terminus of amino acids and their derivatives, b and c are each independently 1 or 2; when fragments -B2-L2-R2 and/or -L3-R3 are protruded from the carboxyl group terminus of amino acids and their derivatives, b and c are each independently 1; when b is 2, two -B2-L2-R2 fragments are the same or different; when c is 2, two -L3-R3 fragments are the same or different; when c is 2, two -L3-R3 fragments are the same or different;
or a salt, tautomer, stereoisomer, or solvate thereof.

2. The amino acid-based cationic lipid according to claim 1, wherein R1 is a C10-40 linear aliphatic hydrocarbon group containing one, two or three unsaturated bonds, preferably a linear C10-20 aliphatic hydrocarbon group containing one or two unsaturated bonds; more preferably, R1 is selected from the group consisting of the following structures: 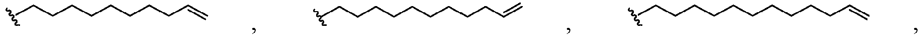 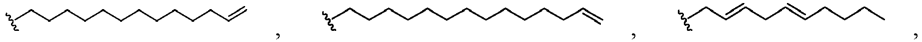 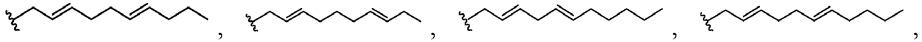 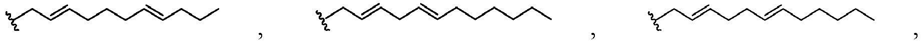 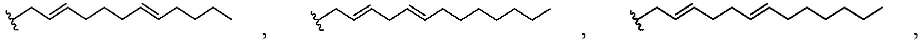 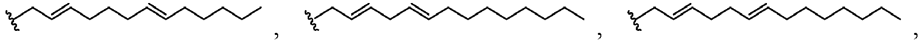 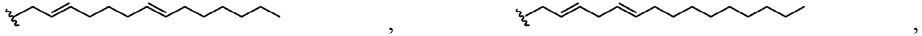 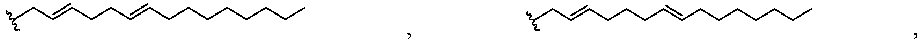 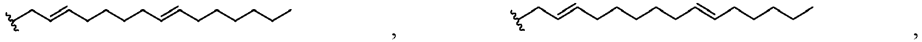 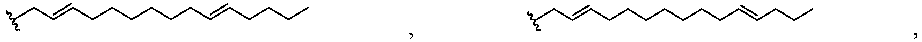 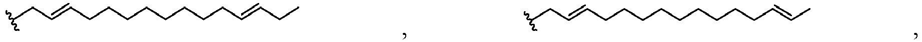 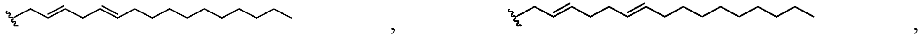 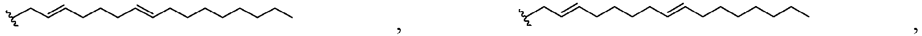 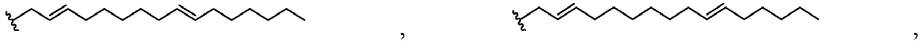 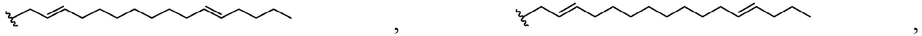 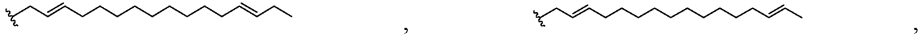 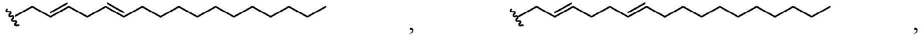 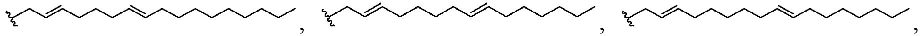 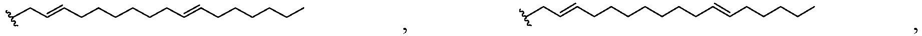 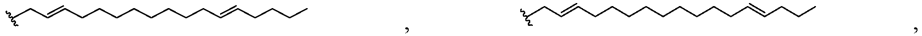 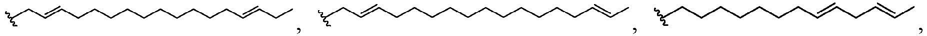 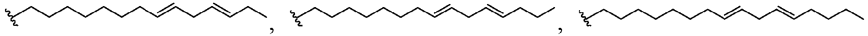 and

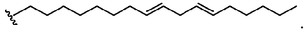

3. The amino acid-based cationic lipid according to claim 1, wherein R1 is a branched aliphatic hydrocarbon group containing two or more unsaturated bonds, preferably a branched C15-40 aliphatic hydrocarbon group containing two or more unsaturated bonds; preferably, R1 is selected from the group consisting of the following structures: 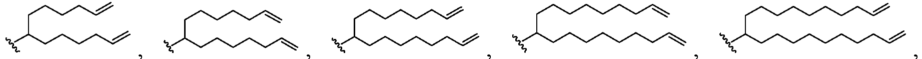 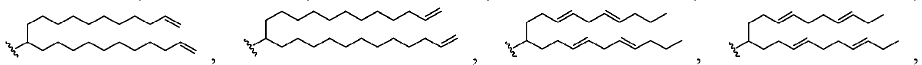 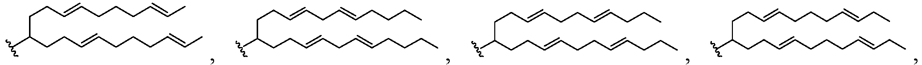 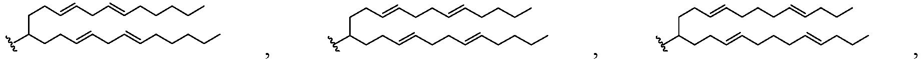 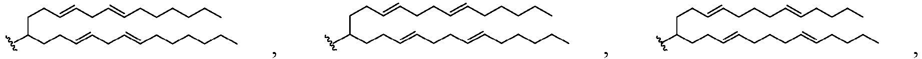 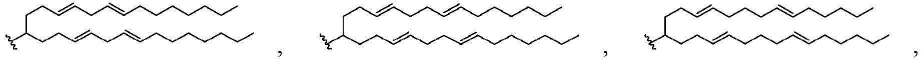 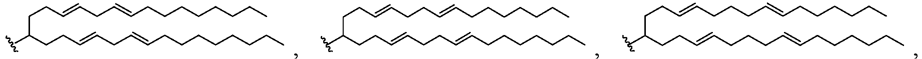 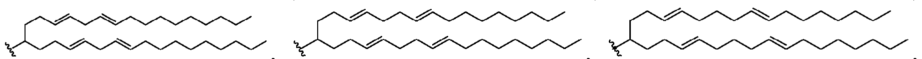 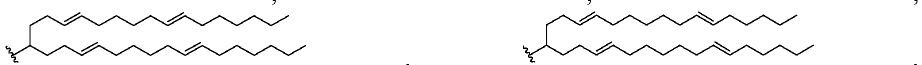 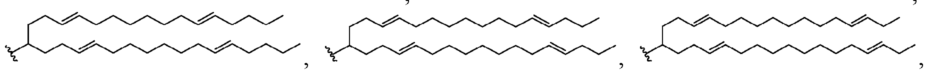 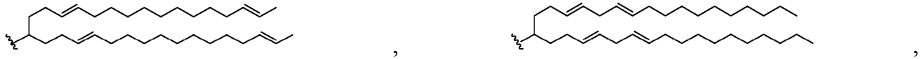 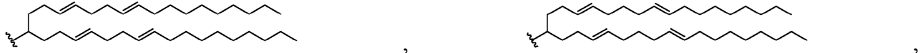 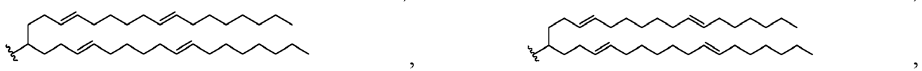 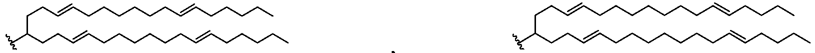 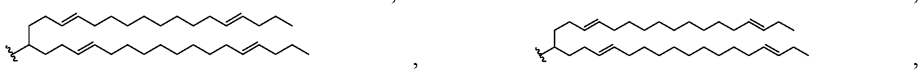 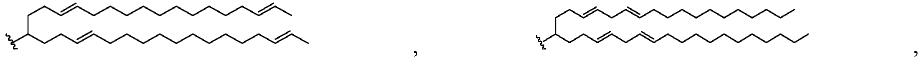 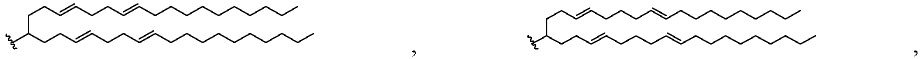 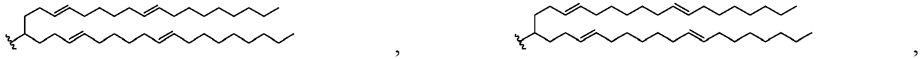 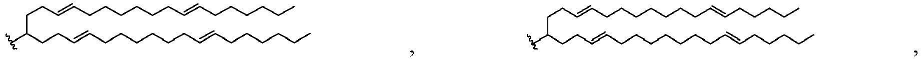 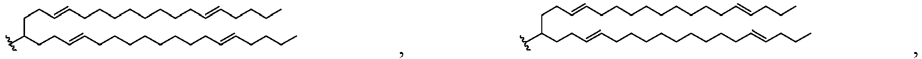 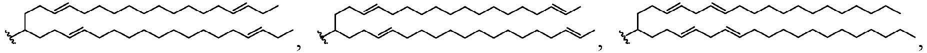 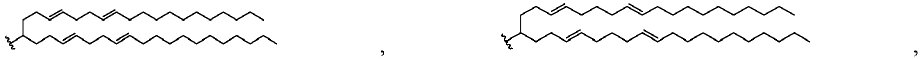 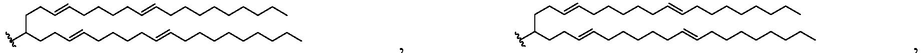 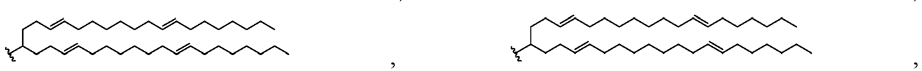 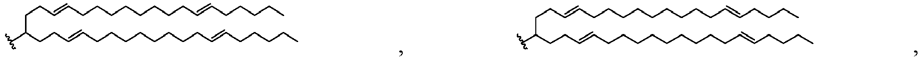 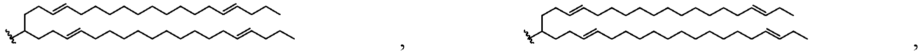 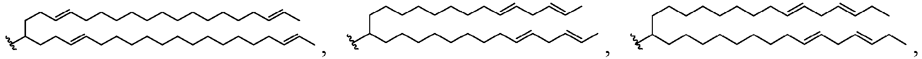 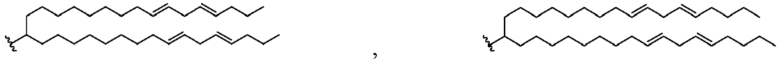 and

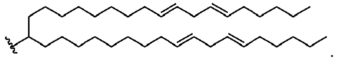

4. The amino acid-based cationic lipid according to claim 1, wherein the amino acid or amino acid derivative is preferably selected from the group consisting of arginine, aspartic acid, asparagine, cysteine, glutamic acid, glutamine, histidine, lysine, methionine, phenylalanine, serine, threonine, tryptophan, tyrosine, and an amino acid derivative of any of the aforementioned amino acids, more preferably selected from the group consisting of lysine, glutamic acid and histidine, more preferably selected from the group consisting of the following structures and the corresponding forms having 1 to 3 protected terminal groups:

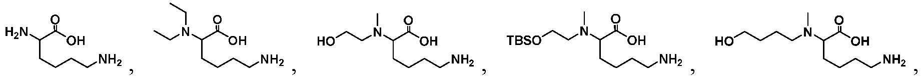

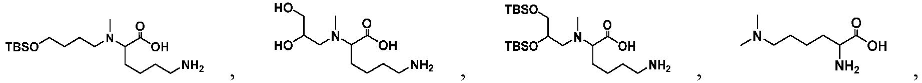

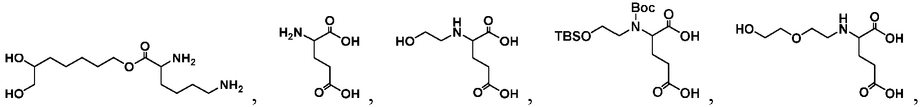

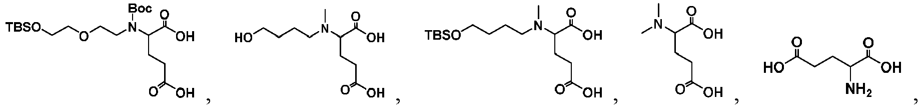

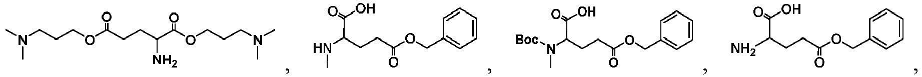

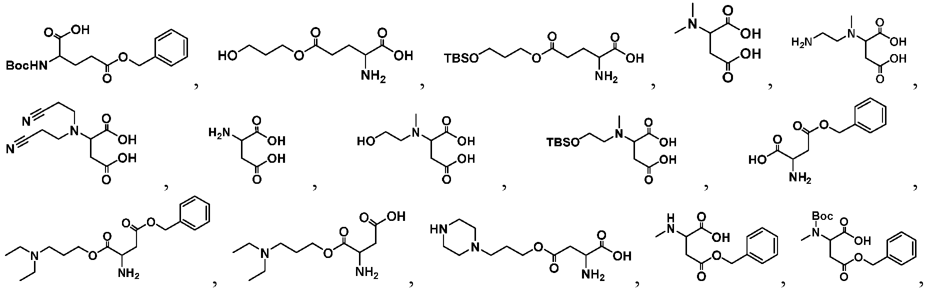

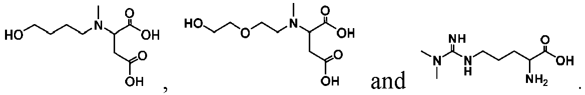

5. The amino acid-based cationic lipid according to claim 1, wherein the AA is preferably 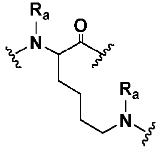 or 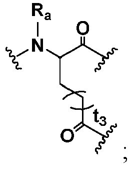 wherein, Ra is independently selected from the group consisting of a linking bond, H, a methyl group, an ethyl group, a propyl group, and an isopropyl group at each occurrence; or, AA is the case wherein one or two carbonyl groups in any of the above structures are independently capped by an oxygen atom or a secondary amine group, including: 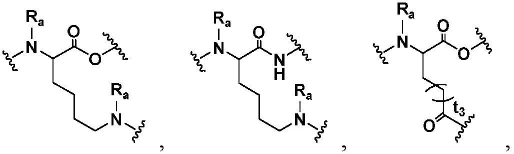 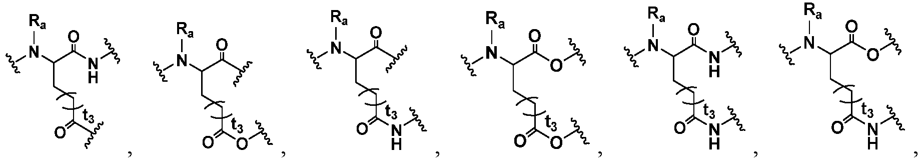 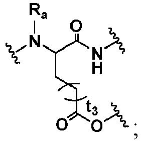 AA is preferably selected from the group consisting of the following structures:

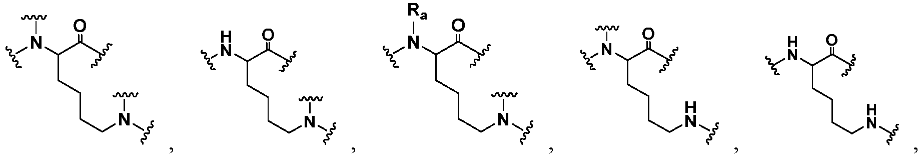

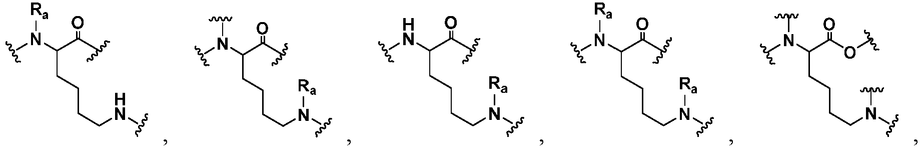

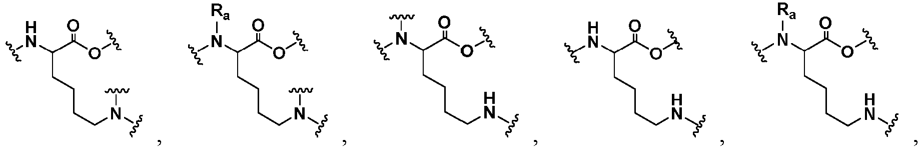

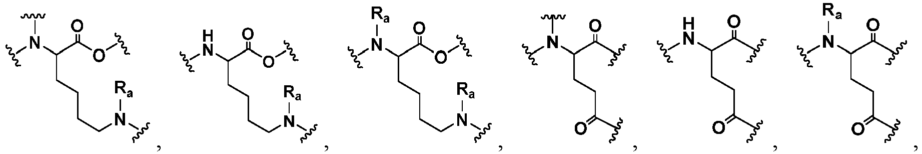

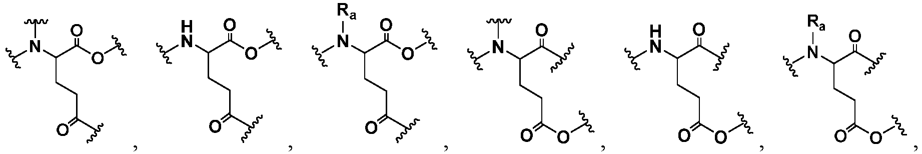

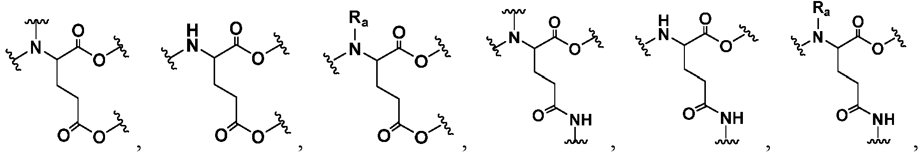

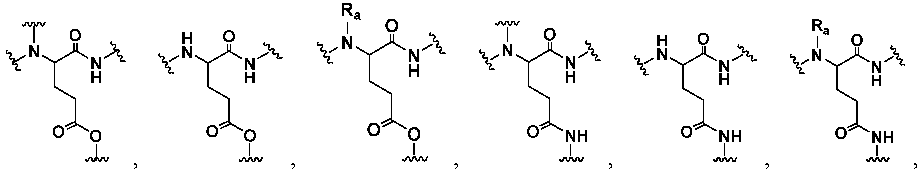

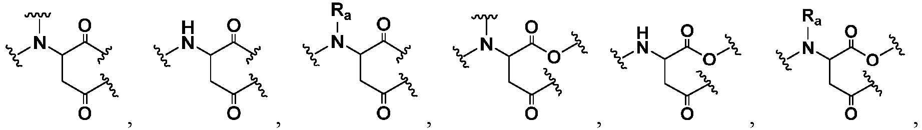

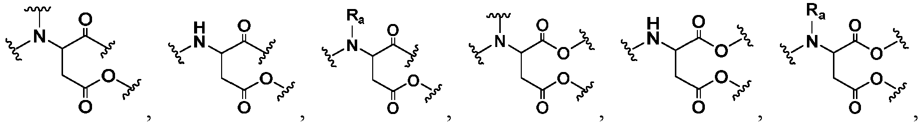

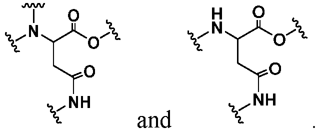

6. The amino acid-based cationic lipid according to claim 1, wherein B2 is independently a linking bond or a C1-20 alkylene group at each occurrence; the C1-20 alkylene group is selected from the group consisting of a methylene group, an ethylene group, a propylene group, a butylene group, a pentylene group, a hexylene group, a heptylene group, an octylene group, a nonylene group, a decylene group, a undecylene group, a dodecylene group, a tridecylene group, a tetradecylene group, a pentadecylene group, a hexadecylene group, a heptadecylene group, an octadecylene group, a nonadecylene group and an eicosylene group; more preferably, B2 is independently a linking bond or a C2-10 alkylene group; the C1-20 alkylene group and C2-10 alkylene group are optionally substituted by 0 to 5 identical or different C1-12 alkyl groups, cycloalkyl groups, heterocyclyl groups or hydroxyl groups; wherein, the C1-12 alkyl group is preferably a methyl group, an ethyl group, or a propyl group.

7. The amino acid-based cationic lipid according to claim 1, wherein L2 is independently selected from the group consisting of a linking bond, -OC(=O)-, -C(=O)O-, -OC(=O)O-, -C(=O)-, -O-, -NH-, -O(CRcRc)sO-, -S-, -C(=O)S-, -SC(=O)-, -NRcC(=O)-, -C(=O)NRc-, -NRcC(=O)NRc-, -OC(=O)NRc-, -NRcC(=O)O-, -SC(=O)NRc- and -NRcC(=O)S- at each occurrence; wherein, Rc is independently a hydrogen atom or a C1-12 alkyl group at each occurrence, and s is an integer from 2 to 4; L2 is more preferably selected from the group consisting of a linking bond, -OC(=O)-, -C(=O)O-, -OC(=O)O-, -C(=O)-, -O-, -O(CH2)sO-, -S-, -C(=O)S-, -SC(=O)-, -NHC(=O)-, -C(=O)NH-, -NHC(=O)NH-, -OC(=O)NH-, -NHC(=O)O-, -SC(=O)NH- and -NHC(=O)S-; more preferably, L2 is independently selected from the group consisting of a linking bond, -C(=O)-, -O-, -NH-, -OC(=O)-, -C(=O)O-, -NHC(=O)- and -C(=O)NH- at each occurrence.

8. The amino acid-based cationic lipid according to claim 1, wherein L3 is independently selected from the group consisting of a linking bond, -O-, -NH-, -C(=O)-, -(CH2)t-, -(CH2)tO-, -O(CH2)t-, -(CH2)tNH-, -(CH2)tC(=O)-, -(CH2)tO(CH2)t-, -C(=O)O(CH2)t-, -(CH2)tC(=O)O- and -(CH2)tOC(=O)- at each occurrence.

9. The amino acid-based cationic lipid according to claim 1, wherein R3 is independently selected from the group consisting of a hydrogen atom, -Rd, -ORd, -NRdRd, -C(=O)Rd-, -C(=O)ORd, -OC(=O)Rd, -OC(=O)ORd and R01 at each occurrence; the R3 is more preferably, independently selected from any of the following cases:
Case (1): R3 is independently selected from the group consisting of a hydrogen atom, an alkyl group, an alkoxy group, -C(=O)ORd, -OC(=O)Rd, -OC(=O)ORd, an epoxy group, a hydroxyl group, a protected hydroxyl group, a sulfhydryl group, a protected sulfhydryl group, a carboxyl group, a protected carboxyl group, an amino group, a protected amino group, an aldehyde group, a protected aldehyde group, an active ester group, a carbonate group, a urethane groups, an isocyanate group, an isothiocyanate group, a succinimidyl group, a maleimide group, a protected maleimide group, a dimethylamino group, an alkenyl group, an enoate group, an azido group, a cyano group, a dithiopyridyl group, an α-haloacetyl alkynyl group, an alkynyl group, a folate group, a rhodamine group, a biotin group, a monosaccharide group, a polysaccharide group, 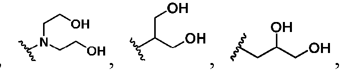 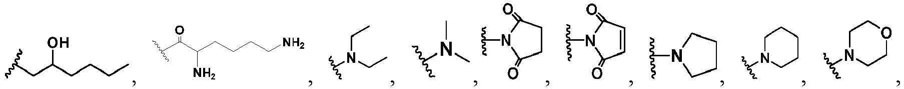 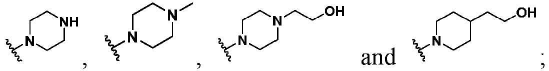
Case (2): R3 is independently a functional group with therapeutic targeting properties; R3 is preferably a residue of folic acid, N-acetylgalactosamine, or a functional derivative thereof;
more preferably, R3 is selected from the group consisting of the following structures:

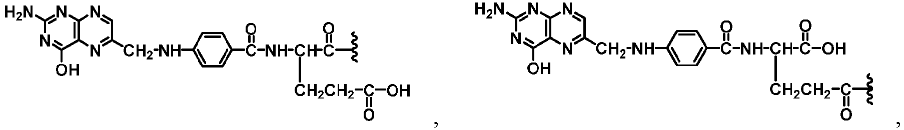

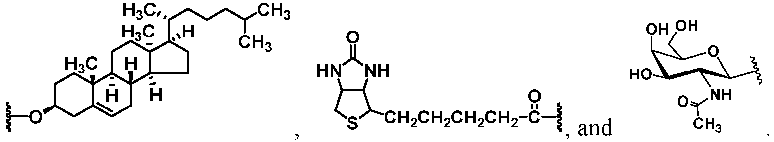

10. The amino acid-based cationic lipid according to claim 9, wherein the -L3-R3 fragment is selected from the group consisting of the following structures: 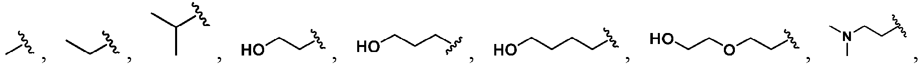 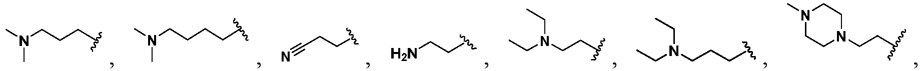 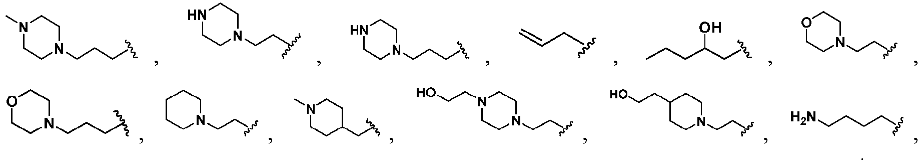 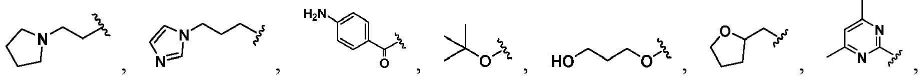 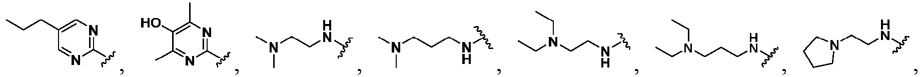 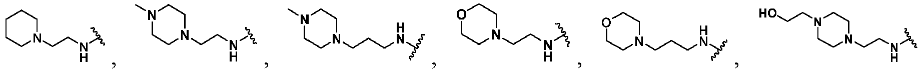 and

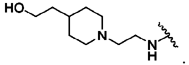

11. The amino acid-based cationic lipid according to claim 1, wherein the R2 is independently a linear alkyl group, a branched alkyl group, a linear alkenyl group, a branched alkenyl group, a linear alkynyl group, a branched alkynyl group, or 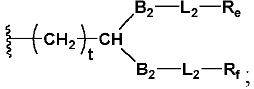
the linear alkyl group is preferably a C1-25 linear alkyl group, more preferably independently a C1-17 linear alkyl group which is specifically selected from the group consisting of a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group and a heptadecyl group;
the branched alkyl group, branched alkenyl group or branched alkynyl group is represented as 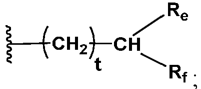 wherein, Re and Rf are each independently a C1-20 alkyl group, a C2-20 alkenyl group or a C2-20 alkynyl group; more preferably, Re and Rf are each independently selected from the group consisting of a pentyl group, a hexyl group, a heptyl group, an octyl group, a nonyl group, a decyl group, an undecyl group, a dodecyl group, and an octadeca-6,9-dienyl group; more preferably, the branched alkyl group, branched alkenyl group or branched alkynyl group is selected from the group consisting of the following structures: 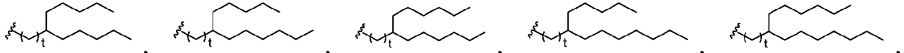 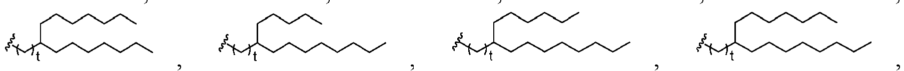 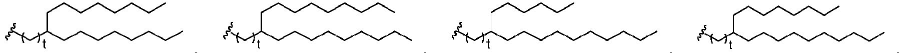 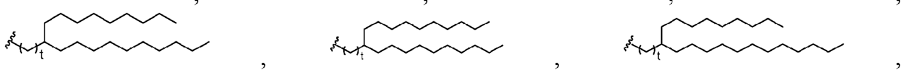 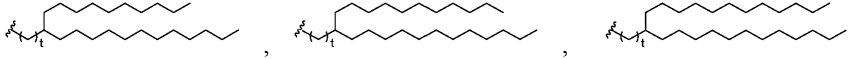
and 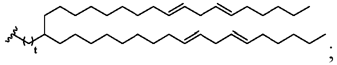
the 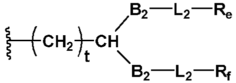 is represented as 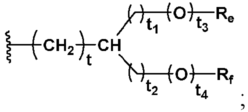 wherein, t1 and t2 are each independently an integer of 0 to 5, t3 and t4 are each independently 0 or 1, and t1, t2, t3, and t4 are not simultaneously 0; more preferably, 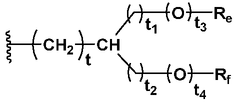 is 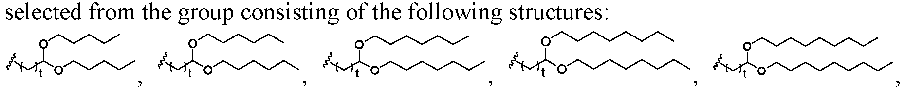 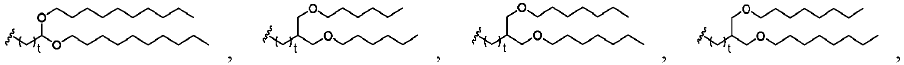 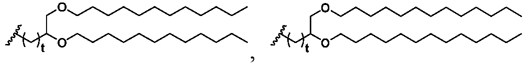
and

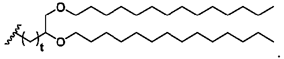

12. The amino acid-based cationic lipid according to claim 7, wherein the -B2-L2-R2 is independently selected from the group consisting of the following structures at each occurrence: 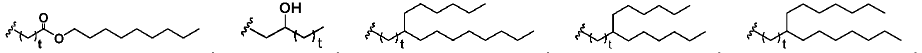 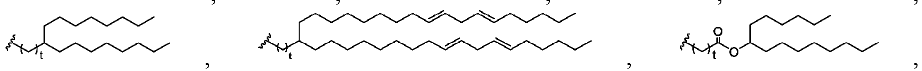 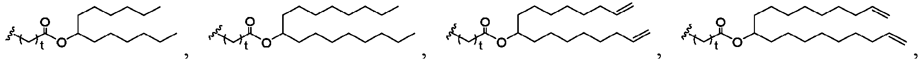 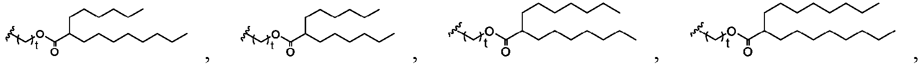 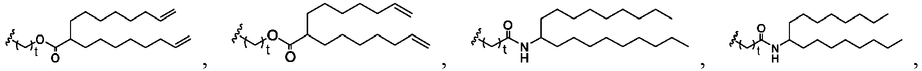 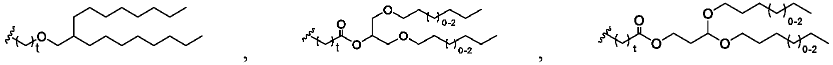 and

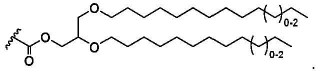

13. The amino acid-based cationic lipid according to claim 1, wherein the structure of the amino acid-based cationic lipid is selected from the group consisting of the following general formulas: 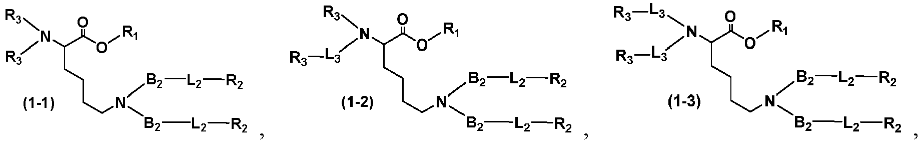 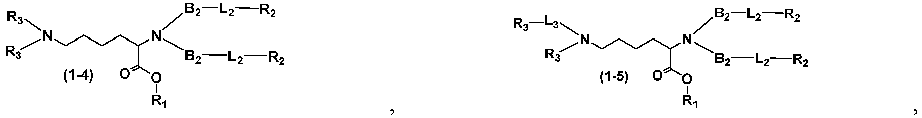 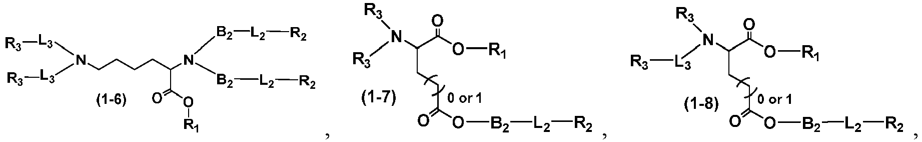 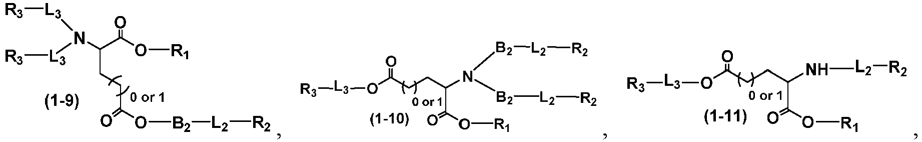 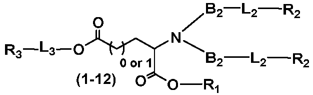 and 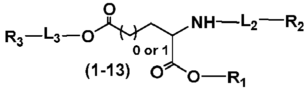 ; wherein, none of B2, L2 and L3 is a linking bond; more preferably, L2 is independently selected from the group consisting of -C(=O)-, -C(=O)O-, -OC(=O)-, -OC(=O)O-, -C(=O)NH-, -NHC(=O)-, -OC(=O)NH- and -NHC(=O)O- at each occurrence; more preferably, L2 is independently selected from the group consisting of -C(=O)-, -C(=O)O-, -OC(=O) -, and -OC(=O)O- at each occurrence.

14. The amino acid-based cationic lipid according to any one of claims 1 to 13, wherein the amino acid-based cationic lipid is selected from the group consisting of the following structures:

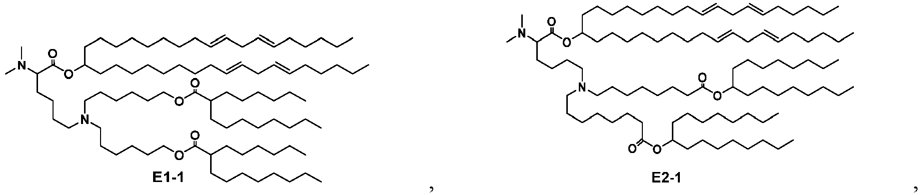

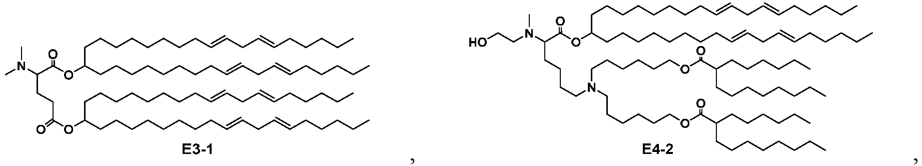

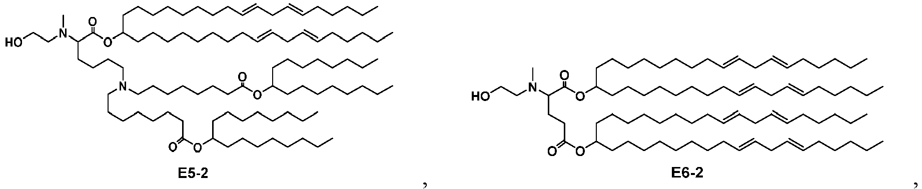

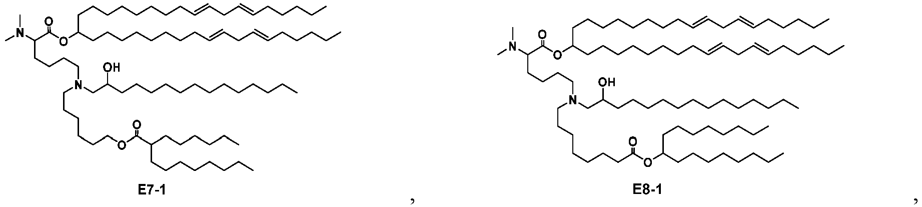

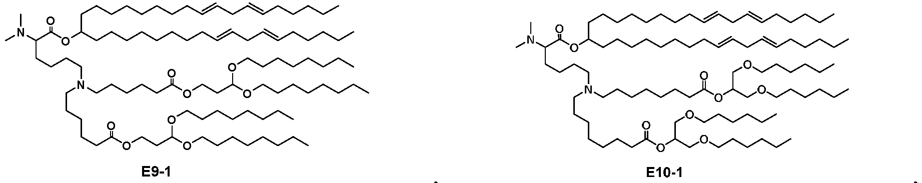

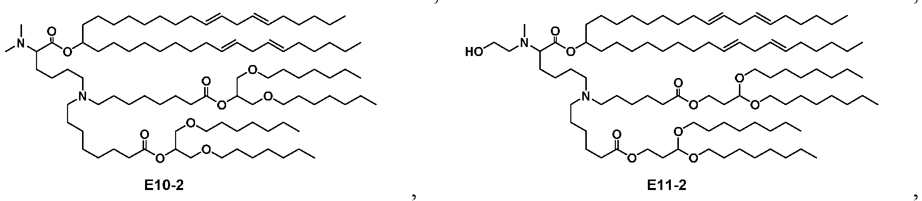

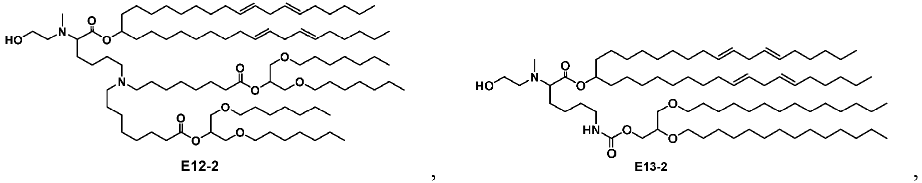

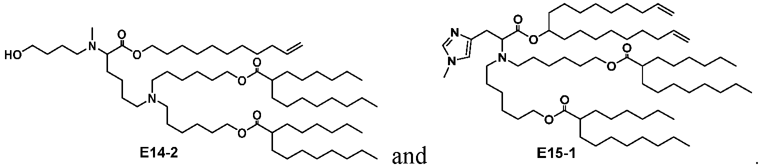

15. A lipid composition containing an amino acid-based cationic lipid of any one of claims 1 to 14.

16. The lipid composition according to claim 15, wherein the lipid composition contains one or more types of lipids selected from the group consisting of phospholipid, steroid lipid and PEGylated lipid, and the lipid composition is selected from any of the following cases:
Case (1): also contains a phospholipid;
Case (2): also contains a steroid lipid;
Case (3): also contains a PEGylated lipid;
Case (4): also contains a phospholipid and steroid lipid;
Case (5): also contains a phospholipid and PEGylated lipid;
Case (6): also contains a steroid lipid and PEGylated lipid;
Case (7): also contains a phospholipid, a steroid lipid and a PEGylated lipid;
more preferably, the lipid composition also contains a neutral lipid, a steroid lipid and a PEGylated lipid, simultaneously.

17. The lipid composition according to claim 16, wherein the phospholipid is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-O-octadecenyl-sn-glycero-3-phosphatidylcholine, 1-oleoyl-2-cholesterylhemisuccinyl-sn-glycero-3-phosphocholine, 1-O-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamines, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamines, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof;
or the steroid lipid is selected from the group consisting of cholesterol, fecal sterol, sitosterol, ergosterol, campesterol, stigmasterol, rapeseed sterol, lycopene, ursolic acid, α-tocopherol, and combinations thereof;
or the PEGylated lipid is selected from the group consisting of 1,2-dimyristoyl-sn-glycerol methoxypolyethylene glycol, 1,2-distearoyl-sn-glycero-3-phosphoethanolamine-N-[amino(polyethylene glycol)], PEG-cholesterol, PEG-diacylglycamide, PEG-dialkyloxypropyl, PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine and PEG2000-2,3-distearoylglycerol, and combinations thereof;
or the structure of the PEGylated lipid is selected from the group consisting of the following structures and combinations thereof: 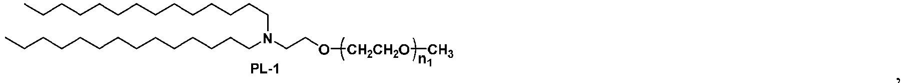 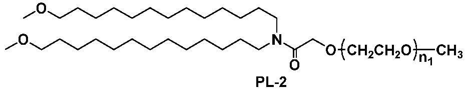 and 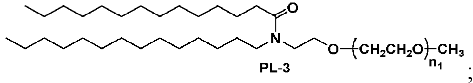 wherein, n1 is an integer from 25 to 300.

18. The lipid composition according to any one of claims 16 to 17, wherein the lipid composition comprises 20-80% amino acid-based cationic lipid, 5-15% phospholipid, 25-55% steroid lipid, and 0.5-10% PEGylated lipid; the percentage is the molar percentage of each lipid in the total lipid in a solution containing solvent.

19. The lipid composition according to claim 18, wherein the molar percentage of the amino acid-based cationic lipid in the total lipids in a solution containing solvent is 30-65%, more preferably 35%, 40%, 45%, 46%, 47%, 48%, 49%, 50%, or 55%;
or, the molar percentage of phospholipid in the total lipids in a solution containing solvent is 7.5-13%, more preferably 8%, 9%, 10%, 11%, or 12%;
or, the molar percentage of steroid lipid in the total lipids in a solution containing solvent is 35-50%, more preferably 40%, 41%, 42%, 43%, 44%, 45%, 46%, 47%, 48%, 49%, or 50%;
or, the molar percentage of PEGylated lipid in total lipids in a solution containing solvent is 0.5-5%, preferably 1-3%, more preferably approximately 1.5%, 1.6%, 1.7%, 1.8%, or 1.9%.

20. A lipid pharmaceutical composition containing a lipid composition of any one of claims 15 to 19 and a drug, wherein the drug is selected from the group consisting of a nucleic acid drug, a gene vaccine, an antitumor drug, a small molecule drug, a peptide drug, and a protein drug.

21. The lipid pharmaceutical composition according to claim 20, wherein the nucleic acid drug is selected from the group consisting of RNA, DNA, antisense nucleic acid, plasmid, interfering nucleic acid, aptamer, antagomir and ribozyme; the RNA is selected from the group consisting of mRNA, saRNA, circRNA, miRNA and siRNA; preferably, the nucleic acid drug is selected from the group consisting of DNA, mRNA, miRNA and siRNA.

22. The lipid pharmaceutical composition according to any one of claims 20 to 21, wherein the pharmaceutical composition is used as a drug, selected from the group consisting of an antineoplastic agent, an antiviral agent, an antifungal agent and a vaccine.

23. A formulation of LNP pharmaceutical composition containing any lipid pharmaceutical composition of claim 22 and a pharmaceutically acceptable diluent or excipient; the diluent or excipient is preferably selected from the group consisting of deionized water, ultrapure water, phosphate buffer and normal saline, more preferably phosphate buffer or normal saline, most preferably normal saline.

24. A liposome or lipid nanoparticle containing the lipid composition of any one of claims 15 to 19.

25. The liposome or lipid nanoparticle according to claim 24, wherein the lipid nanoparticle is an LNP pharmaceutical composition, an LPP pharmaceutical composition, or a PNP pharmaceutical composition, preferably an LNP pharmaceutical composition, more preferably an LNP-nucleic acid pharmaceutical composition, more preferably an LNP-mRNA pharmaceutical composition.
